# FASCICULE DE BREVET EUROPEEN

(11) **EP 0 678 089 B1**
(45) Date de publication et mention de la délivrance du brevet: **09.10.1996**
(21) Numéro de dépôt: 94903940.8
(22) Date de dépôt: 03.01.1994
(51) Int. Cl.: C07D 207/16, C07K 5/062, A61K 31/40, A61K 38/05, C07D 207/08, C07D 207/09, C07D 403/12, C07D 403/06

(54) **N-ACYL PYRROLIDINES ET MEDICAMENTS POUR LE TRAITEMENT OU LA PREVENTION DES DESORDRES LIES A LA CKK ET LA GASTRINE**
N-ACYLPYRROLIDINE UND ARZNEIMITTEL ZUR BEHANDLUNG ODER PROPHYLAXE VON MIT CKK UND GASTRIN IN ZUSAMMENHANG STEHENDEN KRANKHEITEN
N-ACYL PYRROLIDINES AND DRUGS FOR THE TREATMENT OR PREVENTION OF CHOLECYSTOKININ AND GASTRIN-RELATED DISORDERS

(30) Priorité: 07.01.1993 FR 9300078
(43) Date de publication de la demande: 25.10.1995
(73) Titulaire: RHONE-POULENC RORER S.A., 92160 Antony (FR)
(72) Inventeur: CAPET, Marc, F-94320 Thiais (FR); DUBROEUCQ, Marie-Christine, F-95880 Enghien-les-Bains (FR); MANFRE, Franco, F-94450 Limeil-Brévannes (FR)
(74) Mandataire: Savina, Jacques
(86) Numéro de dépôt international: FR9400009
(87) Numéro de publication internationale: WO9415915

(56) Documents cités:
- EP-A- 0 124 317
- WO-A-91/13907
- FR-A- 2 678 938
- TETRAHEDRON LETTERS. vol. 34, no. 3 , 1993 , OXFORD, GB; pages 381-384; Magaard, Victoria W.; Sanchez, Robert M.; Bean, John W.; Moore, Michael L.: "A convenient synthesis of the conformationally constrained amino acid 5,5-dimethylproline"
- TETRAHEDRON LETTERS. vol. 34, no. 10 , 1993 , OXFORD, GB; pages 1665-1668; Baldwin, Jack E.; Hulme, Christopher; Edwards, Alison J.; Schofield, Christopher J.; Parkes, Kevin E. B.: "Synthesis of a bicyclic .gamma.-lactam dipeptide analog"

## Description

La présente invention concerne des dérivés de formule : 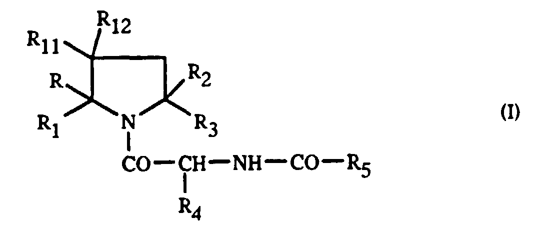 leurs sels, leur préparation et les médicaments les contenant.

Dans la formule (I),
R représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy,
R₁ représente un atome d'hydrogène ou un radical alkyle,
R₂ représente une chaîne -(CH₂)ₙ-CO-R₆, -(CH₂)ₘ-O-CO-R"₆, -(CH₂)ₘ-NR₉R₁₀ ou un radical oxazolinyle éventuellement substitué par un ou plusieurs radicaux alkyle ou alkyl-3 oxadiazolyle,
R₃ représente un atome d'hydrogène ou un radical alkyle,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 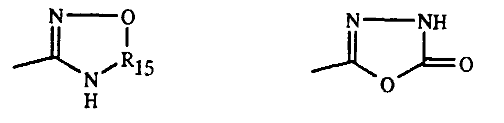 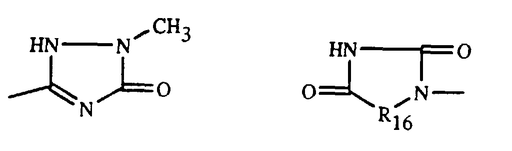 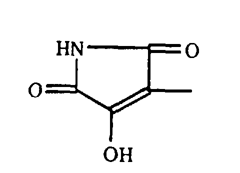 ou diméthyl-2,2 dioxo-4,6 dioxanne-1,3-yl-5,
R₆ représente un radical hydroxy, alcoxy, cycloalkyloxy, cycloalkylalkyloxy, phényle ou -NR₉R₁₀,
R"₆ représente un radical alcoxy, cycloalkyloxy, cycloalkylalkyloxy, phényle ou -NR₉R₁₀,
R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₁ représente un atome d'hydrogène ou un radical alkyle ou phénylalkyle,
R₁₂ représente un radical alkyle, phénylalkyle, phénylsulfonyle, -(CH₂)ₚ-CO-R₁₇, cyano, -CXO, -CX=NOH, -CX=N-O-alk-COOX, -CHX-OH, -CHX-O-CO-alk, -NH₂ ou -NH-CO-alk,
R₁₃ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₄ représente un radical tétrazolyl-5,
R₁₅ représente C=O ou S=O,
R₁₆ représente O ou C=O,
R₁₇ représente un radical hydroxy, alcoxy, cycloalkyloxy, cycloalkylalkyloxy, phényle, alkyle, phénylalkyloxy ou -NR₉R₁₀,
n est égal à 0, 1 ou 2,
m est égal à 1 ou 2,
p est égal à 0 ou 1
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque R représente un radical alkyle insaturé, celui-ci est de préférence un radical isopropylidène.

Lorsque R représente un radical cycloalkyle, celui-ci est de préférence un radical cyclohexyle.

Lorsque R représente un radical cycloalkyle insaturé, celui-ci est de préférence un radical tétrahydrophényle, cyclopentadiènyle ou dihydrophényle.

Lorsque R représente un radical polycycloalkyle, celui-ci est de préférence un radical norbornyle ou adamantyle.

Lorsque R représente un radical polycycloalkyle insaturé, celui-ci est de préférence un radical norbornènyle.

Lorsque R₇ et R₈ forment avec l'atome d'azote auquel ils sont rattachés un hétérocycle, celui-ci est de préférence un cycle pipéridino éventuellement substitué par un ou plusieurs radicaux alkyle ou un cycle tétrahydro-1,2,3,4 quinoléine.

Lorsque R₉ et R₁₀ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle.

Les composés de formule (I) comportant un ou plusieurs centres asymétriques présentent des formes isomères. Ces isomères font également partie de l'invention.

Les composés de formule (I) pour lesquels R₅ représente un radical phénylamino dont le noyau phényle est éventuellement substitué peuvent être préparés par action d'un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 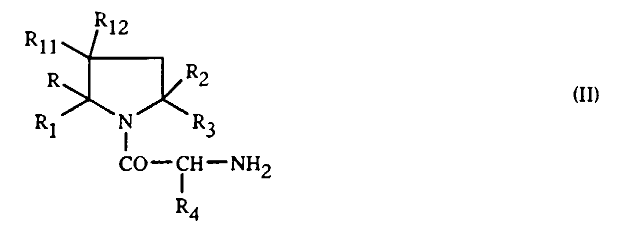 dans laquelle R, R₁, R₂, R₃, R₄, R₁₁ et R₁₂ ont les mêmes significations que dans la formule (I), sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 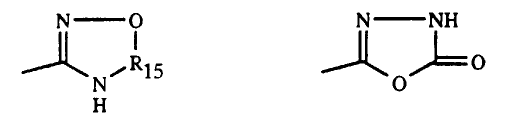 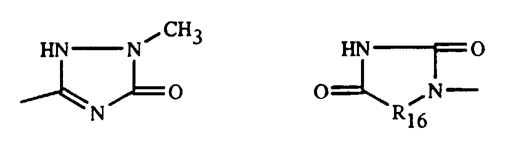 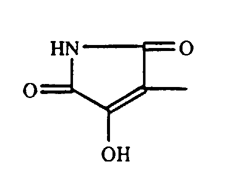 ou diméthyl-2,2 dioxo-4,6 dioxanne-1,3-yl-5.

Cette réaction s'effectue généralement au sein d'un solvant inerte tel que le tétrahydrofuranne, le N,N-diméthyl-formamide, un solvant chloré (chloroforme, dichloro-1,2 éthane par exemple) ou un solvant aromatique (benzène, toluène par exemple), à une température comprise entre 20°C et la température d'ébullition du solvant.

Le dérivé réactif de l'acide carbamique peut être obtenu dans les mêmes conditions de solvant et de température.

Les dérivés de formule (II) peuvent être obtenus par déprotection d'un dérivé de formule : 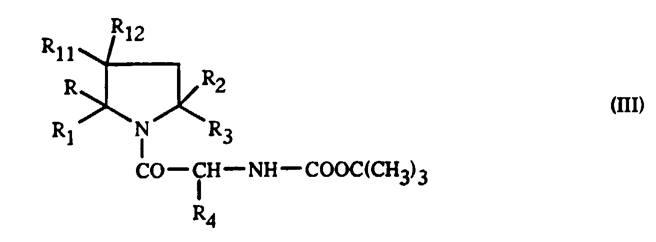 dans laquelle R, R₁, R₂, R₃, R₄, R₁₁ et R₁₂ ont les mêmes significations que dans la formule (I).

Cette déprotection s'effectue de préférence au moyen d'iodotriméthylsilane, au sein d'un solvant inerte tel qu'un solvant chloré (chloroforme, dichloro-1,2 éthane par exemple) ou l'acétonitrile, à une température comprise entre 15 et 40°C.

Les dérivés de formule (III) pour lesquels R₂ représente une chaîne -(CH₂)ₙ-CO-R₆, R₆ n'étant pas un radical hydroxy et R₁₂ représente un radical alkyle, phénylalkyle, phénylsulfonyle, cyano, -CXO ou -(CH₂)ₚ-CO-R₁₇, R₁₇ n'étant pas un radical hydroxy peuvent être obtenus par action d'un dérivé de formule : 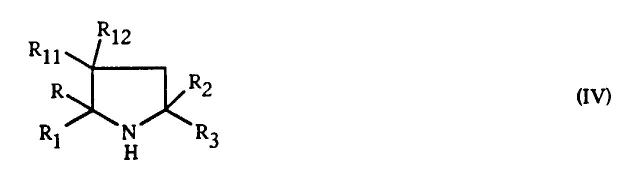 dans laquelle R, R₁, R₃ et R₁₁ ont les mêmes significations que dans la formule (I) et R₂ et R₁₂ ont les mêmes significations que précédemment, sur un acide de formule : 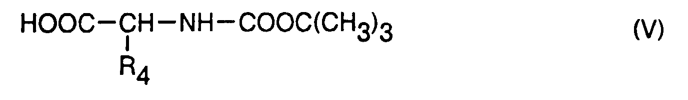 dans laquelle R₄ est défini comme dans la formule (I).

Cette réaction s'effectue au sein d'un solvant inerte tel que l'acétonitrile, le tétrahydrofuranne ou un solvant chloré, en présence d'un agent de condensation utilisé en chimie peptidique tel qu'un carbodiimide (N,N'-dicyclohexyl-carbodiimide par exemple) ou un chloroformiate d'alkyle, à une température comprise entre 10 et 40°C.

Les dérivés de formule (V) peuvent être obtenus selon les méthodes habituelles de protection des amino acides.

Les dérivés de formule (IV) pour lesquels R₂ représente une chaîne -(CH₂)ₙ-CO-R₆, n étant égal à 0 et R₆ n'étant pas hydroxy, R₁₂ représente un radical cyano, phénylsulfonyle, -CXO ou -(CH₂)ₚ-CO-R₁₇, p étant égal à 0 et R₁₇ n'étant pas un radical hydroxy peuvent être obtenus par réaction d'un dérivé de formule : 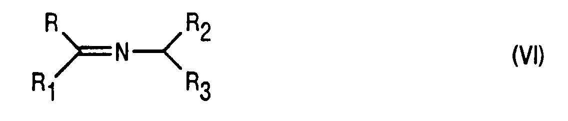 dans laquelle R, R₁ et R₃ ont les mêmes significations que dans la formule (I) et R₂ a les mêmes significations que précédemment, sur un dérivé de formule : 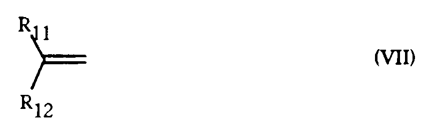 dans laquelle R₁₁ a les mêmes significations que dans la formule (I) et R₁₂ a les mêmes significations que précédemment.

Cette réaction s'effectue au sein d'un solvant inerte tel que l'acétonitrile, le tétrahydrofuranne, le toluène, en présence d'un sel métallique tel que l'acétate d'argent, le bromure de lithium, le bromure de magnésium, l'iodure de sodium, l'iodure de zinc, et d'une base azotée telle que la triéthylamine, à une température comprise entre 0°C et la température d'ébullition du milieu réactionnel.

Les dérivés de formule (VII) sont commercialisés ou peuvent être obtenus par adaptation des méthodes décrites par G. H. DEWAR et coll., Eur. J. Med. Chem., 20, 228 (1985), ELLES, Chimie Moderne, tome 4, 53 (1959) et dans le brevet DE 752481.

Les dérivés de formule (VI) peuvent être obtenus par action d'une cétone R-CO-R₁ dans laquelle R et R₁ ont les mêmes significations que dans la formule (I), sur une amine H₂N-CH(R₂)R₃ dans laquelle R₂ a les mêmes significations que précédemment et R₃ a les mêmes significations que dans la formule (I).

Cette réaction s'effectue généralement soit au moyen d'un agent déshydratant tel que le tamis moléculaire 4 Å, au sein d'un solvant inerte tel que le dichlorométhane, à une température comprise entre 0°C et la température d'ébullition du milieu réactionnel ou bien par distillation azéotropique de l'eau dans un solvant aromatique tel que le toluène, en présence éventuellement d'un acide tel que l'acide paratoluènesulfonique.

Les dérivés de formule (IV) pour lesquels R₂ représente une chaîne -(CH₂)ₙ-CO-R₆, n est égal à 0, R₁₂ représente un radical cyano, phénylsulfonyle, -CXO ou -(CH₂)ₚ-CO-R₁₇, p est égal à 0 et R₁₇ représente un radical alcoxy, cycloalkyloxy, phénylalkyloxy ou cycloalkylalkyloxy peuvent également être obtenus par adaptation des méthodes décrites par S. KANEMASA et coll., Bull. Chem. Soc. Japan, 62, 869 (1982); D. A. BARR et coll., J. Chem. Soc. Perkin. Trans. I, 1550 (1989) et O. TSUGE et coll., J. Org. Chem., 53, 1384 (1988).

Les dérivés de formule (IV) pour lesquels R1 représente un radical alkyle, R₂ représente une chaîne -(CH₂)ₙ-CO-R₆, n étant égal à 0 et R₆ n'étant pas hydroxy, R₁₂ représente un radical alkyle, phénylalkyle ou -(CH₂)ₚ-CO-R₁₇ et R₁₇ n'est pas un radical hydroxy peuvent être obtenus par réaction d'un dérivé de formule : 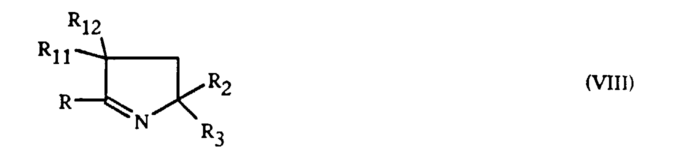 dans laquelle R et R₃ ont les mêmes significations que dans la formule (I) et R₂ a les mêmes significations que précédemment, sur un dérivé de formule :

R₁-M (IX)

dans laquelle R₁ représente un radical alkyle et R₁-M représente un organomagnésien ou un organolithien.

Cette réaction s'effectue au sein d'un solvant inerte tel que le tétrahydrofuranne ou l'éther, en présence d'un acide de Lewis tel que le trifluorure de bore ou le tétrachlorure de titane, à une température comprise entre 78°C et la température d'ébullition du milieu réactionnel.

Les dérivés de formule (VIII) pour lesquels R₃ représente un radical alkyle peuvent être obtenus par action d'un dérivé de formule (VIII) correspondant pour lequel R₃ représente un atome d'hydrogène sur un dérivé de formule Hal-R₃ dans laquelle R₃ représente un radical alkyle et Hal représente un atome d'halogène et, de préférence, iode.

Cette réaction s'effectue généralement au sein d'un solvant inerte tel que le tétrahydrofuranne ou l'éther, en présence d'une base telle que l'hydrure de sodium, le sel de sodium ou de lithium de l'hexaméthyldisilazane, à une température comprise entre -78°C et la température d'ébullition du milieu réactionnel.

Les dérivés de formule (VIII) pour lesquels R₃ représente un atome d'hydrogène peuvent être obtenus par déprotection et déshydratation d'un dérivé de formule : 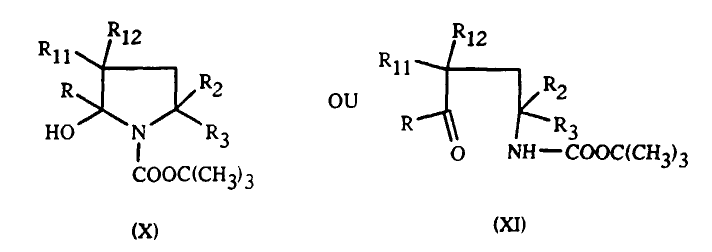 dans lesquelles R, R₂, R₃, R₁₁ et R₁₂ ont les mêmes significations que précédemment, ou d'un mélange de ces dérivés.

Ces déprotection et déshydratation s'effectuent généralement au moyen d'acide trifluoroacétique ou d'iodotriméthylsilane, au sein d'un solvant inerte tel qu'un solvant chloré (dichlorométhane par exemple), à une température voisine de 20°C.

Les dérivés de formules (X) et (XI) peuvent être obtenus par adaptation des méthodes décrites par J. EZQUERRA et coll., Tetrahedron Lett., 34, 6317 (1993), par action d'un dérivé de formule :

R-M (XII)

dans laquelle R a les mêmes significations que précédemment et R-M représente un dérivé organomagnésien, organolithien ou un cuprate, sur un dérivé de formule : 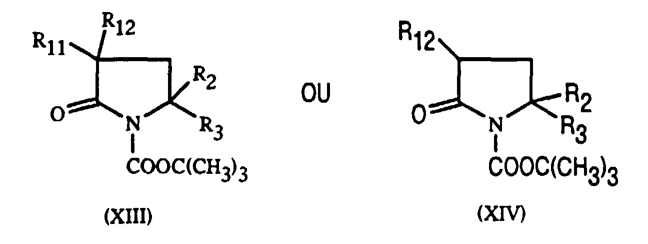 dans lesquelles R₂, R₃, R₁₁ et R₁₂ ont les mêmes significations que précédemment.

Cette réaction s'effectue au sein d'un solvant inerte tel que le tétrahydrofuranne, à une température comprise entre -78°C et 20°C.

Les dérivés de formule (XIII) pour lesquels R₁₁ représente un radical alkyle ou phénylalkyle, R₁₂ représente un radical alkyle, phénylalkyle ou -(CH₂)ₚ-CO-R₁₇ et R₁₇ n'est pas un radical hydroxy peuvent être obtenus par action d'un dérivé de formule (XIV) dans laquelle R₂ et R₃ ont les mêmes significations que précédemment, sur un dérivé de formule R₁₁-R₁₈ dans laquelle R₁₈ représente un atome d'halogène et de préférence de brome ou un reste tosyle et R₁₁ représente un radical alkyle ou phénylalkyle.

Cette réaction s'effectue généralement au sein d'un solvant inerte tel que le tétrahydrofuranne, en présence d'une base telle que le diisopropylamidure de lithium ou un sel de sodium ou de lithium de l'hexaméthyldisilazane, à une température comprise entre -78°C et la température d'ébullition du milieu réactionnel.

Les dérivés de formule (XIV) peuvent être obtenus par adaptation des méthodes décrites par N. LANGLOIS et coll., Tetrahedron Lett., 34, 2477 (1993); J. E. BALDWIN et coll., Tetrhedron, 45, 7459 (1989); J. EZQUERRA et coll., Tétrahedron, 49, 8665 (1993), par action d'un dérivé de formule : 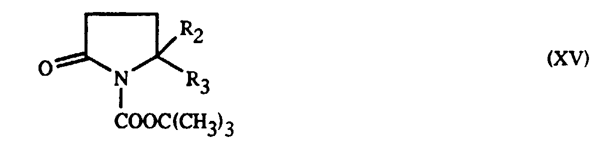 dans laquelle R₂ et R₃ ont les mêmes significations que précédemment, sur un dérivé de formule R₁₂-R₁₈ dans laquelle R₁₈ représente un atome d'halogène et de préférence de brome ou un reste tosyle et R₁₂ a les mêmes significations que précédemment.

Les dérivés de formule (XV) peuvent être obtenus par action de dicarbonate de ditert-butyle sur un dérivé de formule : 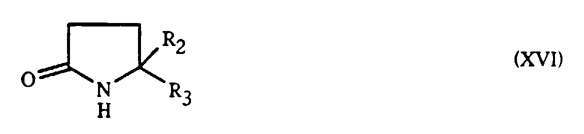 dans laquelle R₂ et R₃ ont les mêmes significations que précédemment.

Cette réaction s'effectue généralement en présence de triéthylamine ou de diméthylamino-4 pyridine, au sein d'un solvant chloré tel que le dichlorométhane, à une température voisine de 20°C.

Les dérivés de formule (XVI) sont commercialisés ou peuvent être obtenus par estérification de l'acide pyroglutamique selon les méthodes décrites par M. HOLLOSI et coll., Acta Chim. (budapest), 71, 101 (1972); B. RIGO et coll., J. Heterocycl. Chem., 25, 49 (1988); J.H. BILLMANN, J.L. RANDALL, J. Am. Chem. Soc., 66, 745 (1944); R.B. ANGIER, V.K. SMITH, J. Org. Chem., 21, 1540 (1956); J.C. SAUER, H. ADKINS, J. Am. Chem. Soc., 60, 402 (1938).

Les dérivés de formule (IV) pour lesquels R₂ représente une chaîne -(CH₂)ₙ-CO-R₆ et n est égal à 1 ou 2 peuvent être obtenus par adaptation des méthodes décrites par S. ROSSET et coll., Tetrahedron Lett., 32, 7521 (1991); T. GALLAGHER et coll., J. Chem. Soc. Perkin Trans. I, 2193 (1991) et J. F. W. KEANA, J. Org. Chem., 48, 2644 (1983).

Les dérivés de formule (IV) pour lesquels R₁ représente un atome d'hydrogène, R₂ représente une chaîne -(CH₂)ₙ-CO-R₆, n est égal à 0, R₁₂ représente un radical alkyle, phénylalkyle ou -(CH₂)ₚ-CO-R₁₇ et R₁₇ n'est pas un radical hydroxy peuvent être obtenus par hydrogénation des dérivés de formule (VIII) correspondants pour lesquels R et R₃ ont les mêmes significations que dans la formule (I) et R₁, R₂ et R₁₂ ont les mêmes significations que précédemment.

Cette hydrogénation s'effectue de préférence au moyen d'hydrogène, en présence d'un catalyseur tel que l'oxyde de platine, au sein d'un solvant inerte tel que l'éthanol, à une température voisine de 20°C, ou au moyen de borohydrure de sodium et de carbonate de potassium au sein d'un mélange eau-alcool (éthanol de préférence), à une température comprise entre 0 et 20°C.

Les dérivés de formule (III) pour lesquels R₆ et/ou R₁₇ représentent un radical hydroxy peuvent être obtenus par hydrolyse ou, selon le cas, hydrogénolyse des esters correspondants de formule (III).

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau, le méthanol ou un mélange de ces solvants, à une température comprise entre 20°C et 40°C. Lorsque l'on utilise des esters de phénylalkyle, il peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle.

Les dérivés de formule (III) pour lesquels R₂ représente une chaîne -(CH₂)ₘ-O-CO-R"₆ peuvent être obtenus par réaction d'un dérivé de formule : 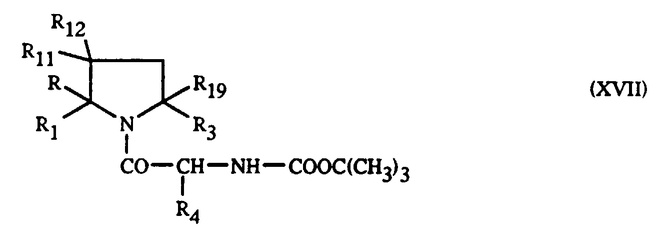 dans laquelle R, R₁, R₃, R₄, R₁₁ et R₁₂ ont les mêmes significations que dans la formule (I) et R₁₉ représente une chaîne -(CH₂)ₘ-OH, soit sur un halogénure de formule Hal-CO-R"₆ dans laquelle Hal représente un atome d'halogène et R"₆ a les mêmes significations que dans la formule (I) soit sur un anhydride de formule (R"₆CO)₂O dans laquelle R"₆ a les mêmes significations que dans la formule (I).

Cette réaction s'effectue au sein d'un solvant inerte tel qu'un solvant chloré, en présence d'une trialkylamine, à une température comprise entre 20°C et la température d'ébullition du milieu réactionnel.

Les dérivés de formule (XVII) peuvent être obtenus par réduction d'un dérivé de formule (III) correspondant pour lequel R₂ représente une chaîne -(CH₂)ₙ-CO-R₆, n est égal à 0 ou 1 et R₆ représente un radical hydroxy ou alcoxy.

Cette réaction s'effectue au sein d'un alcool (méthanol, éthanol, tertiobutanol), le tétrahydrofuranne ou un mélange de ces solvants, en présence de borohydrure de sodium ou de diborane, à une température comprise entre 20°C et la température d'ébullition du milieu réactionnel.

Les dérivés de formule (III) pour lesquels R₂ représente une chaîne -(CH₂)ₘ-O-CO-R"₆, R"₆ représente un radical -NR₉R₁₀ et R₉ représente un atome d'hydrogène peuvent également être obtenus par condensation d'un dérivé de formule (XVII) dans laquelle R₁₉ représente une chaîne -(CH₂)ₘ-OH sur un isocyanate de formule R₁₀NCO.

Cette réaction s'effectue au sein d'un solvant inerte tel qu'un solvant chloré, le tétrahydrofuranne ou le N,N-diméthyl-formamide, éventuellement en présence d'un quantité catalytique d'un alcoxyde de métal alcalin, à une température comprise entre 20°C et la température d'ébullition du milieu réactionnel.

Les dérivés de formule (III) pour lesquels R₂ représente un radical -(CH₂)ₘ-NR₉R₁₀ peuvent être obtenus par action d'une amine HNR₉R₁₀ dans laquelle R₉ et R₁₀ ont les mêmes significations que dans la formule (I) sur un dérivé de formule (XVII) dans laquelle R₁₉ représente un radical -(CH₂)ₘ-O-SO₂-CH₃.

Cette réaction s'effectue généralement soit en présence d'un large excès d'amine, à une température comprise entre 0 et 10°C soit lorsque l'on utilise le chlorhydrate de l'amine, au sein d'un solvant chloré, en présence d'une trialkylamine, à une température comprise entre 20°C et la température d'ébullition du milieu réactionnel.

Les dérivés de formule (XVII) dans laquelle R₁₉ représente un radical -(CH₂)ₘ-O-SO₂-CH₃ peuvent être obtenus par réaction d'un dérivé de formule (XVII) correspondant pour lequel R₁₉ représente un radical -(CH₂)ₘ-OH avec le chlorure de méthanesulfonyle.

Cette réaction s'effectue généralement au sein d'un solvant inerte tel que l'acétonitrile ou le chlorure de méthylène, en présence de triéthylamine, à une température comprise entre 0°C et la température d'ébullition du milieu réactionnel.

Les dérivés de formule (III) pour lesquels R₂ représente un radical -(CH₂)ₙ-CO-R₆, R₆ représente un radical hydroxy peuvent être obtenus par saponification d'un dérivé de formule (III) correspondant pour lequel R₆ représente un radical alcoxy.

Cette réaction s'effectue au sein de solvants inertes tels que le méthanol, le dioxane, le tétrahydrofuranne et l'eau ou un mélange de ces solvants, en présence d'une base telle que la soude, la potasse, l'hydroxyde de lithium, à une température comprise entre 0 et 25°C.

Les dérivés de formule (III) pour lesquels R₂ représente un radical -(CH₂)ₙ-CO-R₆ et R₆ représente un radical alcoxy, cycloalcoxy ou cycloalkylalkyloxy peuvent être obtenus par estérification des dérivés de formule (III) correspondants pour lesquels R₂ représente un radical -(CH₂)ₙ-CO-R₆ et R₆ représente un radical hydroxy.

Cette réaction s'effectue de préférence au moyen d'un alcool R₂₀-OH dans lequel R₂₀ représente un radical alkyle, cycloalkyle, cycloalkylalkyle, en présence de chlorure de tosyle, au sein de la pyridine, à une température comprise entre 0 et 25°C.

Les dérivés de formule (III) pour lesquels R₂ représente un radical -(CH₂)ₙ-CO-R₆ et R₆ représente un radical phényle peuvent être obtenus par action d'un dérivé de formule (III) correspondant pour lequel R₂ représente un radical -(CH₂)ₙ-CO-R₆ et R₆ représente un radical alcoxy avec le bromure de phénylmagnésium.

Cette réaction s'effectue de préférence au sein d'un solvant inerte tel que le tétrahydrofuranne ou l'éther éthylique, à une température comprise entre -70°C et la température d'ébullition du mélange réactionnel.

Les dérivés de formule (III) pour lesquels R₂ représente un radical oxazolinyle éventuellement substitué peuvent être obtenus par action d'un dérivé de formule (III) correspondant pour lequel R₂ représente un radical -(CH₂)ₙ-CO-R₆, n est égal à 0 et R₆ représente un radical hydroxy, sur I'amino-2 éthanol éventuellement substitué par un ou plusieurs radicaux alkyle.

Cette réaction s'effectue au sein d'un solvant inerte tel que le toluène en éliminant l'eau formée, à la température d'ébullition du milieu réactionnel.

Les dérivés de formule (III) pour lesquels R₂ représente un radical alkyl-3 oxadiazolyle peuvent être obtenus par action d'un dérivé de formule (III) correspondant pour lequel R₂ représente un radical -(CH₂)ₙ-CO-R₆, n est égal à 0 et R₆ représente un radical alcoxy sur une alkylamidoxime.

Cette réaction s'effectue au sein d'un solvant inerte tel que le tétrahydrofurane, en présence d'hydrure de sodium, à une température comprise entre 25°C et la température d'ébullition du milieu réactionnel.

Les dérivés de formule (III) pour lesquels R₁₂ représente un radical -CHX-O-CO-alk peuvent être obtenus par réaction des dérivés de formule (III) dans laquelle R₁₂ représente un radical -CHX-OH avec un dérivé de formule Hal-CO-alk dans laquelle Hal représente un atome d'halogène (brome et chlore de préférence) et alk représente un radical alkyle ou un dérivé de formule (alk-CO)₂O dans laquelle alk représente un radical alkyle.

Cette réaction s'effectue au sein d'un solvant inerte tel qu'un solvant chloré, en présence d'une base organique telle qu'une trialkylamine, à une température comprise entre 20°C et la température d'ébullition du milieu réactionnel.

Les dérivés de formule (III) pour lesquels R₁₂ représente un radical -CHX-OH peuvent être obtenus par réduction des composés de formule (III) dans laquelle R₁₂ représente un radical -CXO.

Cette réduction s'effectue généralement au moyen de borohydrure de sodium ou de diborane, au sein d'un solvant tel qu'un alcool (méthanol, éthanol, tertiobutanol par exemple), le tétrahydrofuranne ou un mélange de ces solvants, à une température comprise entre -20°C et la température d'ébullition du milieu réactionnel.

Les dérivés de formule (III) pour lesquels R₁₂ représente un radical -NH-CO-alk peuvent être obtenus par réaction des composés de formule (III) dans laquelle R₁₂ représente un radical NH₂ avec un dérivé de formule Hal-CO-alk dans laquelle Hal représente un atome d'halogène (brome et chlore de préférence) ou un dérivé de formule (alk-CO)₂O.

Cette réaction s'effectue au sein d'un solvant inerte tel qu'un solvant chloré, en présence d'une base organique telle qu'une trialkylamine, à une température comprise entre 20°C et la température d'ébullition du milieu réactionnel.

Les dérivés de formule (III) pour lesquels R₁₂ représente un radical -NH₂ peuvent être obtenus par réaction des composés de formule (III) dans laquelle R₁₂ représente un radical -(CH₂)ₚ-CO-R₁₇, p est égal à 0 et R₁₇ représente un radical hydroxy avec le diphénylphosphorylazide suivie de l'hydrolyse de l'intermédiaire obtenu.

Cette réaction s'effectue généralement en présence de triéthylamine, au sein d'un solvant tel qu'un alcool (méthanol, éthanol, tertiobutanol par exemple), le tétrahydrofuranne ou un mélange de ces solvants, à une température comprise entre -20°C et la température d'ébullition du milieu réactionnel. L'hydrolyse s'effectue généralement par addition d'eau ou d'un acide au milieu réactionnel, à une température comprise entre -20°C et la température d'ébullition du milieu réactionnel.

Les dérivés de formule (III) pour lesquels R₂ représente une chaîne -(CH₂)ₙ-CO-R₆ et R₆ représente un radical -NR₉R₁₀ peuvent être obtenus par action d'un dérivé de formule (III) correspondant pour lequel R₆ représente un radical hydroxy ou un dérivé réactif de cet acide sur une amine de formule HNR₉R₁₀ dans laquelle R₉ et R₁₀ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (N,N-diméthyl-formamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les anilines éventuellement substituées sont commercialisées ou peuvent être obtenues par application ou adaptation des méthodes décrites par R. SCHRÖTER, Methoden der organischen Chemie, Houben Weil, Band XI/1, p 360; G.J. ESSELEN et coll., J. Am. Chem. Soc., 36, 322 (1914); G. ADRIANT et coll., Bull. Soc. Chim. Fr, 1511 (1970); W.A. JACOBS et coll., J. Am. Chem. Soc., 39, 2438 (1917) et J. Am. Chem. Soc., 39, 1438 (1917) et dans les exemples.

Les composés de formule (I) pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène peuvent également être préparés par action d'un dérivé de formule (II), sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène.

Cette réaction s'effectue généralement au sein d'un solvant inerte tel que le tétrahydrofuranne, le N,N-diméthyl-formamide, un solvant chloré (chloroforme, dichloro-1,2 éthane par exemple), un solvant aromatique (benzène, toluène par exemple), à une température comprise entre 10°C et la température d'ébullition du solvant.

Les phénylisocyanates sont commercialisés ou peuvent être obtenus par application ou adaptation des méthodes décrites par R. RICHTER et coll., The Chemistry of Cyanate and their thio derivatives, S. PATAI, part 2, Wiley New York (1977) et dans les exemples.

Les composés de formule (I) pour lesquels R₅ représente un radical phényle éventuellement substitué, naphtyle, indolyle, quinolyle peuvent être préparés par action d'un dérivé de formule (II) sur un acide de formule HOOC-R₅ dans laquelle R₅ a les mêmes significations que précédemment ou un dérivé réactif de cet acide.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (N,N-diméthyl-formamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels R₅ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH, -CX=N-O-alk-COOH et/ou R₁₂ représente un radical -(CH₂)ₚ-COOH, R, R₁, R₂, R₃, R₄, R₆, R₁₁ et R₁₂ sont définis comme dans la formule (I) peuvent également être préparés par hydrolyse ou, selon le cas, hydrogénolyse des esters correspondants de formule (I).

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau, le méthanol ou un mélange de ces solvants, à une température comprise entre 20°C et 40°C. Lorsque l'on utilise des esters de phénylalkyle, il est peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle. Lorsqu'on utilise des esters de tert-butyle, il est avantageux d'effectuer l'hydrolyse au moyen d'un acide tel que l'acide trifluoroacétique.

Les composés de formule (I) pour lesquels R₅ représente un radical phénylamino dont le noyau phényle est substitué par un radical hydroxyiminoalkyle ou alcoxyiminoalkyle peuvent également être préparés par action du dérivé acylé de formule (I) correspondant sur un dérivé de formule :

H₂N-OR₂₁ (XVIII)

dans laquelle R₂₁ représente un atome d'hydrogène ou un radical alkyle.

Cette réaction s'effectue généralement au sein d'un solvant inerte tel qu'un alcool (méthanol, éthanol par exemple), l'eau ou un mélange de ces solvants, à la température d'ébullition du solvant et éventuellement en présence d'une base telle que la pyridine.

Les composés de formule (I) pour lesquels R₂ représente une chaîne -(CH₂)ₙ-CO-R₆, R₆ n'étant pas un radical hydroxy_{,} R₁₂ représente un radical alkyle, phénylalkyle, phénylsulfonyle, cyano, -CXO ou -(CH₂)ₚ-CO-R₁₇, R₁₇ n'étant pas un radical hydroxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène peuvent également être préparés par action d'un dérivé de formule (IV) dans laquelle R₂ et R₁₂ ont les mêmes significations que précédemment sur un acide de formule : 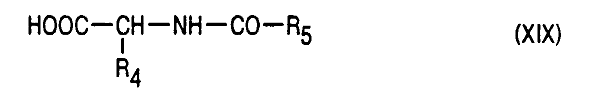 dans laquelle R₅ a les mêmes significations que ci-dessus ou un dérivé réactif de cet acide et R₄ a les mêmes significations que dans la formule (I).

Cette réaction s'effectue de préférence en présence d'un agent de condensation utilisé en chimie peptidique tel qu'un carbodimide au sein d'un solvant tel que l'acétonitrile, le tétrahydrofuranne ou un solvant chloré ou au moyen de chlorure de thionyle dans le dichlorométhane à une température comprise entre 10° C et la température d'ébullition du solvant.

Les acides de formule (XIX) peuvent être obtenus par application ou adaptation de la méthode décrite par J.R. JOHNSON et coll., J. Am. Chem. Soc., 69, 2370 (1947) ou, pour les composés pour lesquels R₅ représente un radical phénylamino éventuellement substitué, par action d'un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène sur un dérivé de formule : 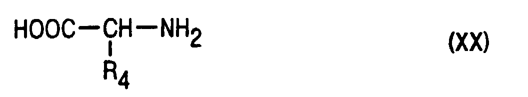 dans laquelle R₄ a les mêmes significations que dans la formule (I).

Cette réaction s'effectue généralement en solution aqueuse en présence d'une base telle qu'un bicarbonate de métal alcalin ou dans le dioxanne aqueux, à une température voisine de 20°C.

Les composés de formule (I) pour lesquels R₁₂ représente un radical -CX=N-O-alk-COOX ou -CX=NOH peuvent être obtenus par condensation d'un dérivé de formule (XVIII) dans laquelle R₂₁ représente un atome d'hydrogène ou alk-COOX sur un composé de formule (I) correspondant pour lequel R₁₂ représente un radical -CXO.

Cette réaction s'effectue généralement au sein d'un solvant inerte tel qu'un alcool (méthanol, éthanol par exemple), l'eau ou un mélange de ces solvants, éventuellement en présence d'une base telle que la pyridine, à une température comprise entre 20°C et la température d'ébullition du milieu réactionnel.

Il est entendu pour l'homme de métier que, la mise en oeuvre des procédés selon l'invention décrits précédemment, il peut être nécessaire afin d'éviter des réactions secondaires d'introduire des groupes protecteurs des fonctions amine, alcool, acide, cétone tels que ceux décrits par T. W. GREENE, protective groups in organic synthesis, John Wiley and Sons, New York. Par exemple les fonctions amine peuvent être bloquées sous forme de carbamates de tert-butyle ou de méthyle puis régénérées au moyen d'iodotriméthylsilane ou de carbamates de benzyle puis régénérées par hydrogénation après avoir mis en oeuvre le procédé selon l'invention. Les fonctions alcool peuvent par exemple être bloquées sous forme de benzoate puis régénérées par hydrolyse en milieu alcalin après avoir mis en oeuvre le procédé selon l'invention. Les fonctions cétone peuvent être bloquées sous forme de dioxolanne-1,3 puis régénérées au moyen de d'un mélange acide chlorhydrique-acide acétique.

Les énantiomères des composés de formule (I) contenant au moins un site asymétrique peuvent être obtenus par dédoublement des racémiques par exemple par chromatographie sur colonne chirale ou par synthèse à partir des précurseurs chiraux.

Les composés de formule (I) peuvent être purifiés par les méthodes connues habituelles, par exemple par cristallisation, chromatographie ou extractions.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalino-terreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la dépression, de la neurodégénération, des attaques de panique, de la maladie de Parkinson, de la diskynésie tardive, du syndrome du colon irritable, de la pancréatite aiguë, des ulcères, des désordres de la motilité intestinale, de certaines tumeurs sensibles à la CCK, comme régulateur de l'appétit, dans le sevrage aux traitements chroniques et abus d'alcool ou de médicaments et comme constricteur de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la Cl₅₀ des composés de formule (I) est généralement inférieure ou égale à 2000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 13-16 (1989); REYFELD et coll., Am. J. Physiol., 240, G255-266 (1981); BEINFELD et coll., Neuropeptides, 3, 411-427 (1983).

Les composés de formule (I) présentent une toxicité faible. Leur DL₅₀ est généralement supérieure à 40 mg/kg par voie sous cutanée chez la souris.

D'un intérêt particulier sont les composés de formule (I) pour lesquels R représente un radical phényle éventuellement substitué par un ou plusieurs atomes d'halogène, R₁ représente un atome d'hydrogène, R₂ représente une chaîne -(CH₂)ₙ-CO-R₆, R₃ représente un atome d'hydrogène, R₄ représente un atome d'hydrogène, R₅ représente un radical phénylamino dont le noyau phényle est sustitué par un ou plusieurs substituants choisis parmi les radicaux carboxy, -S-alk-COOX, -alk-COOX et tétrazolyl-5, R₆ représente un radical hydroxy ou alcoxy, R₁₁ représente un atome d'hydrogène, R₁₂ représente un radical phénylsulfonyle, -(CH₂)ₚ-CO-R₁₇, R₁₇ représente un radical hydroxy, phényle ou -NR₉R₁₀, p est égal à 0, n est égal à 0 et X représente un atome d'hydrogène, leurs sels et leurs isomères.

D'un intérêt particulier sont les composés suivants :
- acide {[(tert-butoxycarbonyl-2 phényl-5 phénylsulfonyl-4 pyrrolidinyl-1)-2 oxo-2 éthyl]-3 uréido}-3 benzoïque-(2RS,4SR,5RS),
- {[(carboxyméthylthio-3 phényl)-3 uréido]-2 acétyl}-1 phényl-5 pyrrolidinedicarboxylate-2,4 acide de (2) tert-butyle-(2RS,4SR,5SR),
- acide ({[tert-butoxycarbonyl-2 (fluoro-2 phényl)-5 phénylsulfonyl-4 pyrrolidinyl-1]-2 oxo-2 éthyl}-3 uréido)-3 benzoïque-(2RS,4SR,5RS),
- acide ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoïque(2RS,4SR,5RS),
- {[(carboxyméthyl-3 phényl)-3 uréido]-2 acétyl}-1 fluoro-2 phényl)-5 pyrrolidinedicarboxylate-2,4 acide de (2) tert-butyle-(2RS,4SR,5SR),
- acide {[(carboxyméthyl-3 phényl)-3 uréido]-2 acétyl}-1 (fluoro-2 phényl)-5 pyrrolidinedicarboxylate-2,4 acide de (2) tert-butyle-(2RS,4SR,5SR),
- acide {[(tert-butoxycarbonyl-2 phényl-5 (pyrrolidinyl-1) carbonyl-4 pyrrolidinyl-1)-2 oxo-2 éthyl]-3 uréido}-3 benzoïque-(2RS,4SR,5SR),
- acide {[(tert-butoxycarbonyl-2 diméthylcarbamoyl-4 phényl-5 pyrrolidinyl-1)-2 oxo-2 éthyl]-3 uréido}-3 benzoïque-(2RS,4SR,5SR),
- acide ({[benzoyl-4 tert-butoxycarbonyl-2 (fluoro-2 phényl)-5 pyrrolidinyl-1]-2 oxo-2 éthyl}-3 uréido)-3 benzoïque-(2RS-4SR,5SR),
- acide [({[tert-butoxycarbonyl-2 (fluoro-2 phényl)-5 phénylsulfonyl-4 pyrrolidinyl-1-(2R*,45*,5R*)]-2 oxo-2 éthyl}-3 uréido)-3 phényl]-2 propionique,
- acide [({[tert-butoxycarbonyl-2 (fluoro-2 phényl)-5 phénylsulfonyl-4 pyrrolidinyl-1-(2R*,45*,5R*)]-2 oxo-2 éthyl}-3 uréido)-3 phényl]-2 méthoxy-2 acétique-(S),
- acide {[(tert-butoxycarbonyl-2 (fluoro-2 phényl)-5 morpholinocarbonyl-4 pyrrolidinyl-1)-2 oxo-2 éthyl]-3 uréido}-3 benzoïque-(2RS,4SR,5SR),
- acide {[(tert-butoxycarbonyl-2 diéthylaminocarbonyl-4 (fluoro-2 phényl)-5 pyrrolidinyl-1)-2 oxo-2 éthyl]-3 uréido}-3 benzoïque-(2RS,4SR,5SR),
- [({[tert-butoxycarbonyl-2 (fluoro-2 phényl)-5 phénylsulfonyl-4 pyrrolidinyl-1]-2 oxo-2 éthyl}-3 uréido)-3 phényl]-5 tétrazole-(2RS,4SR,5RS),
- acide ({[(fluoro-2 phényl)-5 isobutylcarbamoyl-2 phénylsulfonyl-4 pyrrolidinyl-1]-2 oxo-2 éthyl}-3 uréido)-3 phénylacétique-(2RS,4SR,5RS),
- acide-(-) ({[tert-butoxycarbonyl-2 (fluoro-2 phényl)-5 phénylsulfonyl-4 pyrrolidinyl-1]-2 oxo-2 éthyl}-3 uréido)-3 benzoïque-(2R*,4S*,5R*),
- (+)-{[(carboxyméthyl-3 phényl)-3 uréido]-2 acétyl}-1 (fluoro-2 phényl)-5 pyrrolidinedicarboxylate-2,4 acide de (2) tert-butyle-(2R*,4S*,5S*),
leurs sels et leurs isomères.

Les exemples suivants illustrent l'invention sans la limiter.

### Exemple 1

A A une solution de 0,54 g de {[(benzyloxycarbonyl-3 phényl)-3 uréido]-2 acétyl}-1 benzyl-4 phényl-5 pyrrolidinecarboxylate-2 de tert-butyle(2S,4R,5R) dans 25 cm³ d'acétate d'éthyle, on ajoute 0,1 g de palladium à 10% sur charbon. La suspension est agitée pendant dix-huit heures à une température voisine de 20°C sous atmosphère d'hydrogène (130 kPa). Le catalyseur est séparé par filtration sur célite et le filtrat est concentré à sec sous pression réduite. Le résidu est chromatographié sur silice [éluant : dichlorométhane-méthanol (99,5-0,5 en volumes)]. les fractions contenant le produit attendu sont réunies et concentrées sous pression réduite. Le résidu est remis en suspension dans 10 cm³ d'oxyde de diisopropyle, filtré et séché sous vide à une température voisine de 40°C. On obtient ainsi 0,36 g d'acide {[(benzyl-4 tert-butoxycarbonyl-2 phényl-5 pyrrolidinyl-1)-2 oxo-2 éthyl]-3 uréido}-3 benzoïque-(2S,4R,5R) (Rf = 0,25; éluant : chlorure de méthylène-méthanol (90/10)).
B Le {[(benzyloxycarbonyl-3 phényl)-3 uréido]-2 acétyl}-1 benzyl-4 phényl-5 pyrrolidinecarboxylate-2 de tert-butyle-(2S,4R,5R) peut être obtenu de la manière suivante : à une solution de 0,32 g de benzyl-4 phényl-5 pyrrolidinecarboxylate-2 de tert-butyle et de 0,31 g d'acide [(benzyloxycarbonyl-3 phényl)-3 uréido]-2 acétique dans 25 cm³ d'acétonitrile, on ajoute à une température voisine de 20°C, 0,2 g de N,N'-dicyclohexylcarbodiimide. Le mélange réactionnel est agité pendant soixante-douze heures à une température voisine de 20°C, filtré, rincé par 5 cm³ d'acétonitrile et concentré sous pression réduite. On obtient ainsi 0,54 g de {[(benzyloxycarbonyl-3 phényl)-3 uréido]-2 acétyl}-1 benzyl-4 phényl-5 pyrrolidinecarboxylate-2 de tert-butyle-(2S,4R,5R) sous forme d'une meringue utilisée telle quelle dans les synthèses ultérieures.
C Le benzyl-4 phényl-5 pyrrolidinecarboxylate-2 de tert-butyle peut être préparé de la manière suivante : à une solution de 1,65 g benzyl-4 phényl-5 Δ5-pyrrolinecarboxylate-2 de tert-butyle dans 40 cm³ d'éthanol, on ajoute 0,15 g d'oxyde de platine. La suspension est agitée pendant huit heures à une température voisine de 20°C sous atmosphère d'hydrogène (130 kPa). Le catalyseur est séparé par filtration sur célite et le filtrat est concentré à sec sous pression réduite. Le résidu est chromatographié sur silice [éluant : dichlorométhane-méthanol (100-0 puis 99,5-0,5 en volumes)]. Les fractions contenant le produit attendu sont réunies et concentrées sous pression réduite. On obtient ainsi 0,32 g de benzyl-4 phényl-5 pyrrolidinecarboxylate-2 de tert-butyle sous forme d'une laque utilisée telle quelle dans les synthèses ultérieures.
D Le benzyl-4 phényl-5 Δ5-pyrrolinecarboxylate-2 de tert-butyle peut être préparé de la manière suivante : à une solution de 2,4 g de benzyl-4 tert-butoxycarbonyl-1 hydroxy-5 phényl-5 pyrrolidinecarboxylate-2 de tert-butyle dans 35 cm³ de dichlorométhane, on ajoute, à une température voisine de 20°C, 2,15 cm³ d'acide trifluoroacétique. Le mélange réactionnel est agité pendant vingt heures à une température voisine de 20°C, puis on ajoute 100 cm³ de dichlorométhane. La phase organique est lavée par deux fois 80 cm³ d'une solution aqueuse saturée en hydrogénocarbonate de sodium puis 100 cm³ d'eau distillée, séchée sur sulfate de magnésium et concentrée sous pression réduite. Le résidu est purifié par chromatographie sur silice (éluant : dichlorométhane). Les fractions contenant le produit attendu sont réunies et concentrées sous pression réduite. On obtient ainsi 2 g de benzyl-4 phényl-5 Δ5-pyrrolinecarboxylate-2 de tert-butyle sous forme d'une huile utilisée telle quelle dans les synthèses ultérieures.
E Le benzyl-4 tert-butoxycarbonyl-1 hydroxy-5 phényl-5 pyrrolidinecarboxylate-2 de tert-butyle peut être préparé de la manière suivante : à une suspension de 0,8 g de magnésium dans 20 cm³ de tétrahydrofuranne, on ajoute en quarante minutes, à une température comprise entre 20 et 30°C, une solution de 2,85 cm³ de bromobenzène dans 60 cm³ de tétrahydrofuranne. Le milieu réactionnel est encore agité à une température voisine de 24°C pendant vingt minutes puis on ajoute en vingt minutes une solution de 8,4 g de benzyl-4 tert-butoxycarbonyl-1 oxo-5 pyrrolidinecarboxylate-2 de tert-butyle dans 80 cm³ de tétrahydrofuranne maintenue à une température voisine de 5°C. Le milieu réactionnel est encore agité pendant vingt heures à une température voisine de 20°C. Le milieu réactionnel est ensuite versé dans 150 cm³ d'une solution aqueuse de chlorure d'ammonium à 10%. La phase aqueuse est extraite par trois fois 100 cm³ d'oxyde de diéthyle. Les phases organiques sont réunies et lavées par 100 cm³ d'eau, séchées sur sulfate de magnésium et concentrées sous pression réduite à une température voisine de 50°C. Le résidu est purifié par chromatographie sur silice (éluant : dichlorométhane). Les fractions contenant le produit attendu sont réunies et concentrées sous pression réduite. On obtient ainsi 2,5 g de benzyl-4 tert-butoxycarbonyl-1 hydroxy-5 phényl-5 pyrrolidinecarboxylate-2 de tert-butyle sous forme d'une meringue utilisée telle quelle dans les synthèses ultérieures.
F Le benzyl-4 tert-butoxycarbonyl-1 oxo-5 pyrrolidinecarboxylate-2 de tert-butyle peut être préparé de la manière suivante : à une solution de 5,15 cm³ de diisopropylamine dans 50 cm³ de tétrahydrofuranne refroidie à une température voisine de -75°C, on ajoute en quinze minutes 18,8 cm³ d'une solution 1,6 M de butyl lithium dans l'hexane. Le milieu réactionnel est agité pendant trente minutes à une température voisine de -78°C puis on ajoute en vingt-cinq minutes une solution de 8,55 g de tert-butoxycarbonyl-1 oxo-5 pyrrolidinecarboxylate-2 de tert-butyle-(S) dans 60 cm³ de tétrahydrofuranne. Le milieu réactionnel est agité à une température voisine de -75°C pendant une heure, puis on ajoute en dix minutes une solution de 5,13 cm³ de bromure de benzyle dans 30 cm³ de tétrahydrofuranne. Le milieu réactionnel est encore agité pendant quatre heures à une température voisine de -78°C, puis on ajoute successivement 200 cm³ d'une solution aqueuse saturée en chlorure d'ammonium et 100 cm³ d'oxyde de diéthyle. La phase organique est séparée par décantation, lavée par deux fois 100 cm³ d'eau distillée, séchée sur sulfate de magnésium et concentrée sous pression réduite. Le résidu est remis en suspension dans 70 cm³ d'éther de pétrole, filtré et séché sous pression réduite à 30°C. On obtient ainsi 5,3 g de benzyl-4 tert-butoxycarbonyl-1 oxo-5 pyrrolidinecarboxylate-2 de tert-butyle fondant à 125°C.

Le tert-butoxycarbonyl-1 oxo-5 pyrrolidinecarboxylate-2 de tert-butyle-(S) peut être obtenu selon la méthode décrite par J. ACKERMANN et M. MATTHES, Helv. Chim. Acta, 73, 122-132, (1990).
G L'acide [(benzyloxycarbonyl-3 phényl)-3 uréido]-2 acétique peut être préparé de la manière suivante : à une solution de 3,97 g de glycine et de 14,62 g de carbonate de potassium dans 90 cm³ d'eau, on ajoute en quinze minutes 13,4 g d'isocyanato-3 benzoate de benzyle en solution dans 70 cm³ de dioxanne-1,4. Le mélange réactionnel est agité pendant quatre heures à une température voisine de 20°C puis acidifié à pH 1 avec une solution aqueuse 4N d'acide chlorhydrique. Le produit insoluble est séparé par filtration, lavé avec trois fois 50 cm³ d'eau et séché à l'air. On obtient ainsi 13 g d'acide [(benzyloxycarbonyl-3 phényl)-3 uréido]-2 acétique utilisé tel quel dans les synthèses ultérieures.
H L'isocyanato-3 benzoate de benzyle peut être préparé de la manière suivante : à une suspension de 2 g de charbon dans un mélange de 12,5 cm³ de chloroformiate de trichlorométhyle et de 50 cm³ de toluène, on ajoute en quinze minutes à une température voisine de -25°C, une solution d'amino-3 benzoate de benzyle dans 150 cm³ de toluène préparée en neutralisant 27 g de chlorhydrate d'amino-3 benzoate de benzyle par 14,4 cm³ de triéthylamine dans 150 cm³ de toluène et filtrant la suspension ainsi obtenue. Le mélange réactionnel est agité à une température voisine de 25°C pendant deux heures, puis chauffé à une température voisine de 110°C pendant deux heures. Après refroidissement à une température voisine de 25°C, le milieu réactionnel est dégazé par barbottage d'azote, filtré sur papier filtre et concentré sous pression réduite à une température voisine de 25°C. On obtient ainsi 27 g d'isocyanato-3 benzoate de benzyle sous forme d'une huile utilisée telle quelle dans les synthèses ultérieures.

L'amino-3 benzoate de benzyle peut être préparé selon la méthode décrite par H.A. SHONLE et coll., J. Amer. Chem. Soc., 43, 361 (1921).

### Exemple 2

A A une solution de 1,25 g de {[(benzyloxycarbonyl-3 phényl)-3 uréido]-2 acétyl}-1 phényl-5 pyrrolidinedicarboxylate-2,4 de (2) tert-butyle et de (4) méthyle-(2RS,4RS,5SR) dans 100 cm³ d'acétate d'éthyle, on ajoute 0,2 g de palladium à 5% sur charbon. La suspension est agitée pendant quatre heures à une température voisine de 25°C sous atmosphère d'hydrogène (130 kPa). Le catalyseur est séparé par filtration sur célite, rincé par deux fois 10 cm³ de méthanol et le filtrat est concentré à sec sous pression réduite. Le résidu est cristallisé dans l'acétate d'éthyle, repris dans 25 cm³ d'une solution aqueuse décinormale d'acide chlorhydrique, filtré, lavé par deux fois 10 cm³ d'eau et séché sous vide à une température voisine de 40°C. On obtient ainsi 0,9 g d'acide {[(tert-butoxycarbonyl-2 méthoxycarbonyl-4 phényl-5 pyrrolidinyl-1)-2 oxo-2 éthyl]-3 uréido}-3 benzoïque-(2RS,4RS,5SR) fondant à 243°C.
B Le {[(benzyloxycarbonyl-3 phényl)-3 uréido]-2 acétyl}-1 phényl-5 pyrrolidinedicarboxylate-2,4 de (2) tert-butyle et de (4) méthyle-(2RS,4RS,5SR) peut être préparé de la manière suivante : à une solution de 3,06 g de phényl-5 pyrrolidinedicarboxylate-2,4 de (2) tert-butyle et de (4) méthyle-(2RS,4RS,5SR) et de 3,3 g d'acide [(benzyloxycarbonyl-3 phényl)-3 uréido]-2 acétique dans 100 cm³ d'acétonitrile, on ajoute à une température voisine de 20°C, 2,3 g de N,N'-dicyclohexylcarbodiimide. Le mélange réactionnel est agité pendant vingt heures à une température voisine de 20°C, concentré sous pression réduite, repris dans 100 cm³ d'acétate d'éthyle et filtré. Le précipité est rincé par deux fois 25 cm³ d'acétate d'éthyle puis le filtrat est concentré sous pression réduite. Le résidu est purifié par chromatographie sur silice [éluant : cyclohexane-acétate d'éthyle (70-30 en volumes)]. Les fractions contenant le produit attendu sont réunies et concentrées sous pression réduite. On obtient ainsi 2 g de {[(benzyloxycarbonyl-3 phényl)-3 uréido]-2 acétyl}-1 phényl-5 pyrrolidinedicarboxylate-2,4 de (2) tert-butyle et de (4) méthyle-(2RS,4RS,5SR) sous forme d'une huile utilisée telle quelle dans les synthèses ultérieures.
C Le phényl-5 pyrrolidinedicarboxylate-2,4 de (2) tert-butyle et de (4) méthyle-(2RS,4RS,5SR) peut être préparé de la manière suivante : à une suspension de 5 g d'acétate d'argent dans une solution de 1,8 cm³ d'acrylate de méthyle et de 4,5 g de benzylidèneaminoacétate de tert-butyle dans 200 cm³ d'acétonitrile, on ajoute goutte à goutte 2,8 cm³ de triéthylamine à une température voisine de 20°C. Le mélange réactionnel est agité pendant quatre heures à une température voisine de 20°C, puis versé dans 200 cm³ d'une solution aqueuse saturée en chlorure d'ammonium. La phase aqueuse est filtrée et extraite par trois fois 100 cm³ d'acétate d'éthyle. Les extraits sont réunis, lavés par 100 cm³ d'eau, séchés sur sulfate de magnésium, filtrés et concentrés sous pression réduite. Le résidu est purifié par chromatographie sur silice [éluant : cyclohexane-acétate d'éthyle (70-30 en volumes)]. Les fractions contenant le produit attendu sont réunies et concentrées sous pression réduite. On obtient ainsi 4,5 g de phényl-5 pyrrolidinedicarboxylate-2,4 de (2) tert-butyle et de (4) méthyle-(2RS,4RS,5SR) sous forme d'une huile utilisée telle quelle dans les synthèses ultérieures.
D Le benzylidèneaminoacétate de tert-butyle peut être préparé de la manière suivante : à une suspension de 3 g de tamis moléculaire 4Å dans une solution de 3,35 g de chlorhydrate de glycinate de tert-butyle dans 2,05 cm³ de benzaldéhyde et 50 cm³ de dichlorométhane, on ajoute goutte à goutte 2,8 cm³ de triéthylamine à une température voisine de 20°C. Le mélange réactionnel est agité pendant soixante-douze heures à une température voisine de 20°C et concentré sous pression réduite. Le résidu est repris dans 50 cm³ d'oxyde de diéthyle, filtré et le précipité rincé par deux fois 50 cm³ d'oxyde de diéthyle. Le filtrat est concentré sous pression réduite. On obtient ainsi 4,4 g de benzylidèneaminoacétate de tert-butyle sous forme d'une huile utilisée telle quelle dans les synthèses ultérieures.

### Exemple 3

A une solution de 1 g d'acide {[(tert-butoxycarbonyl-2 méthoxycarbonyl-4 phényl-5 pyrrolidinyl-1)-2 oxo-2 éthyl]-3 uréido}-3 benzoïque-(2RS,4RS,5SR) dans 50 cm³ de méthanol, on ajoute 0,21 g d'hydroxyde de potassium en solution dans 15 cm³ d'eau distillée. Le mélange réactionnel est agité pendant soixante-douze heures à une température voisine de 20°C, puis concentré sous pression réduite. Le résidu est dilué avec 100 cm³ d'eau, lavé par 2 fois 100 cm³ d'acétate d'éthyle, acidifié à pH 1 avec une solution aqueuse 4N d'acide chlorhydrique. Le précipité qui apparait est séparé par filtration, lavé par deux fois 50 cm³ d'eau distillée, séché sous pression réduite à une température voisine de 40°C. Le produit brut obtenu est purifié par chromatographie sur silice [éluant : dichlorométhane-méthanol (90-10 en volumes)]. Les fractions contenant le produit attendu sont réunies et concentrées à sec sous pression réduite. On obtient, après cristallisation dans une solution aqueuse 0,1 N d'acide chlorhydrique, 0,65 g de {[(carboxy-3 phényl)-3 uréido]-2 acétyl}-1 phényl-5 pyrrolidinedicarboxylate-2,4 acide de (2) tert-butyle-(2RS,4SR,5SR) fondant à 193°C.

### Exemple 4

A On opère d'une manière analogue à celle décrite à l'exemple 2 B, mais à partir de 1,7 g de diméthylcarbamoyl-4 phényl-5 pyrrolidinecarboxylate-2 de tert-butyle-(2RS,4RS,5SR), de 1,15 g d'acide (méthyl-3 phényl)-3 uréido]-2 acétique et de 1,15 g de N,N'-dicyclohexylcarbodiimide dans 50 cm³ d'acétonitrile. Après traitement, on obtient 2,2 g de diméthylcarbamoyl-4 {[(méthyl-3 phényl)-3 uréido]-2 acétyl}-1 phényl-5 pyrrolidinecarboxylate-2 de tert-butyle-(2RS,4RS,5SR) fondant à 199°C.
B Le diméthylcarbamoyl-4 phényl-5 pyrrolidinecarboxylate-2 de tert-butyle-(2RS,4RS,5SR) peut être préparé comme décrit à l'exemple 2 C, mais à partir de 4,4 g de benzylidèneaminoacétate de tert-butyle, de 1,92 cm³ de N,N-diméthyl-acrylamide, de 5 g d'acétate d'argent et de 2,8 cm³ de triéthylamine dans 200 cm³ d'acétonitrile. Après traitement, on obtient 1,7 g de diméthylcarbamoyl-4 phényl-5 pyrrolidinecarboxylate-2 de tert-butyle-(2RS,4RS,5SR) fondant à 97°C.
C L'acide [(méthyl-3 phényl)-3 uréido]-2 acétique peut être préparé comme décrit à l'exemple 1 G, mais à partir de 18,8 g de glycine et de 21 g d'hydrogénocarbonate de sodium en solution dans 200 cm³ d'eau et de 32,3 cm³ d'isocyanate de méta-tolyle. Après traitement, on obtient 40,3 g d'acide [(méthyl-3 phényl)-3 uréido]-2 acétique utilisé tel quel dans les synthèses ultérieures.

### Exemple 5

A On opère d'une manière analogue à celle décrite à l'exemple 2 B, mais à partir de 1,7 g de cyano-4 phényl-5 pyrrolidinecarboxylate-2 de tert-butyle-(2RS,4SR,5SR), de 1,3 g d'acide [(méthyl-3 phényl)-3 uréido]-2 acétique et de 1,3 g de N,N'-dicyclohexylcarbodiimide dans 50 cm³ d'acétonitrile. Après traitement, on obtient 1,5 g de cyano-4 {[(méthyl-3 phényl)-3 uréido]-2 acétyl}-1 phényl-5 pyrrolidinecarboxylate-2 de tert-butyle-(2RS,4SR,5SR) sous forme d'un solide amorphe [Rf = 0,17; éluant : cyclohexane-acétate d'éthyle (50/50)].
B Le cyano-4 phényl-5 pyrrolidinecarboxylate-2 de tert-butyle-(2RS,4SR,5SR) peut être préparé comme décrit à l'exemple 2 C, mais à partir de 4,4 g de benzylidèneaminoacétate de tert-butyle, de 1,3 cm³ d'acrylonitrile, de 5 g d'acétate d'argent et de 2,8 cm³ de triéthylamine dans 200 cm³ d'acétonitrile. Après traitement, on obtient 1,7 g de cyano-4 phényl-5 pyrrolidinecarboxylate-2 de tert-butyle-(2RS,4SR,5SR) fondant à 112°C et 1 g de cyano-4 phényl-5 pyrrolidinecarboxylate-2 de tert-butyle-(2RS,4RS,5SR) fondant à 70°C.

### Exemple 6

On opère d'une manière analogue à celle décrite à l'exemple 2 B, mais à partir de 1 g de cyano-4 phényl-5 pyrrolidinecarboxylate-2 de tert-butyle-(2RS,4RS,5SR), de 0,76 g d'acide [(méthyl-3 phényl)-3 uréido]-2 acétique et de 0,76 g de N,N'-dicyclohexylcarbodiimide dans 50 cm³ d'acétonitrile. Après traitement, on obtient 1,2 g de cyano-4 {[(méthyl-3 phényl)-3 uréido]-2 acétyl}-1 phényl-5 pyrrolidinecarboxylate-2 de tert-butyle-(2RS,4RS,5SR) fondant à 150°C.

### Exemple 7

A On opère d'une manière analogue à celle décrite à l'exemple 2 B, mais à partir de 4,5 g d'acétyl-4 phényl-5 pyrrolidinecarboxylate-2 de tert-butyle-(2RS,4RS,5SR), de 3,2 g d'acide [(méthyl-3 phényl)-3 uréido]-2 acétique et de 3,5 g de N,N'-dicyclohexylcarbodiimide dans 50 cm³ d'acétonitrile. Après traitement, on obtient 1 g d'acétyl-4 {[(méthyl-3 phényl)-3 uréido]-2 acétyl}-1 phényl-5 pyrrolidinecarboxylate-2 de tert-butyle-(2RS,4RS,5SR) fondant à 100°C.
B L'acétyl-4 phényl-5 pyrrolidinecarboxylate-2 de tert-butyle-(2RS,4RS,5SR) peut être préparé comme décrit à l'exemple 2 C, mais à partir de 4,4 g de benzylidèneaminoacétate de tert-butyle, de 1,6 cm³ de méthylvinylcétone, de 5 g d'acétate d'argent et de 2,8 cm³ de triéthylamine dans 200 cm³ d'acétonitrile. Après traitement, on obtient 4,6 g d'acétyl-4 phényl-5 pyrrolidinecarboxylate-2 de tert-butyle-(2RS,4RS,5SR) sous forme d'une huile utilisée telle quelle dans les synthèses ultérieures.

### Exemple 8

A On opère d'une manière analogue à celle décrite à l'exemple 3, mais à partir de 1,6 g de {[(méthyl-3 phényl)-3 uréido]-2 acétyl}-1 phényl-5 pyrrolidinedicarboxylate-2,4 de (2) tert-butyle et de (4) méthyle-(2RS,4RS,5SR) et de 0,18 g d'hydroxyde de potassium dans 20 cm³ d'eau distillée et 60 cm³ de méthanol. Après traitement, on obtient 0,7 g de {[(méthyl-3 phényl)-3 uréido]-2 acétyl}-1 phényl-5 pyrrolidinedicarboxylate-2,4 acide de (2) tert-butyle-(2RS,4SR,5SR) fondant à 160°C.
B Le {[(méthyl-3 phényl)-3 uréido]-2 acétyl}-1 phényl-5 pyrrolidinedicarboxylate-2,4 de (2) tert-butyle et de (4) méthyle-(2RS,4RS,5SR) peut être préparé comme décrit à l'exemple 2 B, mais à partir de 1,22 g de phényl-5 pyrrolidinedicarboxylate-2,4 de (2) tert-butyle et de (4) méthyle-(2RS,4RS,5SR), de 0,83 g d'acide [(méthyl-3 phényl)-3 uréido]-2 acétique et de 0,83 g de N,N'-dicyclohexylcarbodiimide dans 35 cm³ d'acétonitrile. Après traitement, on obtient 1,6 g de {[(méthyl-3 phényl)-3 uréido]-2 acétyl}-1 phényl-5 pyrrolidinedicarboxylate-2,4 de (2) tert-butyle et de (4) méthyle-(2RS,4RS,5SR) fondant à 130°C.

### Exemple 9

A On opère d'une manière analogue à celle décrite à l'exemple 2 A, mais à partir de 2,5 g de {[(benzyloxycarbonyl-3 phényl)-3 uréido]-2 acétyl}-1 méthyl-4 phényl-5 pyrrolidinedicarboxylate-2,4 de (4) benzyle et de (2) tert-butyle-(2RS,4RS,5SR) et de 0,25 g de palladium à 10% sur charbon dans 100 cm³ d'éthanol. Après traitement, on obtient 1,4 g de ([(carboxy-3 phényl)-3 uréido]-2 acétyl}-1 méthyl-4 phényl-5 pyrrolidinedicarboxylate-2,4 acide de (2) tert-butyle-(2RS,4RS,5SR) [Rf = 0,15; éluant : chlorure de méthylène-méthanol (80/20)].
B Le {[(benzyloxycarbonyl-3 phényl)-3 uréido]-2 acétyl}-1 méthyl-4 phényl-5 pyrrolidinedicarboxylate-2,4 de (4) benzyle et de (2) tert-butyle-(2RS,4RS,5SR) peut être préparé comme décrit à l'exemple 2 B, mais à partir de 1,98 g de méthyl-4 phényl-5 pyrrolidinedicarboxylate-2,4 de (4) benzyle et de (2) tert-butyle-(2RS,4RS,5SR), de 1,64 g d'acide [(benzyloxycarbonyl-3 phényl)-3 uréido]-2 acétique et de 1,03 g de N,N'-di-cyclohexylcarbodiimide dans 50 cm³ d'acétonitrile. Après traitement, on obtient 2,5 g de {[(benzyloxycarbonyl-3 phényl)-3 uréido]-2 acétyl}-1 méthyl-4 phényl-5 pyrrolidinedicarboxylate-2,4 de (4) benzyle et de (2) tert-butyle-(2RS,4RS,5SR) sous forme d'un solide amorphe utilisé tel quel dans les synthèses ultérieures.
C Le méthyl-4 phényl-5 pyrrolidinedicarboxylate-2,4 de (4) benzyle et de (2) tert-butyle-(2RS,4RS,5SR) peut être préparé comme décrit à l'exemple 2 C, mais à partir de 4,4 g de benzylidèneaminoacétate de tert-butyle, de 3,4 cm³ de méthacrylate de benzyle, de 5 g d'acétate d'argent et de 2,8 cm³ de triéthylamine dans 200cm³ d'acétonitrile. Après traitement, on obtient 2,7 g de méthyl-4 phényl-5 pyrrolidinedicarboxylate-2,4 de (4) benzyle et de (2) tert-butyle-(2RS,4RS,5SR) sous forme d'une huile utilisée telle quelle dans les synthèses ultérieures.

### Exemple 10

Une solution de 3 g d'acétyl-4 {[(méthyl-3 phényl)-3 uréido]-2 acétyl}-1 phényl-5 pyrrolidinecarboxylate-2 de tert-butyle-(2RS,4RS,5SR) et de 0,48 g de chlorure d'hydroxylammonium dans un mélange de 6 cm³ de pyridine, de 12 cm³ de méthanol et de 6 cm³ d'eau distillée est chauffée au reflux pendant deux heures. Après refroidissement à une température voisine de 20°C, le milieu est concentré sous pression réduite et dilué par un mélange de 25 cm³ d'eau distillée et de 75 cm³ d'acétate d'éthyle. Après décantation, la phase aqueuse est extraite par deux fois 50 cm³ d'acétate d'éthyle. Les phases organiques sont rassemblées et lavées par 50 cm³ d'eau distillée, séchées sur sulfate de magnésium et concentrées sous pression réduite. Le résidu est cristallisé dans un mélange oxyde de diisopropyle-acétate d'isopropyle 1-1 en volumes. Les cristaux sont filtrés et lavés par trois fois 2,5 cm³ d'un mélange oxyde de diisopropyle-acétate d'isopropyle (1-1 en volumes) et séchés à l'air pour donner 1 g d'isomère E brut. Les filtrats sont réunis et concentrés sous pression réduite pour donner 2,1 g de mélange d'isomères Z et E. L'isomère E brut est recristallisé dans 25 cm³ d'isopropanol pour donner, après filtration et lavage par trois fois 1 cm³ d'oxyde de diisopropyle, 0,5 g d'(hydroxyimino-1 éthyl)-4 {[(méthyl-3 phényl)-3 uréido]-2 acétyl}-1 phényl-5 pyrrolidinecarboxylate-2 de tert-butyle-(2RS,4RS,5SR)-(E) fondant à 215°C.

Le mélange d'isomères Z et E est purifié par quatre chromatographies successives sur silice [éluants : oxyde de diéthyle, oxyde de diéthyle-oxyde de diisopropyle (70-30 en volumes), oxyde de diéthyle-oxyde de diisopropyle (40-60 en volumes), oxyde de diéthyle-oxyde de diisopropyle (50-50 en volumes)]. Les fractions contenant le produit attendu sont réunies et concentrées sous pression réduite. Le résidu est cristallisé dans 35 cm³ d'oxyde de diéthyle. On obtient ainsi 0,4 g d'(hydroxyimino-1 éthyl)-4 {[(méthyl-3 phényl)-3 uréido]-2 acétyl}-1 phényl-5 pyrrolidinecarboxylate-2 de tert-butyle-2-(2RS,4RS,5SR)-(Z) fondant à 166°C.

### Exemple 11

A On opère d'une manière analogue à celle décrite à l'exemple 2 A, mais à partir de 3,3 g de {[(benzyloxycarbonyl-3 phényl)-3 uréido]-2 acétyl}-1 phényl-5 phénylsulfonyl-4 pyrrolidinecarboxylate-2 de tert-butyle-(2RS,4SR,5RS) et de 0,35 g de palladium à 10% sur charbon dans 100 cm³ d'éthanol. Après traitement, on obtient 1,45 g d'acide {[(tert-butoxycarbonyl-2 phényl-5 phénylsulfonyl-4 pyrrolidinyl-1)-2 oxo-2 éthyl]-3 uréido}-3 benzoïque-(2RS,4SR,5RS) fondant à 213°C.
B Le {[(benzyloxycarbonyl-3 phényl)-3 uréido]-2 acétyl}-1 phényl-5 phénylsulfonyl-4 pyrrolidinecarboxylate-2 de tert-butyle-(2RS,4SR,5RS) peut être préparé comme décrit à l'exemple 2 B, mais à partir de 1,94 g de phényl-5 phénylsulfonyl-4 pyrrolidinecarboxylate-2 de tert-butyle-(2RS,4SR,5RS), de 1,64 g d'acide [(benzyloxycarbonyl-3 phényl)-3 uréido]-2 acétique et de 1,03 g de N,N'-dicyclohexylcarbodiimide dans 60 cm³ d'acétonitrile. Après traitement, on obtient 3,3 g de ([(benzyloxycarbonyl-3 phényl)-3 uréido]-2 acétyl}-1 phényl-5 phénylsulfonyl-4 pyrrolidinecarboxylate-2 de tert-butyle-(2RS,4SR,5RS) sous forme d'un solide utilisé tel quel dans les synthèses ultérieures.
C Le phényl-5 phénylsulfonyl-4 pyrrolidinecarboxylate-2 de tert-butyle-(2RS,4SR,5RS) peut être préparé comme décrit à l'exemple 2 C, mais à partir de 4,4 g de benzylidèneaminoacétate de tert-butyle, de 3,4 g de phénylvinylsulfone, de 5 g d'acétate d'argent et de 2,8 cm³ de triéthylamine dans 200 cm³ d'acétonitrile. Après traitement, on obtient 3,35 g de phényl-5 phénylsulfonyl-4 pyrrolidinecarboxylate-2 de tert-butyle-(2RS,4SR,5RS) fondant à 112°C et 1,1 g de phényl-5 phénylsulfonyl-4 pyrrolidinecarboxylate-2 de tert-butyle-(2RS,4RS,5RS) fondant à 204°C.

### Exemple 12

A On opère d'une manière analogue à celle décrite à l'exemple 2 A, mais à partir de 1,9 g de {[(benzyloxycarbonyl-3 phényl)-3 uréido]-2 acétyl}-1 phényl-5 phénylsulfonyl-4 pyrrolidinecarboxylate-2 de tert-butyle-(2RS,4RS,5RS) et de 0,2 g de palladium à 10% sur charbon dans 75 cm³ d'éthanol. Après traitement, on obtient 1,1 g d'acide {[(tert-butoxycarbonyl-2 phényl-5 phénylsulfonyl-4 pyrrolidinyl-1)-2 oxo-2 éthyl]-3 uréido}-3 benzoïque-(2RS,4RS,5RS) [Rf = 0,22; éluant : chlorure de méthylène-méthanol (80/20)].
B Le {[(benzyloxycarbonyl-3 phényl)-3 uréido]-2 acétyl}-1 phényl-5 phénylsulfonyl-4 pyrrolidinecarboxylate-2 de tert-butyle-(2RS,4RS,5RS) peut être préparé comme décrit à l'exemple 2 B, mais à partir de 1,1 g de phényl-5 phénylsulfonyl-4 pyrrolidinecarboxylate-2 de tert-butyle-(2RS,4RS,5RS), de 0,95 g d'acide [(benzyloxycarbonyl-3 phényl)-3 uréido]-2 acétique et de 0,6 g de N,N'-dicyclohexylcarbodiimide dans 40 cm³ d'acétonitrile. Après traitement, on obtient 1,9 g de {[(benzyloxycarbonyl-3 phényl)-3 uréido]-2 acétyl}-1 phényl-5 phénylsulfonyl-4 pyrrolidinecarboxylate-2 de tert-butyle-(2RS,4RS,5RS) fondant à 212°C.

### Exemple 13

A On opère d'une manière analogue à celle décrite à l'exemple 2 A, mais à partir de 1,62 g de benzyloxycarbonylamino-4 {[(benzyloxycarbonyl-3 phényl)-3 uréido]-2 acétyl}-1 phényl-5 pyrrolidinecarboxylate-2 de tert-butyle-(2RS,4SR,5RS) et de 0,16 g de palladium à 10% sur charbon dans 100 cm³ d'éthanol. Après traitement, on obtient 0,43 g d'amino-4 {[(carboxy-3 phényl)-3 uréido]-2 acétyl}-1 pyrrolidinecarboxylate-2 de tert-butyle-(2RS,4SR,5RS) sous forme d'un solide amorphe [Rf = 0,24; éluant : chlorure de méthylène-méthanol (80/20)].
B Le benzyloxycarbonylamino-4 {[(benzyloxycarbonyl-3 phényl)-3 uréido]-2 acétyl}-1 phényl-5 pyrrolidinecarboxylate-2 de tert-butyle-(2RS,4SR,5RS) peut être obtenu de la manière suivante : à une solution de 0,83 g de N,N'-diimidazole-carbonyle dans 30 cm³ de dichloro-1,2 éthane, on ajoute une solution de 2,1 g d'(amino-2 acétyl)-1 benzyloxycarbonylamino-4 phényl-5 pyrrolidinecarboxylate-2 de tert-butyle-(2RS,4SR,5RS) dans 20 cm³ de dichloro-1,2 éthane. Le mélange est agité pendant deux heures à une température voisine de 20°C, puis on ajoute 1,05 g d'amino-3 benzoate de benzyle. Le mélange est chauffé à une température voisine de 80°C pendant quatorze heures. Après refroidissement à une température voisine de 20°C, le milieu réactionnel est dilué par 75 cm³ de dichlorométhane. La phase organique est lavée par deux fois 100 cm³ d'eau distillée puis deux fois 100 cm³ d'une solution aqueuse décinormale d'acide chlorhydrique, deux fois 100 cm³ d'eau distillée, deux fois 100 cm³ d'une solution aqueuse saturée en chlorure de sodium, séchée sur sulfate de magnésium et concentrée sous pression réduite. Le résidu est purifié par chromatographie sur silice (éluant : chlorure de méthylène). Les fractions contenant le produit attendu sont réunies et concentrées sous pression réduite. On obtient ainsi 1,6 g de benzyloxycarbonylamino-4 {[(benzyloxycarbonyl-3 phényl)-3 uréido]-2 acétyl}-1 phényl-5 pyrrolidinecarboxylate-2 de tert-butyle-(2RS,4SR,5RS) sous forme d'une meringue utilisée telle quelle dans les synthèses ultérieures.
C L'(amino-2 acétyl)-1 benzyloxycarbonylamino-4 phényl-5 pyrrolidinecarboxylate-2 de tert-butyle-(2RS,4SR,5RS) peut être obtenu de la manière suivante : à une solution de 4,75 g de benzyloxycarbonylamino-4 (tert-butoxycarbonylamino-2 acétyl)-1 phényl-5 pyrrolidinecarboxylate-2 de tert-butyle-(2RS,4SR,5RS) dans 125 cm³ de chloroforme on ajoute 1,22cm³ d'iodotriméthylsilane. Le mélange réactionnel est agité pendant vingt heures à une température voisine de 20°C, puis est amené à un pH voisin de 7 par addition d'une solution aqueuse saturée d'hydrogénocarbonate de sodium. La phase organique est décantée, lavée par deux fois 100 cm³ solution aqueuse saturée en chlorure de sodium, séchée sur sulfate de magnésium et concentrée sous pression réduite. Le résidu purifié par chromatographie sur silice [éluant : dichlorométhane-méthanol (99-1 en volumes)]. Les fractions contenant le produit attendu sont réunies et concentrées sous pression réduite. On obtient ainsi 2,1 g d'(amino-2 acétyl)-1 benzyloxycarbonylamino-4 phényl-5 pyrrolidinecarboxylate-2 de tert-butyle-(2RS,4SR,5RS) sous forme d'une meringue utilisée telle quelle dans les synthèses ultérieures.
D Le benzyloxycarbonylamino-4 (tert-butoxycarbonylamino-2 acétyl)-1 phényl-5 pyrrolidinecarboxylate-2 de tert-butyle-(2RS,4SR,5RS) peut être obtenu de la manière suivante : une solution de 5,38 g de (tert-butoxycarbonylamino-2 acétyl)-1 phényl-5 pyrrolidinedicarboxylate-2,4 acide de (2) tert-butyle-(2RS,4SR,5SR), de 3,1 cm³ de diphénylposphorazide et de 1,86 cm³ de triéthylamine dans 80 cm³ de toluène est agitée pendant trente minutes à une température voisine de 20°C puis trente minutes à une température voisine de 80°C. On ajoute ensuite goutte à goutte une solution de 1,49 cm³ d'alcool benzylique dans 40 cm³ de toluène contenant 57 mg d'hydrure de sodium. Le milieu réactionnel est agité pendant vingt heures à une température voisine de 50°C. Après retour à une température voisine de 20°C, la phase organique est lavée par trois fois 75 cm³ d'eau distillée puis 50 cm³ d'une solution aqueuse saturée en chlorure de sodium, séchée sur sulfate de magnésium et concentrée sous pression réduite. Le résidu est purifié par chromatographie sur silice [éluant : dichlorométhane puis dichlorométhane-méthanol (99-1 en volumes)]. Les fractions contenant le produit attendu sont réunies et concentrées sous pression réduite. On obtient ainsi 3,75 g de benzyloxycarbonylamino-4 (tert-butoxycarbonylamino-2 acétyl)-1 phényl-5 pyrrolidinecarboxylate-2 de tert-butyle-(2RS,4SR,5RS) sous forme d'une meringue utilisée telle quelle dans les synthèses ultérieures.
E Le (tert-butoxycarbonylamino-2 acétyl)-1 phényl-5 pyrrolidinedicarboxylate-2,4 acide de (2) tert-butyle-(2RS,4SR,5SR) peut être préparé de la manière suivante : une solution de 34,7 g de (tert-butoxycarbonylamino-2 acétyl)-1 phényl-5 pyrrolidinedicarboxylate-2,4 de (2) tert-butyle et de (4) méthyle-(2RS,4RS,5SR) et de 4,81 g d'hydroxyde de potassium dans un mélange de 600 cm³ de méthanol et de 20 cm³ d'eau est agitée pendant vingt heures à une température voisine de 20°C. Le milieu est ensuite concentré sous pression réduite et dilué à l'eau. La phase aqueuse est lavée par trois fois 200 cm³ d'oxyde de diéthyle puis amenée à pH voisin de 1 par une solution aqueuse 4N d'acide chlorhydrique et extraite par trois fois 200 cm³ de dichlorométhane. Les phases organiques réunies sont lavées par deux fois 200 cm³ d'une solution aqueuse saturée en chlorure de sodium, séchées sur sulfate de magnésium et concentrées sous pression réduite. Le résidu est repris à l'oxyde de diisopropyle, filtré et séché sous pression réduite. On obtient ainsi 27,5 g de (tert-butoxycarbonylamino-2 acétyl)-1 phényl-5 pyrrolidinedicarboxylate-2,4 acide de (2) tert-butyle-(2RS,4SR,5SR) fondant à 105°C.
F Le (tert-butoxycarbonylamino-2 acétyl)-1 phényl-5 pyrrolidinedicarboxylate-2,4 de (2) tert-butyle et de (4) méthyle-(2RS,4RS,5SR) peut être préparé de la manière suivante : à une solution de 17 g de phényl-5 pyrrolidinedicarboxylate-2,4 de (2) tert-butyle et de (4) méthyle-(2RS,4RS,5SR) et de 9,75 g d'acide tert-butoxycarbonylamino-2 acétique dans 200 cm³ d'acétonitrile, on ajoute 11,5 g de N,N'-dicyclohexylcarbodiimide. Le mélange réactionnel est agité pendant vingt heures à une température voisine de 20°C puis concentré sous pression réduite. Le résidu est repris par 100 cm³ d'acétate d'éthyle et filtré. Le solide est rincé par deux fois 50 cm³ d'acétate d'éthyle et les filtrats réunis sont consentrés sous pression réduite. Le résidu est cristallisé dans le pentane et séché sous pression réduite à une température voisine de 40°C. On obtient ainsi 22,5 g de (tert-butoxycarbonylamino-2 acétyl)-1 phényl-5 pyrrolidinedicarboxylate-2,4 de (2) tert-butyle et de (4) méthyle-(2RS,4RS,5SR) fondant à 137°C.

### Exemple 14

A On opère d'une manière analogue à celle décrite à l'exemple 2 A, mais à partir de 3,45 g de {[(benzyloxycarbonyl-3 phényl)-3 uréido]-2 acétyl}-1 phényl-5 pyrrolidinedicarboxylate-2,4 de (4) benzyle et de (2) tert-butyle-(2RS,4RS,5SR) et de 0,5 g de palladium à 10% sur charbon dans 150 cm³ d'éthanol. Après traitement, on obtient 2 g de {[(carboxy-3 phényl)-3 uréido]-2 acétyl}-1 pyrrolidinedicarboxylate acide de (2) tert-butyle-(2RS,4RS,5SR) fondant à 180°C.
B Le {[(benzyloxycarbonyl-3 phényl)-3 uréido]-2 acétyl}-1 phényl-5 pyrrolidinedicarboxylate-2,4 de (4) benzyle et de (2) tert-butyle-(2RS,4RS,5SR) peut être préparé comme décrit à l'exemple 2 B, mais à partir de 3,05 g de phényl-5 pyrrolidinedicarboxylate-2,4 de (4) benzyle et de (2) tert-butyle-(2RS,4RS,5SR), de 2,6 g d'acide [(benzyloxycarbonyl-3 phényl)-3 uréido]-2 acétique et de 1,65 g de N,N'-dicyclohexylcarbodiimide dans 75 cm³ d'acétonitrile. Après traitement, on obtient 1,35 g de {[(benzyloxycarbonyl-3 phényl)-3 uréido]-2 acétyl}-1 phényl-5 pyrrolidinedicarboxylate-2,4 de (4) benzyle et de (2) tert-butyle-(2RS,4RS,5SR) fondant à 110°C.
C Le phényl-5 pyrrolidinedicarboxylate-2,4 de (4) benzyle et de (2) tert-butyle-(2RS,4RS,5SR) peut être préparé comme décrit à l'exemple 2 C, mais à partir de 17 g de benzylidèneaminoacétate de tert-butyle, de 12,25 cm³ d'acrylate de benzyle, de 20 g d'acétate d'argent et de 11,2 cm³ de triéthylamine dans 600 cm³ d'acétonitrile. Après traitement, on obtient 18,9 g de phényl-5 pyrrolidinedicarboxylate-2,4 de (4) benzyle et de (2) tert-butyle-(2RS,4RS,5SR) fondant à 70°C.

### Exemple 15

A On opère d'une manière analogue à celle décrite à l'exemple 3, mais à partir de 2,1 g de {[(chloro-4 phényl)-3 uréido]-2 acétyl}-1 phényl-5 pyrrolidinedicarboxylate-2,4 de (2) tert-butyle et de (4) méthyle-(2RS,4RS,5SR) dans un mélange de 4 cm³ d'une solution aqueuse normale d'hydroxyde de potassium, de 20 cm³ d'eau distillée et de 60 cm³ de méthanol. Après traitement, on obtient 1,4 g de {[(chloro-4 phényl)-3 uréido]-2 acétyl}-1 phényl-5 pyrrolidinedicarboxylate-2,4 acide de (2) tert-butyle-(2RS,4SR,5SR) fondant à 248°C.
B Le {[(chloro-4 phényl)-3 uréido]-2 acétyl}-1 phényl-5 pyrrolidinedicarboxylate-2,4 de (2) tert-butyle et de (4) méthyle-(2RS,4RS,5SR) peut être préparé comme décrit à l'exemple 2 B, mais à partir de 1,53 g de phényl-5 pyrrolidinedicarboxylate-2,4 de (2) tert-butyle et de (4) méthyle-(2RS,4RS,5SR), de 1,14 g d'acide (chloro-4 phényl)-3 uréido]-2 acétique et de 1,03 g de N,N'-dicyclohexylcarbodiimide dans 45 cm³ d'acétonitrile. Après traitement, on obtient 2,3 g de {[(chloro-4 phényl)-3 uréido]-2 acétyl}-1 phényl-5 pyrrolidinedicarboxylate-2,4 de (2) tert-butyle et de (4) méthyle-(2RS,4RS,5SR) fondant à 125°C.
C L'acide [(chloro-4 phényl)-3 uréido]-2 acétique peut être préparé comme décrit à l'exemple 1 G, mais à partir de 2,25 g de glycine et de 2,5 g de bicarbonate de sodium en solution dans 35 cm³ d'eau et de 4,6 g d'isocyanate de para-chloro phényle. Après traitement, on obtient 5 g d'acide [(chloro-4 phényl)-3 uréido]-2 acétique fondant à 218°C.

### Exemple 16

A On opère d'une manière analogue à celle décrite à l'exemple 3, mais à partir de 1,8 g d'({[(hydroxy-1 éthyl-(RS))-3 phényl]-3 uréido}-2 acétyl)-1 phényl-5 pyrrolidinedicarboxylate-2,4 de (2) tert-butyle et de (4) méthyle-(2RS,4RS,5SR)-(mélange des formes A et B) et de 0,19 g d'hydroxyde de potassium dans un mélange de 15 cm³ d'eau distillée et de 50 cm³ de méthanol. Après traitement, on obtient 0,8 g d'({[(hydroxy-1 éthyl)-3 phényl]-3 uréido}-2 acétyl)-1 phényl-5 pyrrolidinedicarboxylate-2,4 acide de (2) tert-butyle-(2RS,4SR,5SR)-(mélange des formes A et B) [Rf = 0,05; éluant : chlorure de méthylène-méthanol (90/10)].
B L'({[(hydroxy-1 éthyl-(RS))-3 phényl]-3 uréido}-2 acétyl)-1 phényl-5 pyrrolidinedicarboxylate-2,4 de (2) tert-butyle et de (4) méthyle-(2RS,4RS,5SR)-(mélange des formes A et B) peut être préparé de la manière suivante : à une solution de 1,1 g de N,N'-diimidazole-carbonyle dans 50 cm³ de dichloro-1,2 éthane, on ajoute une solution de 2,2 g d'(amino-2 acétyl)-1 phényl-5 pyrrolidinedicarboxylate-2,4 de (2) tert-butyle et de (4) méthyle-(2RS,4RS,5SR) dans 25 cm³ de dichloro-1,2 éthane. Le mélange est agité pendant une heure à une température voisine de 20°C, puis on ajoute 0,92 g d'(hydroxy-1 éthyl)-3 aniline-(RS). Le mélange est chauffé à une température voisine de 80°C pendant quatre heures. Après refroidissement à une température voisine de 20°C, le milieu réactionnel est dilué par 100 cm³ de dichlorométhane. La phase organique est lavée par deux fois 50 cm³ d'eau distillée puis deux fois 50 cm³ d'une solution aqueuse décinormale d'acide chlorhydrique, séchée sur sulfate de magnésium et concentrée sous pression réduite. Le résidu est purifié par chromatographie sur silice [éluant : acétate d'éthyle-cyclohexane (70-30 en volumes)]. Les fractions contenant le produit attendu sont réunies et concentrées sous pression réduite. On obtient ainsi 1,9 g d'({[(hydroxy-1 éthyl-(RS))-3 phényl]-3 uréido}-2 acétyl)-1 phényl-5 pyrrolidinedicarboxylate-2,4 de (2) tert-butyle et de (4) méthyle-(2RS,4RS,5SR)-(mélange des formes A et B) sous forme d'un solide amorphe utilisé tel quel dans les synthèses ultérieures.
C L'(amino-2 acétyl)-1 phényl-5 pyrrolidinedicarboxylate-2,4 de (2) tert-butyle et de (4) méthyle-(2RS,4RS,5SR) peut être préparé de la manière suivante : à une solution de 9,25 g de (tert-butoxycarbonylamino-2 acétyl)-1 phényl-5 pyrrolidinedicarboxylate-2,4 de (2) tert-butyle et de (4) méthyle-(2RS,4RS,5SR) dans 200 cm³ de chloroforme on ajoute 2,85 cm³ d'iodotriméthylsilane. Le mélange réactionnel est agité pendant deux heures à une température voisine de 20°C, puis est amené à un pH voisin de 7 par addition d'une solution aqueuse saturée d'hydrogénocarbonate de sodium. La phase organique est décantée, lavée par une solution aqueuse saturée en chlorure de sodium, séchée sur sulfate de magnésium et concentrée sous pression réduite. On obtient ainsi 5 g d'(amino-2 acétyl)-1 phényl-5 pyrrolidinedicarboxylate-2,4 de (2) tert-butyle et de (4) méthyle-(2RS,4RS,5SR) sous forme d'une huile utilisée telle quelle dans les synthèses ultérieures.
D Le (tert-butoxycarbonylamino-2 acétyl)-1 phényl-5 pyrrolidinedicarboxylate-2,4 de (2) tert-butyle et de (4) méthyle-(2RS,4RS,5SR) peut être préparé de la manière suivante : à une solution de 17 g de phényl-5 pyrrolidinedicarboxylate-2,4 de (2) tert-butyle et de (4) méthyle-(2RS,4RS,5SR) et de 9,75 g d'acide tert-butoxycarbonylamino-2 acétique dans 200 cm³ d'acétonitrile, on ajoute 11,5 g de N,N'-dicyclohexylcarbodiimide. Le mélange réactionnel est agité pendant vingt heures à une température voisine de 20°C puis concentré sous pression réduite. Le résidu est repris par 100 cm³ d'acétate d'éthyle et filtré. Le solide est rincé par deux fois 50 cm³ d'acétate d'éthyle et les filtrats réunis sont concentrés sous pression réduite. Le résidu est cristallisé dans le pentane et séché sous pression réduite à une température voisine de 40°C. On obtient ainsi 22,5 g de (tert-butoxycarbonylamino-2 acétyl)-1 phényl-5 pyrrolidinedicarboxylate-2,4 de (2) tert-butyle et de (4) méthyle-(2RS,4RS,5SR) fondant à 137°C.

### Exemple 17

A On opère d'une manière analogue à celle décrite à l'exemple 3, mais à partir de 2,6 g de {[(méthoxycarbonylméthylthio-3 phényl)-3 uréido]-2 acétyl}-1 phényl-5 pyrrolidinedicarboxylate-2,4 de (2) tert-butyle et de (4) méthyle-(2RS,4RS,5SR) et de 0,5 g d'hydroxyde de potassium dans un mélange de 15 cm³ d'eau distillée et de 50 cm³ de méthanol. Après traitement, on obtient 1,8 g de {[(carboxyméthylthio-3 phényl)-3 uréido]-2 acétyl)-1 phényl-5 pyrrolidinedicarboxylate-2,4 acide de (2) tert-butyle-(2RS,4SR,5SR) [Rf = 0,1; éluant: chlorure de méthylène-méthanol (90/10)].
B Le {[(méthoxycarbonylméthylthio-3 phényl)-3 uréido]-2 acétyl}-1 phényl-5 pyrrolidinedicarboxylate-2,4 de (2) tert-butyle et de (4) méthyle-(2RS,4RS,5SR) peut être obtenu de la manière suivante : à une solution de 2,4 g d'(amino-2 acétyl)-1 phényl-5 pyrrolidinedicarboxylate-2,4 de (2) tert-butyle et de (4) méthyle-(2RS,4RS,5SR) dans 50 cm³ de tétrahydrofuranne on ajoute une solution de 1,48 g d'(isocyanato-3 phénylthio)-2 acétate de méthyle dans 10 cm³ de tétrahydrofuranne. Le mélange est agité pendant vingt heures à une température voisine de 20°C puis concentré sous pression réduite. Le résidu est purifié par chromatographie sur silice [éluant : acétate d'éthyle-cyclohexane (50-50 en volumes)]. Les fractions contenant le produit attendu sont réunies et concentrées sous pression réduite. On obtient ainsi 2,6 g de {[(méthoxycarbonylméthylthio-3 phényl)-3 uréido]-2 acétyl}-1 phényl-5 pyrrolidinedicarboxylate-2,4 de (2) tert-butyle et de (4) méthyle-(2RS,4RS,5SR) sous forme d'un solide amorphe utilisé tel quel dans les synthèses ultérieures.
C L'(isocyanato-3 phénylthio)-2 acétate de méthyle peut être préparé de la manière suivante : à une suspension de 2,2 g de noir 3S dans 300 cm³ de toluène et 13,3 cm³ de chloroformiate de trichlorométhyle, on ajoute, à une température voisine de -20°C, une solution de 21,7 g d'(amino-3 phénylthio)-2 acétate de méthyle dans 300 cm³ de toluène. Le milieu réactionnel est agité à une température voisne de -20°C pendant deux heures puis à une température voisine de 20°C pendant deux heures et enfin au reflux pendant deux heures. Après refroidissement à une température voisine de 20°C, le milieu est dégazé par barbottage d'azote, filtré sur supercel et concentré sous pression réduite. On obtient ainsi 25 g d'(isocyanato-3 phénylthio)-2 acétate de méthyle sous forme d'une huile utilisée telle quelle dans les synthèses ultérieures.
D L'(amino-3 phénylthio)-2 acétate de méthyle peut être préparé de la manière suivante : à une solution de 25 g d'amino-3 thiophénol dans 400 cm³ d'éthanol, on ajoute 20 cm³ de bromoacétate de méthyle. Le mélange est agité pendant une heure et demie à une température voisine de 20°C puis concentré sous pression réduite. Le résidu est dilué par 300 cm³ d'acétate d'éthyle; la solution organique est lavée par 300 cm³ d'une solution aqueuse saturée en hydrogénocarbonate de sodium puis 300 cm³ d'eau distillée, séchée sur sulfate de magnésium et concentrée sous pression réduite. Le résidu est purifié par chromatographie sur silice [éluant : acétate d'éthyle-cyclohexane (25-75 en volumes)]. Les fractions contenant le produit attendu sont réunies et concentrées sous pression réduite. On obtient ainsi 21,8 g d'(amino-3 phénylthio)-2 acétate de méthyle sous forme d'une huile utilisée telle quelle dans les synthèses ultérieures.

### Exemple 18

A On opère d'une manière analogue à celle décrite à l'exemple 3, mais à partir de 1,5 g de {[(méthylthio-3 phényl)-3 uréido]-2 acétyl}-1 phényl-5 pyrrolidinedicarboxylate.2,4 de (2) tert-butyle et de (4) méthyle-(2RS,4RS,5SR) dans un mélange de 2,8 cm³ d'une solution aqueuse normale d'hydroxyde de potassium, de 15 cm³ d'eau distillée et de 60 cm³ de méthanol. Après traitement, on obtient 1,05 g de {[(méthylthio-3 phényl)-3 uréido]-2 acétyl}-1 phényl-5 pyrrolidinedicarboxylate-2,4 acide de (2) tert-butyle-(2RS,4SR,5SR) fondant à 130°C.
B Le {[(méthylthio-3 phényl)-3 uréido]-2 acétyl}-1 phényl-5 pyrrolidinedicarboxylate-2,4 de (2) tert-butyle et de (4) méthyle-(2RS,4RS,5SR) peut être préparé comme décrit à l'exemple 16 B, mais à partir de 2,7 g d'(amino-2 acétyl)-1 phényl-5 pyrrolidinedicarboxylate-2,4 de (2) tert-butyle et de (4) méthyle-(2RS,4RS,5SR), de 1,3 g de N,N'-diimidazole-carbonyle et de 0,9 cm³ de méthylthio-3 aniline dans 90 cm³ de dichloro-1,2 éthane. Après traitement, on obtient 1,6 g de {[(méthylthio-3 phényl)-3 uréido]-2 acétyl}-1 phényl-5 pyrrolidinedicarboxylate-2,4 de (2) tert-butyle et de (4) méthyle-(2RS,4RS,5SR) sous forme d'un solide amorphe utilisé tel quel dans les synthèses ultérieures.

### Exemple 19

A On opère d'une manière analogue à celle décrite à l'exemple 2 A, mais à partir de 5,8 g de {[(benzyloxycarbonyl-3 phényl)-3 uréido]-2 acétyl}-1 (fluoro-2 phényl)-5 phénylsulfonyl-4 pyrrolidinecarboxylate-2 de tert-butyle-(2RS,4SR,5RS) et de 0,6 g de palladium à 10% sur charbon dans 200 cm³ d'éthanol. Après traitement, on obtient 2,85 g d'acide ({[tert-butoxycarbonyl-2 (fluoro-2 phényl)-5 phénylsulfonyl-4 pyrrolidinyl-1]-2 oxo-2 éthyl}-3 uréido)-3 benzoïque-(2RS,4SR,5RS) [Rf = 0,25; éluant : chlorure de méthylène-méthanol (90/10)].
B Le {[(benzyloxycarbonyl-3 phényl)-3 uréido]-2 acétyl}-1 (fluoro-2 phényl)-5 phénylsulfonyl-4 pyrrolidinecarboxylate-2 de tert-butyle-(2RS,4SR,5RS) peut être préparé comme décrit à l'exemple 2 B, mais à partir de 6 g de (fluoro-2 phényl)-5 phénylsulfonyl-4 pyrrolidinecarboxylate-2 de tert-butyle-(2RS,4SR,5RS), de 4,5 g d'acide [(benzyloxycarbonyl-3 phényl)-3 uréido]-2 acétique et de 2,8 g de N,N'-dicyclohexylcarbodiimide dans 200 cm³ d'acétonitrile. Après traitement, on obtient 5,8 g de {[(benzyloxycarbonyl-3 phényl)-3 uréido]-2 acétyl}-1 (fluoro-2 phényl)-5 phénylsulfonyl-4 pyrrolidinecarboxylate-2 de tert-butyle-(2RS,4SR,5RS) sous forme d'une meringue utilisée telle quelle dans les synthèses ultérieures.
C Le (fluoro-2 phényl)-5 phénylsulfonyl-4 pyrrolidinecarboxylate-2 de tert-butyle-(2RS,4SR,5RS) peut être préparé comme décrit à l'exemple 2 C, mais à partir de 9,5 g de (fluoro-2 benzylidèneamino)acétate de tert-butyle, de 6,7 g de phénylvinylsulfone, de 10 g d'acétate d'argent et de 5,6 cm³ de triéthylamine dans 200 cm³ d'acétonitrile. Après traitement, on obtient 6 g de (fluoro-2 phényl)-5 phénylsulfonyl-4 pyrrolidinecarboxylate-2 de tert-butyle-(2RS,4SR,5RS) fondant à 134°C et 5,5 g de (fluoro-2 phényl)-5 phénylsulfonyl-4 pyrrolidinecarboxylate-2 de tert-butyle-(2RS,4RS,5RS) fondant à 200°C.
D Le (fluoro-2 benzylidèneamino)acétate de tert-butyle peut être préparé comme décrit à l'exemple 2 D, mais à partir de 4,2 cm³ de fluoro-2 benzaldéhyde, 6,7 g de chlorhydrate de glycinate de tert-butyle, 6 g de tamis 4Å et 5,6 cm³ de triéthylamine dans 60 cm³ de dichlorométhane. Après traitement, on obtient 9,5 g de (fluoro-2 benzylidèneamino)acétate de tert-butyle sous forme d'une huile utilisée telle quelle dans les synthèses ultérieures.

### Exemple 20

A On opère d'une manière analogue à celle décrite à l'exemple 3, mais à partir de 4 g de {[(méthoxycarbonyl-3 phényl)-3 uréido]-2 acétyl}-1 phényl-5 phénylcarbamoyl-4 pyrrolidinecarboxylate-2 de tert-butyle-(2RS,4SR,5SR) dans un mélange de 6,7 cm³ d'une solution aqueuse normale d'hydroxyde de potassium, de 30 cm³ d'eau distillée et de 120 cm³ de méthanol. Après traitement, on obtient 0,55 g d'acide {[(tert-butoxycarbonyl-2 phényl-5 phénylcarbamoyl-4 pyrrolidinyl-1)-2 oxo-2 éthyl]-3 uréido}-3 benzoïque-(2RS,4SR,5SR) fondant à 160°C.
B Le {[(méthoxycarbonyl-3 phényl)-3 uréido]-2 acétyl}-1 phényl-5 phénylcarbamoyl-4 pyrrolidinecarboxylate-2 de tert-butyle-(2RS,4SR,5SR) peut être préparé comme décrit à l'exemple 17 B, mais à partir de 3,9 g d'(amino-2 acétyl)-1 phényl-5 phénylcarbamoyl-4 pyrrolidinecarboxylate-2 de tert-butyle-(2RS,4SR,5SR) et de 4,6 g d'isocyanato-3 benzoate de méthyle dans 150 cm³ de tétrahydrofuranne. Après traitement, on obtient 4 g de {[(méthoxycarbonyl-3 phényl)-3 uréido]-2 acétyl}-1 phényl-5 phénylcarbamoyl-4 pyrrolidinecarboxylate-2 de tert-butyle-(2RS,4SR,5SR) sous forme d'une meringue utilisée telle quelle dans les synthèses ultérieures.
C L'(amino-2 acétyl)-1 phényl-5 phénylcarbamoyl-4 pyrrolidinecarboxylate-2 de tert-butyle-(2RS,4SR,5SR) peut être préparé comme décrit à l'exemple 16 C, mais à partir de 4,7 g de (tert-butoxycarbonylamino-2 acétyl)-1 phényl-5 phénylcarbamoyl-4 pyrrolidinecarboxylate-2 de tert-butyle-(2RS,4SR,5SR) et de 1,73 cm³ d'iodotriméthylsilane dans 150 cm³ de chloroforme. Après traitement, on obtient 3,9 g d'(amino-2 acétyl)-1 phényl-5 phénylcarbamoyl-4 pyrrolidinecarboxylate-2 de tert-butyle-(2RS,4SR,5SR) sous forme d'une huile utilisée telle quelle dans les synthèses ultérieures.
D Le (tert-butoxycarbonylamino-2 acétyl)-1 phényl-5 phénylcarbamoyl-4 pyrrolidinecarboxylate-2 de tert-butyle-(2RS,4SR,5SR) peut être préparé de la manière suivante : à une solution de 4,48 g de (tert-butoxycarbonylamino-2 acétyl)-1 phényl-5 pyrrolidinedicarboxylate-2,4 acide de (2) tert-butyle-(2RS,4SR,5SR) et de 0,9 cm³ d'aniline dans 150 cm³ d'acétonitrile, on ajoute 2,06 g de N,N'-dicyclohexylcarbodiimide. Le mélange réactionnel est agité pendant vingt heures à une température voisine de 20°C, puis filtré sur célite. Le filtrat est concentré sous pression réduite et le résidu purifié par chromatographie sur silice [éluant : dichlorométhane-méthanol (98,5-1,5 en volumes)]. Les fractions contenant le produit attendu sont réunies et concentrées sous pression réduite. On obtient ainsi 4,8 g de (tert-butoxycarbonylamino-2 acétyl)-1 phényl-5 phénylcarbamoyl-4 pyrrolidinecarboxylate-2 de tert-butyle-(2RS,4SR,5SR) sous forme d'une meringue utilisée telle quelle dans les synthèses ultérieures.
E Le (tert-butoxycarbonylamino-2 acétyl)-1 phényl-5 pyrrolidinedicarboxylate-2,4 acide de (2) tert-butyle-(2RS,4SR,5SR) peut être préparé de la manière suivante : une solution de 34,7 g de (tert-butoxycarbonylamino-2 acétyl)-1 phényl-5 pyrrolidinedicarboxylate-2,4 de (2) tert-butyle et de (4) méthyle-(2RS,4RS,5SR) et de 4,81 g d'hydroxyde de potassium dans un mélange de 600 cm³ de méthanol et de 20 cm³ d'eau est agitée pendant vingt heures à une température voisine de 20°C. Le milieu est ensuite concentré sous pression réduite et dilué à l'eau. La phase aqueuse est lavée par trois fois 200 cm³ d'oxyde de diéthyle puis amenée à un pH voisin de 1 par une solution aqueuse 4N d'acide chlorhydrique et extraite par trois fois 200 cm³ de dichlorométhane. Les phases organiques réunies sont lavées par deux fois 200 cm³ d'une solution aqueuse saturée en chlorure de sodium, séchées sur sulfate de magnésium et concentrées sous pression réduite. Le résidu est repris à l'oxyde de diisopropyle, filtré et séché sous pression réduite. On obtient ainsi 27,5 g de (tert-butoxycarbonylamino-2 acétyl)-1 phényl-5 pyrrolidinedicarboxylate-2,4 acide de (2) tert-butyle-(2RS,4SR,5SR) fondant à 105°C.

### Exemple 21

A On opère d'une manière analogue à celle décrite à l'exemple 3, mais à partir de 3,6 g de {[(méthoxycarbonyl-3 phényl)-3 uréido]-2 acétyl}-1 méthylphénylcarbamoyl-4 phényl-5 pyrrolidinecarboxylate-2 de tert-butyle-(2RS,4RS,5SR) et de 0,33 g d'hydroxyde de potassium dans un mélange de 20 cm³ d'eau distillée et de 60 cm³ de méthanol. Après traitement, on obtient 1,1 g d'acide {[(tert-butoxycarbonyl-2 phényl-5 méthylphénylcarbamoyl-4 pyrrolidinyl-1)-2 oxo-2 éthyl]-3 uréido}-3 benzoïque-(2RS,4RS,5SR) fondant à 238°C.
B Le {[(méthoxycarbonyl-3 phényl)-3 uréido]-2 acétyl}-1 méthylphénylcarbamoyl-4 phényl-5 pyrrolidinecarboxylate-2 de tert-butyle-(2RS,4RS,5SR) peut être préparé comme décrit à l'exemple 17 B, mais à partir de 2,8 g d'(amino-2 acétyl)-1 méthylphénylcarbamoyl-4 phényl-5 pyrrolidinecarboxylate-2 de tert-butyle-(2RS,4RS,5SR) et de 1,15 g d'isocyanato-3 benzoate de méthyle dans 120 cm³ de tétrahydrofuranne. Après traitement, on obtient 3,6 g de {[(méthoxycarbonyl-3 phényl)-3 uréido]-2 acétyl}-1 méthylphénylcarbamoyl-4 phényl-5 pyrrolidinecarboxylate-2 de tert-butyle-(2RS,4RS,5SR) sous forme d'une meringue utilisée telle quelle dans les synthèses ultérieures.
C L'(amino-2 acétyl)-1 méthylphénylcarbamoyl-4 phényl-5 pyrrolidinecarboxylate-2 de tert-butyle-(2RS,4RS,5SR) peut être préparé comme décrit à l'exemple 16 C, mais à partir de 4,6 g de (tert-butoxycarbonylamino-2 acétyl)-1 méthylphénylcarbamoyl-4 phényl-5 pyrrolidinecarboxylate-2 de tert-butyle-(2RS,4RS,5SR) et de 1,2 cm³ d'iodotriméthylsilane dans 100 cm³ de chloroforme. Après traitement, on obtient 2,8 g d'(amino-2 acétyl)-1 méthylphénylcarbamoyl-4 phényl-5 pyrrolidinecarboxylate-2 de tert-butyle-(2RS,4RS,5SR) sous forme d'une meringue utilisée telle quelle dans les synthèses ultérieures.
D Le (tert-butoxycarbonylamino-2 acétyl)-1 méthylphénylcarbamoyl-4 phényl-5 pyrrolidinecarboxylate-2 de tert-butyle-(2RS,4RS,5SR) peut être préparé de la manière suivante : à une solution de 4,5 g de (tert-butoxycarbonylamino-2 acétyl)-1 phényl-5 pyrrolidinedicarboxylate-2,4 acide de (2) tert-butyle-(2RS,4SR,5SR) et de 1,1 cm³ de N-méthyl-aniline dans 60 cm³ d'acétonitrile, on ajoute 2,1 g de N,N'-dicyclohexylcarbodiimide et 0,1 g d'hydroxy-3 benzotriazole. Le mélange réactionnel est agité pendant vingt heures à une température voisine de 20°C, puis concentré sous pression réduite. Le résidu est repris par 50 cm³ d'acétate d'éthyle, filtré sur célite et le précipité rincé par deux fois 50 cm³ d'acétate d'éthyle. Le filtrat est concentré sous pression réduite Le résidu est repris par de l'oxyde de diisopropyle et filtré. Le précipité est lavé à l'oxyde de diisopropyle, séché sous pression réduite et purifié par chromatographie sur silice [éluant : cyclohexane-acétate d'éthyle (50-50 en volumes)]. Les fractions contenant le produit attendu sont réunies et concentrées sous pression réduite. On obtient ainsi 2,8 g de (tert-butoxycarbonylamino-2 acétyl)-1 méthylphénylcarbamoyl-4 phényl-5 pyrrolidinecarboxylate-2 de tert-butyle-(2RS,4RS,5SR) fondant à 182°C.

### Exemple 22

A On opère d'une manière analogue à celle décrite à l'exemple 2 A, mais à partir de 3,6 g de ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoate de benzyle-(2RS,4SR,5RS) et de 0,35 g de palladium à 10% sur charbon dans 200 cm³ d'éthanol. Après traitement, on obtient 1,1 g d'acide ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoïque-(2RS,4SR,5RS) [Rf = 0,21; éluant : chlorure de méthylène-méthanol (90/10)].
B Le ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoate de benzyle-(2RS,4SR,5RS) peut être préparé comme décrit à l'exemple 2 B, mais à partir de 2,6 g de (diméthyl-3,3 pipéridino)carbonyl-2 (fluoro-2 phényl)-5 phénylsulfonyl-4 pyrrolidine-(2RS,4SR,5RS), de 1,92 g d'acide [(benzyloxycarbonyl-3 phényl)-3 uréido]-2 acétique et de 1,21 g de N,N'-dicyclohexylcarbodiimide dans 50 cm³ d'acétonitrile. Après traitement, on obtient 3,6 g de ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoate de benzyle-(2RS,4SR,5RS) sous forme d'une meringue utilisée telle quelle dans les synthèses ultérieures.
C La (diméthyl-3,3 pipéridino)carbonyl-2 (fluoro-2 phényl)-5 phénylsulfonyl-4 pyrrolidine-(2RS,4SR,5RS) peut être préparée comme décrit à l'exemple 2 C, mais à partir de 2,5 g de diméthyl-3,3 [(fluoro-2 benzylidèneamino)-2-acétyl]-1 pipéridine, de 1,48 g de phénylvinylsulfone, de 2,2 g d'acétate d'argent et de 1,24 cm³ de triéthylamine dans 75 cm³ d'acétonitrile. Après traitement, on obtient 2,3 g de (diméthyl-3,3 pipéridino)carbonyl-2 (fluoro-2 phényl)-5 phénylsulfonyl-4 pyrrolidine-(2RS,4SR,5RS) sous forme d'une huile utilisée telle quelle dans les synthèses ultérieures et 1 g de (diméthyl-3,3 pipéridino)carbonyl-2 (fluoro-2 phényl)-5 phénylsulfonyl-4 pyrrolidine-(2RS,4RS,SRS) fondant à 210°C.
D La [(fluoro-2 benzylidèneamino)-2-acétyl]-1 diméthyl-3,3 pipéridine peut être préparée comme décrit à l'exemple 2 D, mais à partir de 0,92 cm³ de fluoro-2 benzaldéhyde, de 1,5 g d'(amino-2 acétyl)-1 diméthyl-3,3 pipéridine et de 2 g de tamis 4Å dans 50 cm³ de dichlorométhane. Après traitement, on obtient 2,5 g de [(fluoro-2 benzylidèneamino)-2-acétyl]-1 diméthyl-3,3 pipéridine sous forme d'une huile utilisée telle quelle dans les synthèses ultérieures.
E L'(amino-2 acétyl)-1 diméthyl-3,3 pipéridine peut être préparée de la manière suivante : à une solution de 7 g de (tert-butoxycarbonylamino-2 acétyl)-1 diméthyl-3,3 pipéridine dans 200 cm³ de dichlorométhane, on ajoute 11,9 cm³ d'acide trifluoroacétique. Le mélange réactionnel est agité pendant vingt heures à une température voisine de 20°C puis est amené à un pH voisin de 7 par addition d'une solution aqueuse saturée en hydrogénocarbonate de sodium. La phase organique est séparée par décantation et la phase aqueuse saturée en chlorure de sodium puis extraite par deux fois 100 cm³ de dichlorométhane. Les phases organiques sont réunies, séchées sur sulfate de magnésium et concentrées sous pression réduite. Le résidu est purifié par chromatographie sur silice [éluant dichlorométhane-méthanol (90-10 en volumes)]. Les fractions contenant le produit attendu sont réunies et concentrées sous pression réduite. On obtient ainsi 1,5 g d'(amino-2 acétyl)-1 diméthyl-3,3 pipéridine sous forme d'une huile utilisée telle quelle dans les synthèses ultérieures.
F La (tert-butoxycarbonylamino-2 acétyl)-1 diméthyl-3,3 pipéridine peut être préparée de la manière suivante : une solution de 7 g d'acide tert-butoxycarbonylamino-2 acétique et de 7,15 g de N,N-diimidazole carbonyle dans 100 cm³ de dichlorométhane est agitée pendant deux heures à une température voisine de 20°C. On ajoute ensuite 5,4 cm³ de diméthyl-3,3 pipéridine et 0,1 g de diméthylamino-4 pyridine. Le mélange réctionnel est agité pendant soixante-douze heures à une température voisine de 20°C puis versé dans 100 cm³ d'eau distillée. La phase organique est séparée par décantation, lavée par deux fois 50 cm³ d'une solution aqueuse normale d'acide chlorhydrique, 100 cm³ d'eau distillée puis séchée sur sulfate de magnésium et concentrée sous pression réduite. On obtient ainsi 7 g de (tert-butoxycarbonylamino-2 acétyl)-1 diméthyl-3,3 pipéridine sous forme d'une huile utilisée telle quelle dans les synthèses ultérieures.

### Exemple 23

A On opère d'une manière analogue à celle décrite à l'exemple 2 A, mais à partir de 1,6 g de ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoate de benzyle-(2RS,4RS,5RS) et de 0,15 g de palladium à 10% sur charbon dans 100 cm³ d'éthanol. Après traitement, on obtient 0,35 g d'acide ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoïque-(2RS,4RS,5RS) fondant à 258°C.
B Le ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoate de benzyle-(2RS,4RS,5RS) peut être préparé comme décrit à l'exemple 2 B, mais à partir de 1,12 g de (diméthyl-3,3 pipéridino)carbonyl-2 (fluoro-2 phényl)-5 phénylsulfonyl-4 pyrrolidine-(2RS,4RS,5RS), de 0,82 g d'acide [(benzyloxycarbonyl-3 phényl)-3 uréido]-2 acétique et de 0,52 g de N,N'-dicyclohexylcarbodiimide dans 50 cm³ d'acétonitrile. Après traitement, on obtient 1,6 g de ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoate de benzyle-(2RS,4RS,5RS) sous forme d'une meringue utilisée telle quelle dans les synthèses ultérieures.

### Exemple 24

A On opère d'une manière analogue à celle décrite à l'exemple 3, mais à partir de 3,6 g de (fluoro-2 phényl)-5 {[(méthoxycarbonylméthyl-3 phényl)-3 uréido]-2 acétyl}-1 pyrrolidinedicarboxylate-2,4 de (2) tert-butyle et de (4) méthyle-(2RS,4RS,5SR) et de 0,75 g d'hydroxyde de potassium dans un mélange de 20 cm³ d'eau distillée et de 60 cm³ de méthanol. Après traitement, on obtient 1 g de {[(carboxyméthyl-3 phényl)-3 uréido]-2 acétyl}-1 fluoro-2 phényl)-5 pyrrolidinedicarboxylate-2,4 acide de (2) tert-butyle-(2RS,4SR,5SR) [Rf = 0,06; éluant : chlorure de méthylène-méthanol (80/20)].
B Le (fluoro-2 phényl)-5 {[(méthoxycarbonylméthyl-3 phényl)-3 uréido]-2 acétyl}-1 pyrrolidinedicarboxylate-2,4 de (2) tert-butyle et de (4) méthyle-(2RS,4RS,5SR) peut être préparé comme décrit à l'exemple 2 B, mais à partir de 2,3 g de (fluoro-2 phényl)-5 pyrrolidinedicarboxylate-2,4 de (2) tert-butyle et de (4) méthyle-(2RS,4RS,5SR), de 1,89 g d'acide (méthoxycarbonylméthyl-3 phényl)-3 uréido]-2 acétique et de 1,47 g de N,N'-dicyclohexylcarbodiimide dans 50 cm³ d'acétonitrile. Après traitement, on obtient 3,8 g de (fluoro-2 phényl)-5 {[(méthoxycarbonylméthyl-3 phényl)-3 uréido]-2 acétyl}-1 pyrrolidinedicarboxylate-2,4 de (2) tert-butyle et de (4) méthyle-(2RS,4RS,5SR) sous forme d'une meringue utilisée telle quelle dans les synthèses ultérieures.
C Le (fluoro-2 phényl)-5 pyrrolidinedicarboxylate-2,4 de (2) tert-butyle et de (4) méthyle-(2RS,4RS,5SR) peut être préparé comme décrit à l'exemple 2 C, mais à partir de 24 g de (fluoro-2 benzylidèneamino)acétate de tert-butyle, de 9 cm³ d'acrylate de méthyle, de 25 g d'acétate d'argent et de 14 cm³ de triéthylamine dans 400 cm³ d'acétonitrile. Après traitement, on obtient 29 g de (fluoro-2 phényl)-5 pyrrolidinedicarboxylate-2,4 de (2) tert-butyle et de (4) méthyle-(2RS,4RS,5SR) sous frome d'une huile utilisée telle quelle dans les synthèses ultérieures.
D L'acide [(méthoxycarbonylméthyl-3 phényl)-3 uréido]-2 acétique peut être préparé comme décrit à l'exemple 1 G, mais à partir de 9,42 g de glycine et de 34,69 g de carbonate de potassium dans 220 cm³ d'eau et de 24 g d'isocyanato-3 phénylacétate de méthyle dissous dans 170 cm³ de dioxanne-1,4. Après traitement et recristallisation dans l'acétate d'éthyle, on obtient 46,85 g d'acide [(méthoxycarbonylméthyl-3 phényl)-3 uréido]-2 acétique fondant à 136°C.
E L'isocyanato-3 phénylacétate de méthyle peut être préparé de la manière suivante : à une suspension de 1 g de charbon et 6 cm³ de chloroformiate de trichlorométhyle dans 70 cm³ de toluène, on ajoute, à une température voisine de -20°C et sous argon, 8,25 g d'amino-3 phénylacétate de méthyle en solution dans 100 cm³ de toluène. Le mélange réactionnel est agité et maintenu à -20°C pendant quinze minutes, puis, après retour à une température voisine de 20°C, chauffé à reflux pendant deux heures trente minutes. Le mélange est alors dégazé par barbottage d'argon pendant trente minutes, filtré sur célite, rincé avec 50 cm³ de dichlorométhane et concentré sous pression réduite à une température voisine de 50°C. On obtient ainsi 9,30 g d'isocyanato-3 phénylacétate de méthyle sous forme d'une huile utilisée tel quel dans les synthèses ultérieures.

L'amino-3 phénylacétate de méthyle peut être préparé selon la méthode décrite par W.A. JACOBS et coll. J. Amer. Chem. Soc., 34, 2420 (1917)

### Exemple 25

A On opère d'une manière analogue à celle décrite à l'exemple 2 A, mais à partir de 3,6 g de ({[tert-butoxycarbonyl-2 (fluoro-2 phényl)-5 phénylsulfonyl-4 pyrrolidinyl-1]-2 oxo-2 éthyl}-3 uréido)-3 phénylacétate de benzyle-(2RS,4SR,5RS) et de 0,4 g de palladium à 10% sur charbon dans 200cm³ d'éthanol. Après traitement, on obtient 1,45 g d'acide ({[tert-butoxycarbonyl-2 (fluoro-2 phényl)-5 phénylsulfonyl-4 pyrrolidinyl-1]-2 oxo-2 éthyl}-3 uréido)-3 phénylacétique-(2RS,4SR,5RS) [Rf = 0,27; éluant : chlorure de méthylène-méthanol (80/20)].
B Le ({[tert-butoxycarbonyl-2 (fluoro-2 phényl)-5 phénylsulfonyl-4 pyrrolidinyl-1]-2 oxo-2 éthyl}-3 uréido)-3 phénylacétate de benzyle-(2RS,4SR,5RS) peut être préparé comme décrit à l'exemple 2 B, mais à partir de 1,02 g de (fluoro-2 phényl)-5 phénylsulfonyl-4 pyrrolidinecarboxylate-2 de tert-butyle-(2RS,4SR,5RS), de 0,86 g d'acide [(benzyloxycarbonylméthyl-3 phényl)-3 uréido]-2 acétique et de 0,52 g de N,N'-dicyclohexylcarbodiimide dans 50 cm³ d'acétonitrile. Après traitement, on obtient 1,8 g de ({[tert-butoxycarbonyl-2 (fluoro-2 phényl)-5 phénylsulfonyl-4 pyrrolidinyl-1]-2 oxo-2 éthyl}-3 uréido)-3 phénylacétate de benzyle-(2RS,4SR,5RS) sous forme d'une meringue utilisée telle quelle dans les synthèses ultérieures.
C L'acide [(benzyloxycarbonylméthyl-3 phényl)-3 uréido]-2 acétique peut être préparé comme décrit à l'exemple 1 G, mais à partir de 2,5 g de glycine et de 9,21 g de carbonate de potassium en solution dans 90 cm³ d'eau et de 8,96 g d'isocyanato-3 phénylacétate de benzyle en solution dans 75 cm³ de dioxanne-1,4. Après traitement, on obtient 6,75 g d'acide [(benzyloxycarbonylméthyl-3 phényl)-3 uréido]-2 acétique utilisé tel quel dans les synthèses ultérieures.
D L'isocyanato-3 phénylacétate de benzyle peut être préparé comme décrit à l'exemple 17 C, mais à partir de 8,55 g d'amino-3 phénylacétate de benzyle, de 4,74 cm³ de chloroformiate de trichlorométhyle et de 0,9 g de charbon végétal dans 130 cm³ de toluène. Après traitement, on obtient 8,96 g d'isocyanato-3 phénylacétate de benzyle sous forme d'une huile utilisée telle quelle dans les synthèses ultérieures.
E L'amino-3 phénylacétate de benzyle peut être préparé de la manière suivante : à une solution de 20,25 g de nitro-3 phénylacétate de benzyle dans un mélange de 100 cm³ de méthanol et de 1 I d'eau distillée, on ajoute 197,96 g de chlorure d'ammonium et 97,11 g de zinc en poudre. La suspension est chauffée au reflux pendant une heure puis refroidie à une température voisine de 20°C. Le milieu réactionnel est alors filtré et le filtrat extrait par trois fois 500 cm³ d'oxyde de diéthyle. Les extraits sont réunis et lavés par 250 cm³ d'une solution aqueuse saturée en chlorure de sodium, séchés sur sulfate de magnésium et concentrés sous pression réduite. Le résidu est purifié par chromatographie sur silice [éluant cyclohexane-acétate d'éthyle (85-15 puis 70-30 en volumes)]. Les fractions contenant le produit attendu sont réunies et concentrées sous pression réduite. On obtient ainsi 8,58 g d'amino-3 phénylacétate de benzyle sous forme d'une huile utilisée telle quelle dans les synthèses ultérieures.
F Le nitro-3 phénylacétate de benzyle peut être préparé de la manière suivante : à une solution de 13,6 g d'acide nitro-3 phénylacétique dans 130 cm³ de dichloro-1,2 éthane, on ajoute 0,1 cm³ de N,N-diméthyl-formamide puis 16,6 cm³ de chlorure d'oxalyle. Le mélange est agité pendant trois heures à une température voisine de 20°C, puis on ajoute 17,8 cm³ d'alcool benzylique. Le mélange réactionnel est agité pendant vingt heures à une température voisine de 20°C, dilué par 100 cm³ de dichlorométhane et 200 cm³ d'une solution aqueuse saturée en hydrogénocarbonate de sodium. La phase organique est séparée par décantation, lavée par deux fois 100 cm³ d'une solution aqueuse saturée en hydrogénocarbonate de sodium puis 100 cm³ d'une solution aqueuse saturée en chlorure de sodium. Les phases aqueuses sont extraites par deux fois 100 cm³ de dichlorométhane. Les phases organiques sont réunies, séchées sur sulfate de magnésium et concentrées sous pression réduite. Le résidu est purifié par chromatographie sur silice [éluant cyclohexane-acétate d'éthyle (50-50 en volumes)]. Les fractions contenant le produit attendu sont réunies et concentrées sous pression réduite. On obtient ainsi 20,26 g de nitro-3 phénylacétate de benzyle sous forme d'une huile utilisée telle quelle dans les synthèses ultérieures.

### Exemple 26

A On opère d'une manière analogue à celle décrite à l'exemple 3, mais à partir de 1,85 g d'{[(éthoxycarbonyl-3 phényl)-3 uréido]-2 acétyl}-1 phényl-5 (pyrrolidinyl-1)carbonyl-4 pyrrolidinecarboxylate-2 de tert-butyle-(2RS,4SR,5SR) et de 0,18 g d'hydroxyde de potassium dans un mélange de 20 cm³ d'eau distillée et de 50 cm³ de méthanol. Après traitement, on obtient 0,76 g d'acide {[(tert-butoxycarbonyl-2 phényl-5 (pyrrolidinyl-1) carbonyl-4 pyrrolidinyl-1)-2 oxo-2 éthyl]-3 uréido}-3 benzoïque-(2RS,4SR,5SR) [Rf = 0,17; éluant : chlorure de méthylène-méthanol (97,5/2,5)].
B L' {[(éthoxycarbonyl-3 phényl)-3 uréido]-2 acétyl}-1 phényl-5 (pyrrolidinyl-1)carbonyl-4 pyrrolidinecarboxylate-2 de tert-butyle-(2RS,4SR,5SR) peut être préparé comme décrit à l'exemple 17 B, mais à partir de 2,3 g d'(amino-2 acétyl)-1 phényl-5 (pyrrolidinyl-1)carbonyl-4 pyrrolidinecarboxylate-2 de tert-butyle-(2RS,4SR,5SR) et de 1,1 g d'isocyanato-3 benzoate d'éthyle dans 120 cm³ de tétrahydrofuranne. Après traitement, on obtient 1,87 g d'{[(éthoxycarbonyl-3 phényl)-3 uréido]-2 acétyl}-1 phényl-5 (pyrrolidinyl-1)carbonyl-4 pyrrolidinecarboxylate-2 de tert-butyle-(2RS,4SR,5SR) sous forme d'une meringue utilisée telle quelle dans les synthèses ultérieures.
C L'(amino-2 acétyl)-1 phényl-5 (pyrrolidinyl-1)carbonyl-4 pyrrolidinecarboxylate-2 de tert-butyle-(2RS,4SR,5SR) peut être préparé comme décrit à l'exemple 16 C, mais à partir de 3,65 g de (tert-butoxycarbonylamino-2 acétyl)-1 phényl-5 (pyrrolidinyl-1)carbonyl-4 pyrrolidinecarboxylate-2 de tert-butyle-(2RS,4SR,5SR) et de 1,04 cm³ d'iodotriméthylsilane dans 100 cm³ de chloroforme. Après traitement, on obtient 2,3 g d'(amino-2 acétyl)-1 phényl-5 (pyrrolidinyl-1)carbonyl-4 pyrrolidinecarboxylate-2 de tert-butyle-(2RS,4SR,5SR) sous forme d'une meringue utilisée telle quelle dans les synthèses ultérieures.
D Le (tert-butoxycarbonylamino-2 acétyl)-1 phényl-5 (pyrrolidinyl-1)carbonyl-4 pyrrolidinecarboxylate-2 de tert-butyle-(2RS,4SR,5SR) peut être préparé de la manière suivante : à une solution de 5,61 g de (tert-butoxycarbonylamino-2 acétyl)-1 phényl-5 pyrrolidinedicarboxylate-2,4 acide de (2) tert-butyle-(2RS,4SR,5SR) dans 75 cm³ de dichloro-1,2 éthane, on ajoute 2,23 g de N,N-diimidazole carbonyle. Le mélange réactionnel est agité pendant trois heures à une température voisine de 20°C, puis on ajoute 1,05 cm³ de pyrrolidine et 0,1 g de diméthylamino-4 pyridine. Le mélange réactionnel est agité pendant vingt heures à une température voisine de 20°C. La phase organique est ensuite lavée par 100 cm³ d'eau distillée, deux fois 100 cm³ d'une solution aqueuse normale d'acide chlorhydrique, deux fois 100 cm³ d'une solution aqueuse normale d'hydroxyde de sodium, deux fois 100 cm³ d'une solution aqueuse saturée en chlorure de sodium, séchée sur sulfate de sodium et concentrée sous pression réduite. Le résidu est purifié par chromatographie sur silice [éluant : cyclohexane-acétate d'éthyle (30-70 en volumes)]. Les fractions contenant le produit attendu sont réunies et concentrées sous pression réduite. On obtient ainsi 3,67 g de (tert-butoxycarbonylamino-2 acétyl)-1 phényl-5 (pyrrolidinyl-1)carbonyl-4 pyrrolidinecarboxylate-2 de tert-butyle(2RS,4SR,5SR) sous forme d'une meringue utilisée telle quelle dans les synthèses ultérieures.

### Exemple 27

A On opère d'une manière analogue à celle décrite à l'exemple 3, mais à partir de 2,3 g de (diméthyl-3,3 pipéridino)carbonyl-4 {[(éthoxycarbonyl-3 phényl)-3 uréido]-2 acétyl}-1 phényl-5 pyrrolidinecarboxylate-2 de tert-butyle-(2RS,4SR,5SR) et de 0,2 g d'hydroxyde de potassium dans un mélange de 20 cm³ d'eau distillée et de 50 cm³ de méthanol. Après traitement, on obtient 1,16 g d'acide {[(tert-butoxycarbonyl-2 (diméthyl-3,3 pipéridino)carbonyl-4 phényl-5 pyrrolidinyl-1)-2 oxo-2 éthyl]-3 uréido}-3 benzoïque-(2RS,4SR,5SR) [Rf = 0,2; éluant : chlorure de méthylène-méthanol (97,5/2,5)].
B Le (diméthyl-3,3 pipéridino)carbonyl-4 {[(éthoxycarbonyl-3 phényl)-3 uréido]-2 acétyl}-1 phényl-5 pyrrolidinecarboxylate-2 de tert-butyle-(2RS,4SR,5SR) peut être préparé comme décrit à l'exemple 17 B, mais à partir de 2,7 g d'(amino-2 acétyl)-1 (diméthyl-3,3 pipéridino)carbonyl-4 phényl-5 pyrrolidinecarboxylate-2 de tert-butyle-(2RS,4SR,5SR) et de 1,17 g d'isocyanato-3 benzoate d'éthyle dans 120 cm³ de tétrahydrofuranne. Après traitement, on obtient 2,3 g de (diméthyl-3,3 pipéridino)carbonyl-4 {[(éthoxycarbonyl-3 phényl)-3 uréido]-2 acétyl}-1 phényl-5 pyrrolidinecarboxylate-2 de tert-butyle-(2RS,4SR,5SR) sous forme d'une meringue utilisée telle quelle dans les synthèses ultérieures.
C L'(amino-2 acétyl)-1 (diméthyl-3,3 pipéridino)carbonyl-4 phényl-5 pyrrolidinecarboxylate-2 de tert-butyle-(2RS,4SR,5SR) peut être préparé comme décrit à l'exemple 16 C, mais à partir de 3,85 g de (tert-butoxycarbonylamino-2 acétyl)-1 (diméthyl-3,3 pipéridino)carbonyl-4 phényl-5 pyrrolidinecarboxylate-2 de tert-butyle-(2RS,4SR,5SR) et de 1,01 cm³ d'iodotriméthylsilane dans 100 cm³ de chloroforme. Après traitement, on obtient 2,7 g d'(amino-2 acétyl)-1 (diméthyl-3,3 pipéridino)carbonyl-4 phényl-5 pyrrolidinecarboxylate-2 de tert-butyle-(2RS,4SR,5SR) sous forme d'une meringue utilisée telle quelle dans les synthèses ultérieures.
D Le (tert-butoxycarbonylamino-2 acétyl)-1 (diméthyl-3,3 pipéridino)carbonyl-4 phényl-5 pyrrolidinecarboxylate-2 de tert-butyle-(2RS,4SR,5SR) peut être préparé comme décrit à l'exemple 26 D, mais à partir de 4,5 g de (tert-butoxycarbonylamino-2 acétyl)-1 phényl-5 pyrrolidinedicarboxylate-2,4 acide de (2) tert-butyle-(2RS,4SR,5SR), de 1,78 g de N,N-diimidazole carbonyle, de 1,35 cm³ de diméthyl-3,3 pipéridine et de 0,1 g de diméthylamino-4 pyridine dans 75 cm³ de dichloro-1,2 éthane. Après traitement, on obtient 3,86 g de (tert-butoxycarbonylamino-2 acétyl)-1 (diméthyl-3,3 pipéridino)carbonyl-4 phényl-5 pyrrolidinecarboxylate-2 de tert-butyle-(2RS,4SR,5SR) sous forme d'une meringue utilisée telle quelle dans les synthèses ultérieures.

### Exemple 28

A On opère d'une manière analogue à celle décrite à l'exemple 3, mais à partir de 2,62 g d'acétyl-4 {[(méthoxycarbonyl-3 phényl)-3 uréido]-2 acétyl}-1 phényl-5 pyrrolidinecarboxylate-2 de tert-butyle-(2RS,4RS,5SR) dans un mélange de 5 cm³ d'une solution aqueuse normale d'hydroxyde de potassium, de 25 cm³ d'eau distillée et de 100 cm³ de méthanol. Après traitement, on obtient 0,6 g d'acide {[(acétyl-4 tert-butoxycarbonyl-2 phényl-5 pyrrolidinyl-1)-2 oxo-2 éthyl]-3 uréido}-3 benzoïque-(2RS,4SR,5SR) fondant à 145°C.
B L'acétyl-4 {[(méthoxycarbonyl-3 phényl)-3 uréido]-2 acétyl}-1 phényl-5 pyrrolidinecarboxylate-2 de tert-butyle-(2RS,4RS,SSR) peut être préparé comme décrit à l'exemple 2 B, mais à partir de 4,35 g d'acétyl-4 phényl-5 pyrrolidinecarboxylate-2 de tert-butyle-(2RS,4RS,5SR), de 3,8 g d'acide [(méthoxycarbonyl-3 phényl)-3 uréido]-2 acétique et de 3,1 g de N,N'-dicyclohexylcarbodiimide dans 120 cm³ d'acétonitrile. Après traitement, on obtient 8 g d'acétyl-4 ([(méthoxycarbonyl-3 phényl)-3 uréido]-2 acétyl}-1 phényl-5 pyrrolidinecarboxylate-2 de tert-butyle-(2RS,4RS,5SR) sous forme d'une meringue utilisée telle quelle dans les synthèses ultérieures.
C L'acide [(méthoxycarbonyl-3 phényl)-3 uréido]-2 acétique peut être préparé comme décrit à l'exemple 1 G, mais à partir de 5,88 g d'isocyanato-3 benzoate de méthyle, de 2,5 g de glycine et de 9,2 g de carbonate de potassium dans 75 cm³ de dioxanne-1,4 et 90 cm³ d'eau distillée. Après traitement, on obtient 5,27 g d'acide [(méthoxycarbonyl-3 phényl)-3 uréido]-2 acétique fondant à 220°C.

### Exemple 29

A On opère d'une manière analogue à celle décrite à l'exemple 3, mais à partir de 1,1 g de (fluoro-2 phényl)-5 {[(méthoxycarbonylméthyl-3 phényl)-3 uréido]-2 acétyl}-1 méthylphénylcarbamoyl-2 pyrrolidinecarboxylate-4 de méthyle-(2RS,4RS,5SR) et de 0,21 g d'hydroxyde de potassium dans un mélange de 20 cm³ d'eau distillée et de 40 cm³ de méthanol. Après traitement, on obtient 0,5 g d'acide (fluoro-2 phényl)-5 {[(carboxyméthyl-3 phényl)-3 uréido]-2 acétyl}-1 méthylphénylcarbamoyl-2 pyrrolidinecarboxylique-4-(2RS,4SR,5SR) fondant à 164°C.
B Le (fluoro-2 phényl)-5 {[(méthoxycarbonylméthyl-3 phényl)-3 uréido]-2 acétyl}-1 méthylphénylcarbamoyl-2 pyrrolidinecarboxylate-4 de méthyle-(2RS,4RS,5SR) peut être préparé comme décrit à l'exemple 2 B, mais à partir de 0,95 g de (fluoro-2 phényl)-5 méthylphénylcarbamoyl-2 pyrrolidinecarboxylate-4 de méthyle-(2RS,4RS,5SR), de 0,69 g d'acide [(méthoxycarbonylméthyl-3 phényl)-3 uréido]-2 acétique et de 0,54 g de N,N'-dicyclohexylcarbodiimide dans 50 cm³ d'acétonitrile. Après traitement, on obtient 1,1 g de (fluoro-2 phényl)-5 {[(méthoxycarbonylméthyl-3 phényl)-3 uréido]-2 acétyl}-1 méthylphénylcarbamoyl-2 pyrrolidinecarboxylate-4 de méthyle-(2RS,4RS,5SR) sous forme d'une meringue utilisée telle quelle dans les synthèses ultérieures.
C Le (fluoro-2 phényl)-5 méthylphénylcarbamoyl-2 pyrrolidinecarboxylate-4 de méthyle-(2RS,4RS,5SR) peut être préparé de la manière suivante : à une solution de 1,2 g de tert-butoxycarbonyl-1 (fluoro-2 phényl)-5 méthylphénylcarbamoyl-2 pyrrolidinecarboxylate-4 de méthyle-(2RS,4RS,5SR) dans 50 cm³ de chloroforme, on ajoute 0,37 cm³ d'iodotriméthylsilane. Le mélange réactionnel est agité pendant vingt heures à une température voisine de 20°C, puis on ajoute 100 cm³ d'une solution aqueuse saturée en hydrogénocarbonate de sodium. La phase organique est séparée par décantation, lavée par une solution aqueuse saturée en chlorure de sodium, séchée sur sulfate de magnésium et concentrée sous pression réduite. On obtient ainsi 0,95 g de (fluoro-2 phényl)-5 méthylphénylcarbamoyl-2 pyrrolidinecarboxylate-4 de méthyle-(2RS,4RS,5SR) sous forme d'une huile utilisée telle quelle dans les synthèses ultérieures.
D Le tert-butoxycarbonyl-1 (fluoro-2 phényl)-5 méthylphénylcarbamoyl-2 pyrrolidinecarboxylate-4 de méthyle-(2RS,4RS,5SR) peut être préparé de la manière suivante : à une solution de 3,68 g de tert-butoxycarbonyl-1 (fluoro-2 phényl)-5 pyrrolidinedicarboxylate-2,4 acide de (4) méthyle-(2RS,4RS,5SR) dans un mélange de 1,1 cm³ de N-méthyl-aniline et de 50 cm³ d'acétonitrile, on ajoute 2,1 g de N,N'-dicyclohexylcarbodiimide. Le mélange réactionnel est agité pendant quatre heures à une température voisine de 20°C, puis est concentré sous pression réduite. Le résidu est repris par 100 cm³ d'acétate d'éthyle et filtré. Le précipité est rincé par 50 cm³ d'acétate d'éthyle puis les filtrats sont réunis et concentrés sous pression réduite. Le résidu est purifié par chromatographie sur silice [éluant : acétate d'éthyle-cyclohexane (50-50 en volumes)]. Les fractions contenant le produit attendu sont réunies et concentrées sous pression réduite. On obtient ainsi 1,2 g de tert-butoxycarbonyl-1 (fluoro-2 phényl)-5 méthylphénylcarbamoyl-2 pyrrolidinecarboxylate-4 de méthyle-(2RS,4RS,5SR) sous forme d'une huile utilisée telle quelle dans les synthèses ultérieures.
E Le tert-butoxycarbonyl-1 (fluoro-2 phényl)-5 pyrrolidinedicarboxylate-2,4 acide de (4) méthyle-(2RS,4RS,5SR) peut être préparé de la manière suivante : à une solution de 7,2 g de chlorhydrate de (fluoro-2 phényl)-5 pyrrolidinedicarboxylate-2,4 acide de (4) méthyle-(2RS,4RS,5SR) et de 4,9 g de carbonate de sodium dans 40 cm³ d'eau, on ajoute, à une température voisine de 10°C, une solution de 5,2 g de dicarbonate de di tert-butyle dans 30 cm³ de dioxanne-1,4. Le milieu réactionnel est agité pendant deux heures à une température voisine de 20°C. On ajoute ensuite 100 cm³ d'eau distillée et lave par deux fois 50 cm³ d'acétate d'éthyle. La phase aqueuse est acidifiée à un pH voisin de 1 par addition d'une solution aqueuse normale d'acide chlorhydrique et extraite par trois fois 100 cm³ de dichlorométhane. Les extraits organiques sont réunis, lavés par une solution aqueuse saturée en chlorure de sodium, séchés sur sulfate de magnésium et concentrés sous pression réduite. Le résidu est cristallisé dans l'oxyde de diisopropyle. On obtient ainsi 7,35 g de tert-butoxycarbonyl-1 (fluoro-2 phényl)-5 pyrrolidinedicarboxylate-2,4 acide de (4) méthyle-(2RS,4RS,5SR) fondant à 172°C.
F Le chlorhydrate de (fluoro-2 phényl)-5 pyrrolidinedicarboxylate-2,4 acide de (4) méthyle-(2RS,4RS,SSR) peut être préparé de la manière suivante : à une solution de 12 g de (fluoro-2 phényl)-5 pyrrolidinedicarboxylate-2,4 de (2) benzyle et de (4) méthyle-(2RS,4RS,5SR) dans 150 cm³ d'éthanol, on ajoute 0,6 g de palladium à 10% sur charbon. Le milieu réactionnel est agité pendant vingt heures à une température voisine de 20°C sous une pression de 129 kPa d'hydrogène, puis est purgé à l'azote. Le catalyseur est séparé par filtration et rincé par trois fois 25 cm³ d'une solution aqueuse normale d'acide chlorhydrique. Les phases aqueuses réunies sont concentrées sous pression réduite et le résidu repris dans le dichlorométhane et filtré. Le filtrat est concentré sous pression réduite et le résidu est cristallisé dans l'acétonitrile. On obtient ainsi 7,2 g de chlorhydrate de (fluoro-2 phényl)-5 pyrrolidinedicarboxylate-2,4 acide de (4) méthyle-(2RS,4RS,5SR) fondant à 212°C.
G Le (fluoro-2 phényl)-5 pyrrolidinedicarboxylate-2,4 de (2) benzyle et de (4) méthyle-(2RS,4RS,5SR) peut être préparé comme décrit à l'exemple 2 C, mais à partir de 11 g de (fluoro-2 benzylidèneamino)acétate de benzyle, de 3,6 cm³ d'acrylate de méthyle, de 10 g d'acétate d'argent et de 5,6 cm³ de triéthylamine dans 200 cm³ d'acétonitrile. Après traitement, on obtient 12 g de (fluoro-2 phényl)-5 pyrrolidinedicarboxylate-2,4 de (2) benzyle et de (4) méthyle-(2RS,4RS,5SR) sous forme d'une huile utilisée telle quelle dans les synthèses ultérieures.
H Le (fluoro-2 benzylidèneamino)acétate de benzyle peut être préparé comme décrit à l'exemple 2 D, mais à partir de 4,2 cm³ de fluoro-2 benzaldéhyde, de 8,07 g de chlorhydrate de glycinate de benzyle, de 6 g de tamis 4Å et de 5,6 cm³ de triéthylamine dans 75 cm³ de dichlorométhane. Après traitement, on obtient 11 g de (fluoro-2 benzylidèneamino)acétate de benzyle sous forme d'une huile utilisée telle quelle dans les synthèses ultérieures.

### Exemple 30

A On opère d'une manière analogue à celle décrite à l'exemple 3, mais à partir de 1,6 g de (diméthyl-3,3 pipéridino)carbonyl-2 (fluoro-2 phényl)-5 {[(méthoxycarbonylméthyl-3 phényl)-3 uréido]-2 acétyl}-1 pyrrolidinecarboxylate-4 de méthyle-(2RS,4RS,5SR) et de 0,3 g d'hydroxyde de potassium dans un mélange de 20 cm³ d'eau distillée et de 40 cm³ de méthanol. Après traitement, on obtient 0,55 g d'acide {[(carboxyméthyl-3 phényl)-3 uréido]-2 acétyl}-1 (diméthyl-3,3 pipéridino)carbonyl-2 (fluoro-2 phényl)-5 pyrrolidinecarboxylique-4-(2RS,4SR,5SR) fondant à 173°C.
B Le (diméthyl-3,3 pipéridino)carbonyl-2 (fluoro-2 phényl)-5 {[(méthoxycarbonylméthyl-3 phényl)-3 uréido]-2 acétyl}-1 pyrrolidinecarboxylate-4 de méthyle-(2RS,4RS,5SR) peut être préparé comme décrit à l'exemple 2 B, mais à partir de 1,2 g de (diméthyl-3,3 pipéridino)carbonyl-2 (fluoro-2 phényl)-5 pyrrolidinecarboxylate-4 de méthyle-(2RS,4RS,5SR), de 0,86 g d'acide [(méthoxycarbonylméthyl-3 phényl)-3 uréido]-2 acétique et de 0,67 g de N,N'-dicyclohexylcarbodiimide dans 50 cm³ d'acétonitrile. Après traitement, on obtient 1,6 g de (diméthyl-3,3 pipéridino)carbonyl-2 (fluoro-2 phényl)-5 {[(méthoxycarbonylméthyl-3 phényl)-3 uréido]-2 acétyl}-1 pyrrolidinecarboxylate-4 de méthyle-(2RS,4RS,5SR) sous forme d'une meringue utilisée telle quelle dans les synthèses ultérieures.
C Le (diméthyl-3,3 pipéridino)carbonyl-2 (fluoro-2 phényl)-5 pyrrolidinecarboxylate-4 de méthyle-(2RS,4RS,5SR) peut être préparé comme décrit à l'exemple 29 C, mais à partir de 1,5 g de tert-butoxycarbonyl-1 (diméthyl-3,3 pipéridino)carbonyl-2 (fluoro-2 phényl)-5 pyrrolidinecarboxylate-4 de méthyle-(2RS,4RS,5SR) et de 0,47 g d'iodotriméthylsilane dans 50 cm³ de chloroforme. Après traitement, on obtient 1,2 g de (diméthyl-3,3 pipéridino)carbonyl-2 (fluoro-2 phényl)-5 pyrrolidinecarboxylate-4 de méthyle-(2RS,4RS,5SR) sous forme d'une huile utilisée telle quelle dans les synthèses ultérieures.
D Le tert-butoxycarbonyl-1 (diméthyl-3,3 pipéridino)carbonyl-2 (fluoro-2 phényl)-5 pyrrolidinecarboxylate-4 de méthyle-(2RS,4RS,5SR) peut être préparé de la manière suivante : à une solution de 3,48 g de tert-butoxycarbonyl-1 (fluoro-2 phényl)-5 pyrrolidinedicarboxylate-2,4 acide de (4) méthyle-(2RS,4RS,5SR) dans 50 cm³ de dichloro-1,2 éthane, on ajoute 1,78 g de N,N-diimidazole carbonyle. Le mélange est agité pendant deux heures à une température voisine de 20°C, puis on ajoute 1,35 cm³ de diméthyl-3,3 pipéridine diluée dans 20 cm³ de dichloro-1,2 éthane et 0,1 g de diméthylamino-4 pyridine. Le milieu réactionnel est agité pendant vingt heures à une température voisine de 20°C puis versé dans 100 cm³ d'eau distillée. La phase organique est séparée par décantation, lavée par deux fois 50 cm³ d'une solution aqueuse décinormale d'acide chlorhydrique puis par une solution aqueuse saturée en chlorure de sodium, séchée sur sulfate de magnésium et concentrée sous pression réduite. Le résidu est purifié par chromatographie sur silice [éluant : acétate d'éthyle-cyclohexane (30-70 en volumes)]. Les fractions contenant le produit attendu sont réunies et concentrées sous pression réduite. On obtient ainsi 1,5 g de tert-butoxycarbonylamino-1 (diméthyl-3,3 pipéridino)carbonyl-2 (fluoro-2 phényl)-5 pyrrolidinecarboxylate-4 de méthyle-(2RS,4RS,5SR) sous forme d'une meringue utilisée telle quelle dans les synthèses ultérieures.

### Exemple 31

A On opère d'une manière analogue à celle décrite à l'exemple 3, mais à partir de 1,34 g de diméthylcarbamoyl-4 {[(méthoxycarbonyl-3 phényl)-3 uréido]-2 acétyl}-1 phényl-5 pyrrolidinecarboxylate-2 de tert-butyle-(2RS,4SR,5SR) et de 0,14 g d'hydroxyde de potassium dans un mélange de 40 cm³ d'eau distillée et de 60 cm³ de méthanol. Après traitement, on obtient 0,6 g d'acide {[(tert-butoxycarbonyl-2 diméthylcarbamoyl-4 phényl-5 pyrrolidinyl-1)-2 oxo-2 éthyl]-3 uréido}-3 benzoïque-(2RS,4SR,5SR) [Rf = 0,2; éluant : chlorure de méthylène-méthanol (90/10)].
B Le diméthylcarbamoyl-4 {[(méthoxycarbonyl-3 phényl)-3 uréido]-2 acétyl}-1 phényl-5 pyrrolidinecarboxylate-2 de tert-butyle-(2RS,4SR,5SR) peut être préparé comme décrit à l'exemple 17 B, mais à partir de 0,92 g d'(amino-2 acétyl)-1 diméthylcarbamoyl-4 phényl-5 pyrrolidinecarboxylate-2 de tert-butyle-(2RS,4SR,5SR) et de 0,46 g d'isocyanato-3 benzoate de méthyle dans 70 cm³ de tétrahydrofuranne. Après traitement, on obtient 1,34 g de diméthylcarbamoyl-4 {[(méthoxycarbonyl-3 phényl)-3 uréido]-2 acétyl}-1 phényl-5 pyrrolidinecarboxylate-2 de tert-butyle-(2RS,4SR,5SR) sous forme d'une meringue utilisée telle quelle dans les synthèses ultérieures.
C L'(amino-2 acétyl)-1 diméthylcarbamoyl-4 phényl-5 pyrrolidinecarboxylate-2 de tert-butyle-(2RS,4SR,5SR) peut être préparé comme décrit à l'exemple 16 C, mais à partir de 1,7 g de (tert-butoxycarbonylamino-2 acétyl)-1 diméthylcarbamoyl-4 phényl-5 pyrrolidinecarboxylate-2 de tert-butyle-(2RS,4SR,5SR) et de 0,51 cm³ d'iodotriméthylsilane dans 50 cm³ de chloroforme. Après traitement, on obtient 0,97 g d'(amino-2 acétyl)-1 diméthylcarbamoyl-4 phényl-5 pyrrolidinecarboxylate-2 de tert-butyle-(2RS,4SR,5SR) sous forme d'une huile utilisée telle quelle dans les synthèses ultérieures.
D Le (tert-butoxycarbonylamino-2 acétyl)-1 diméthylcarbamoyl-4 phényl-5 pyrrolidinecarboxylate-2 de tert-butyle-(2RS,4SR,5SR) peut être préparé de la manière suivante : à une solution de 2 g de (tert-butoxycarbonylamino-2 acétyl)-1 phényl-5 pyrrolidinedicarboxylate-2,4 acide de (2) tert-butyle-(2RS,4SR,5SR) dans 60 cm³ d'oxyde de diéthyle, on ajoute 0,1 g de N,N-diméthyl-formamide puis 0,42 cm³ de chlorure d'oxalyle. Le milieu réactionnel est agité pendant quatre heures à une température voisine de 20°C, puis on introduit de la diméthylamine par barbottage pendant quarante-cinq minutes. Le mélange est agité pendant vingt heures à une température voisine de 20°C. Le précipité qui s'est formé est séparé par filtration et est lavé par trois fois 20 cm³ de dichlorométhane. Les phases organiques sont réunies, diluées par 100 cm³ de dichlorométhane et lavées successivement par deux fois 150 cm³ d'eau, deux fois 150 cm³ d'une solution aqueuse décinormale d'acide chlorhydrique, 150 cm³ d'eau et deux fois 150 cm³ d'une solution aqueuse saturée en chlorure de sodium. La phase organique est séchée sur sulfate de magnésium et concentrée sous pression réduite. Le résidu est purifié par chromatographie sur silice [éluant: dichlorométhane-méthanol (90-10 en volumes)]. Les fractions contenant le produit attendu sont réunies et concentrées sous pression réduite. On obtient ainsi 1 g de (tert-butoxycarbonylamino-2 acétyl)-1 diméthylcarbamoyl-4 phényl-5 pyrrolidinecarboxylate-2 de tert-butyle-(2RS,4SR,5SR) sous forme d'une meringue utilisée telle quelle dans les synthèses ultérieures.

### Exemple 32

A On opère d'une manière analogue à celle décrite à l'exemple 2 A, mais à partir de 2,6 g de {[(benzyloxycarbonyl-3 phényl)-3 uréido]-2 acétyl}-1 phényl-5 phénylcarbamoyl-4 pyrrolidinecarboxylate-2 de tert-butyle-(2RS,4RS,5SR) et de 0,3 g de palladium à 10% sur charbon dans 150 cm³ d'éthanol. Après traitement, on obtient 2,05 g d'acide {[(tert-butoxycarbonyl-2 phényl-5 phénylcarbamoyl-4 pyrrolidinyl-1)-2 oxo-2 éthyl]-3 uréido}-3 benzoïque-(2RS,4RS,5SR) [Rf = 0,15; éluant : chlorure de méthylène-méthanol (80/20)].
B Le {[(benzyloxycarbonyl-3 phényl)-3 uréido]-2 acétyl}-1 phényl-5 phénylcarbamoyl-4 pyrrolidinecarboxylate-2 de tert-butyle-(2RS,4RS,5SR) peut être préparé de la manière suivante : à une solution de 1,13 g de N,N'-diimidazole carbonyle dans 30 cm³ de dichlorométhane, on ajoute une solution de 2,69 g d'(amino-2 acétyl)-1 phényl-5 phénylcarbamoyl-4 pyrrolidinecarboxylate-2 de tert-butyle-(2RS,4RS,5SR) dans 20 cm³ de dichlorométhane. Le milieu réactionnel est agité pendant deux heures à une température voisine de 20°C, puis on ajoute une solution de 1,44 g d'amino-3 benzoate de benzyle dans 25 cm³ de dichlorométhane. Le milieu réactionnel est ensuite chauffé à une température voisine de 40°C pendant vingt heures. Après refroidissement à une température voisine de 20°C, le milieu réactionnel est dilué par 150 cm³ de dichlorométhane. La phase organique est ensuite lavée successivement par deux fois 150 cm³ d'eau distillée, deux fois 150 cm³ d'une solution aqueuse décinormale d'acide chlorhydrique, 150 cm³ d'eau distillée et deux fois 150 cm³ d'une solution aqueuse saturée en chlorure de sodium. La phase organique est séchée sur sulfate de magnésium et concentrée sous pression réduite. Le résidu est purifié par deux chromatographies sur silice [éluants : cyclohexane-acétate d'éthyle (30-70 puis 50-50 en volumes)]. Les fractions contenant le produit attendu sont réunies et concentrées sous pression réduite. On obtient ainsi 2,6 g de {[(benzyloxycarbonyl-3 phényl)-3 uréido]-2 acétyl}-1 phényl-5 phénylcarbamoyl-4 pyrrolidinecarboxylate-2 de tert-butyle-(2RS,4RS,5SR) sous forme d'une meringue utilisée telle quelle dans les synthèses ultérieures.
C L'(amino-2 acétyl)-1 phényl-5 phénylcarbamoyl-4 pyrrolidinecarboxylate-2 de tert-butyle-(2RS,4RS,5SR) peut être préparé comme décrit à l'exemple 16 C, mais à partir de 5,05 g de (tert-butoxycarbonylamino-2 acétyl)-1 phényl-5 phénylcarbamoyl-4 pyrrolidinecarboxylate-2 de tert-butyle-(2RS,4RS,5SR) et de 0,83 cm³ d'iodotriméthylsilane dans 100 cm³ de chloroforme. Après traitement, on obtient 2,69 g d'(amino-2 acétyl)-1 phényl-5 phénylcarbamoyl-4 pyrrolidinecarboxylate-2 de tert-butyle-(2RS,4RS,5SR) sous forme d'une huile utilisée telle quelle dans les synthèses ultérieures.
D Le (tert-butoxycarbonylamino-2 acétyl)-1 phényl-5 phénylcarbamoyl-4 pyrrolidinecarboxylate-2 de tert-butyle-(2RS,4RS,5SR) peut être préparé comme décrit à l'exemple 20 D, mais à partir de 4,74 g de (tert-butoxycarbonylamino-2 acétyl)-1 phényl-5 pyrrolidinecarboxylate-2,4 acide de (2) tert-butyle-(2RS,4RS,5SR), de 1 cm³ d'aniline et de 2,2 g de N,N'-dicyclohexylcarbodiimide dans 50 cm³ d'acétonitrile. Après traitement, on obtient 3,07 g de (tert-butoxycarbonylamino-2 acétyl)-1 phényl-5 phénylcarbamoyl-4 pyrrolidinecarboxylate-2 de tert-butyle-(2RS,4RS,5SR) sous forme d'une meringue utilisée telle quelle dans les synthèses ultérieures.
E Le (tert-butoxycarbonylamino-2 acétyl)-1 phényl-5 pyrrolidinedicarboxylate.2,4 acide de (2) tert-butyle-(2RS,4RS,5SR) peut être préparé de la manière suivante : une suspension de 0,6 g de palladium à 5% sur charbon dans une solution de 5,74 g de (tert-butoxycarbonylamino-2 acétyl)-1 phényl-5 pyrrolidinedicarboxylate-2,4 de (4) benzyle et de (2) tert-butyle-(2RS,4RS,5SR) dans 350 cm³ de méthanol est agitée pendant une heure sous une atmospère d'hydrogène (130 kPa) et à une température voisine de 20°C. Le catalyseur est séparé par filtration et rincé par trois fois 50 cm³ de méthanol. Le filtrat est concentré sous pression réduite. on obtient ainsi 4,74 g de (tert-butoxycarbonylamino-2 acétyl)-1 phényl-5 pyrrolidinedicarboxylate-2,4 acide de (2) tert-butyle-(2RS,4RS,5SR) sous forme d'une meringue utilisée telle quelle dans les synthèses ultérieures.
F Le (tert-butoxycarbonylamino-2 acétyl)-1 phényl-5 pyrrolidinedicarboxylate-2,4 de (4) benzyle et de (2) tert-butyle-(2RS,4RS,5SR) peut être préparé comme décrit à l'exemple 16 D, mais à partir de 3,8 g de phényl-5 pyrrolidinedicarboxylate-2,4 de (4) benzyle et de (2) tert-butyle-(2RS,4RS,5SR), de 1,75 g d'acide tert-butoxycarbonylamino-2 acétique et de 2,06 g de N,N'-dicyclohexylcarbodiimide dans 50 cm³ d'acétonitrile. Après traitement, on obtient 2,06 g de (tert-butoxycarbonylamino-2 acétyl)-1 phényl-5 pyrrolidinedicarboxylate-2,4 de (4) benzyle et de (2) tert-butyle-(2RS,4RS,5SR) sous forme d'une meringue utilisée telle quelle dans les synthèses ultérieures.

### Exemple 33

On opère d'une manière analogue à celle décrite à l'exemple 10, mais à partir de 1,7 g d'acide {[(acétyl-4 tert-butoxycarbonyl-2 phényl-5 pyrrolidinyl-1)-2 oxo-2 éthyl]-3 uréido}-3 benzoïque-(2RS,4SR,5SR) et de 0,26 g de chlorure d'hydroxylammonium dans un mélange de 3 cm³ de pyridine, de 6 cm³ de méthanol et de 3 cm³ d'eau distillée. Après traitement, on obtient 0,43 g d'acide ({[tert-butoxycarbonyl-2 (hydroxyimino-1 éthyl)-4 phényl-5 pyrrolidinyl-1]-2 oxo-2 éthyl}-3 uréido)-3 benzoique-(2RS,4SR,5SR) fondant à 180°C.

### Exemple 34

A On opère d'une manière analogue à celle décrite à l'exemple 3, mais à partir de 1,1 g d'{[(éthoxycarbonyl-3 phényl)-3 uréido]-2 acétyl}-1 (fluoro-2 phényl)-5 (phényl-2 dioxolann-1,3-yl-2)-4 pyrrolidinecarboxylate-2 de tert-butyle-(2R5,45R,5SR) et de 0,09 g d'hydroxyde de potassium dans 10 cm³ d'eau distillée et 30 cm³ de méthanol. Après traitement, on obtient 0,15 g d'acide ({[benzoyl-4 tert-butoxycarbonyl-2 (fluoro-2 phényl)-5 pyrrolidinyl-1]-2 oxo-2 éthyl}-3 uréido)-3 benzoïque-(2RS-4SR,5SR) [Rf = 0,27; éluant : chlorure de méthylène-méthanol (90/10)] et 0,35 g de benzoyl-4 (fluoro-2 phényl)-5 {[(méthoxycarbonyl-3 phényl)-3 uréido]-2 acétyl}-1 pyrrolidinecarboxylate-2 de tert-butyle-(2RS,4SR,5SR) [Rf = 0,13; éluant : cyclohexane-acétate d'éthyle (70/30)].
B L'{[(éthoxycarbonyl-3 phényl)-3 uréido]-2 acétyl}-1 (fluoro-2 phényl)-5 (phényl-2 dioxolann-1,3-yl-2)-4 pyrrolidinecarboxylate-2 de tert-butyle-(2RS,4SR,5SR) peut être préparé de la manière suivante : une solution de 1 g de benzoyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5SR) et de 0,05g d'acide para-toluènesulfonique dans un mélange de 0,09 cm³ d'éthylène glycol et de 100 cm³ de toluène est chauffée au reflux pendant quatre heures. Après refroidissement à une température voisine de 20°C, la phase organique est lavée à l'eau, séchée sur sulfate de magnésium et concentrée sous pression réduite. On obtient ainsi 1,1 g d'{[(éthoxycarbonyl-3 phényl)-3 uréido]-2 acétyl}-1 (fluoro-2 phényl)-5 (phényl-2 dioxolann-1,3-yl-2)-4 pyrrolidinecarboxylate-2 de tert-butyle-(2RS,4SR,5SR) sous forme d'une huile utilisée telle quelle dans les synthèses ultérieures.
C Le benzoyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5SR) peut être préparé de la manière suivante : une solution de 1,3 g de benzoyl-4 {[(éthoxycarbonyl-3 phényl)-3 uréido]-2 acétyl}-1 (fluoro-2 phényl)-5 pyrrolidinecarboxylate-2 de tert-butyle-(2RS,4RS,5SR) et de 0,12 g d'hydroxyde de potassium dans un mélange de 30 cm³ de méthanol et de 10 cm³ d'eau distillée est agitée à une température voisine de 20°C pendant 4 jours. Le milieu réactionnel est concentré sous pression réduite, dilué par 100 cm³ d'eau distillée et amené à pH voisin de 1 par une solution aqueuse normale d'acide chlorhydrique. La phase aqueuse est extraite par trois fois 100 cm³ d'acétate d'éthyle. Les extraits organiques sont réunis, lavés par une solution aqueuse saturée en chlorure de sodium et concentrés sous pression réduite. Le résidu est purifié par chromatographie sur silice [éluant : dichlorométhane-méthanol (90/10 en volumes)]. Les fractions contenant le produit attendu sont réunies et concentrées sous pression réduite. On obtient ainsi 1 g de benzoyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5SR) sous forme de solide amorphe utilisé tel quel dans les synthèses ultérieures.
D Le benzoyl-4 {[(éthoxycarbonyl-3 phényl)-3 uréido]-2 acétyl}-1 (fluoro-2 phényl)-5 pyrrolidinecarboxylate-2 de tert-butyle-(2RS,4RS,5SR) peut être préparé comme décrit à l'exemple 2 B, mais à partir de 1,26 g de benzoyl-4 (fluoro-2 phényl)-5 pyrrolidinecarboxylate-2 de tert-butyle-(2RS,4RS,5SR), de 0,91 g d'acide [(éthoxycarbonyl-3 phényl)-3 uréido]-2 acétique et de 0,7 g de N,N'-dicyclohexylcarbodiimide dans 50 cm³ d'acétonitrile. Après traitement, on obtient 2 g de benzoyl-4 {[(éthoxycarbonyl-3 phényl)-3 uréido]-2 acétyl}-1 (fluoro-2 phényl)-5 pyrrolidinecarboxylate-2 de tert-butyle-(2RS,4RS,5SR) sous forme de solide amorphe utilisé tel quel dans les synthèses ultérieures.
E Le benzoyl-4 (fluoro-2 phényl)-5 pyrrolidinecarboxylate-2 de tert-butyle-(2RS,4RS,5SR) peut être préparé comme décrit à l'exemple 2 C, mais à partir de 7,1 g de (fluoro-2 benzylidèneamino)acétate de tert-butyle, de 3,9 g de phényl vinyl cétone, de 7,5 g d'acétate d'argent et de 4,2 cm³ de triéthylamine dans 200 cm³ d'acétonitrile. Après traitement, on obtient 2,5 g de benzoyl-4 (fluoro-2 phényl)-5 pyrrolidinecarboxylate-2 de tert-butyle-(2RS,4RS,5SR) sous forme de solide amorphe utilisé tel quel dans les synthèses ultérieures.

La phényl vinyl cétone peut être préparée selon la méthode décrite par R.T. PARFIT et coll., Eur. J. Med. Chem., 20, 228 (1985).

F L'acide [(éthoxycarbonyl-3 phényl)-3 uréido]-2 acétique peut être préparé comme décrit à l'exemple 1 G, mais à partir de 10 g d'isocyanato-3 benzoate d'éthyle, de 3,95 g de glycine et de 4,4 g d'hydrogénocarbonate de sodium dans 60 cm³ d'eau distillée. Après traitement, on obtient 5,3 g d'acide [(éthoxycarbonyl-3 phényl)-3 uréido]-2 acétique fondant à 174°C.

### Exemple 35

A On opère d'une manière analogue à celle décrite à l'exemple 3, mais à partir de 2,1 g de ({[(fluoro-2 phényl)-5 méthylphénylcarbamoyl-2 phénylsulfonyl-4 pyrrolidinyl-1]-2 oxo-2 éthyl}-3 uréido)-3 phénylacétate de méthyle-(2RS,4SR,5RS) et de 0,17 g d'hydroxyde de potassium dans un mélange de 20 cm³ d'eau distillée et de 60 cm³ de méthanol. Après traitement, on obtient 0,8 g d'acide ({[(fluoro-2 phényl)-5 méthylphénylcarbamoyl-2 phénylsulfonyl-4 pyrrolidinyl-1]-2 oxo-2 éthyl}-3 uréido)-3 phénylacétique-(2RS,4SR,5RS) [Rf = 0,26; éluant : chlorure de méthylène-méthanol (90/10)].
B Le {([(fluoro-2 phényl)-5 méthylphénylcarbamoyl-2 phénylsulfonyl-4 pyrrolidinyl-1]-2 oxo-2 éthyl}-3 uréido)-3 phénylacétate de méthyle-(2RS,4SR,5RS) peut être préparé comme décrit à l'exemple 2 B, mais à partir de 1,9 g de (fluoro-2 phényl)-5 phénylsulfonyl-4 N-méthyl N-phénylpyrrolidinecarboxamide-2-(2RS,4SR,5RS), de 1,15 g d'acide (méthoxycarbonylméthyl-3 phényl)-3 uréido]-2 acétique et de 0,9 g de N,N'-dicyclohexylcarbodiimide dans 50 cm³ d'acétonitrile. Après traitement, on obtient 2,1 g de ({[(fluoro-2 phényl)-5 méthylphénylcarbamoyl-2 phénylsulfonyl-4 pyrrolidinyl-1]-2 oxo-2 éthyl}-3 uréido)-3 phénylacétate de méthyle-(2RS,4SR,5RS) sous forme d'une meringue utilisée telle quelle dans les synthèses ultérieures.
C Le (fluoro-2 phényl)-5 phénylsulfonyl-4 N-méthyl N-phényl-pyrrolidinecarboxamide-2-(2RS,4SR,5RS) peut être préparé comme décrit à l'exemple 29 C, mais à partir de 2,5 g de tert-butoxycarbonyl-1 (fluoro-2 phényl)-5 phénylsulfonyl-4 N-méthyl N-phényl-pyrrolidinecarboxamide-2-(2RS,4SR,5RS) et de 0,66 cm³ d'iodotriméthylsilane dans 50 cm³ de chloroforme. Après traitement, on obtient 1,9 g de (fluoro-2 phényl)-5 phénylsulfonyl-4 N-méthyl N-phényl-pyrrolidinecarboxamide-2-(2RS,4SR,5RS) sous forme d'une meringue utilisée telle quelle dans les synthèses ultérieures.
D Le tert-butoxycarbonyl-1 (fluoro-2 phényl)-5 phénylsulfonyl-4 N-méthyl N-phényl-pyrrolidinecarboxamide-2-(2RS,4SR,5RS) peut être préparé comme décrit à l'exemple 29 D, mais à partir de 4,5 g d'acide tert-butoxycarbonyl-1 (fluoro-2 phényl)-5 phénylsulfonyl-4 pyrrolidinecarboxylique-2-(2RS,4SR,5RS), de 1,1 cm³ de N-méthyl-aniline et de 2,1 g de N,N'-dicyclohexylcarbodiimide dans 100 cm³ d'acétonitrile. Après traitement, on obtient 2,5 g de tert-butoxycarbonyl-1 (fluoro-2 phényl)-5 phénylsulfonyl-4 N-méthyl N-phényl-pyrrolidinecarboxamide-2-(2RS,4SR,5RS) sous forme d'une meringue utilisée telle quelle dans les synthèses ultérieures.
E L'acide tert-butoxycarbonyl-1 (fluoro-2 phényl)-5 phénylsulfonyl-4 pyrrolidinecarboxylique-2-(2RS,4SR,5RS) peut être préparé comme décrit à l'exemple 29 E, mais à partir de 5 g de chlorhydrate de l'acide (fluoro-2 phényl)-5 phénylsulfonyl-4 pyrrolidinecarboxylique-2-(2RS,4SR,5RS), de 6,5 g de dicarbonate de di tert-butyle et de 5,8 g de carbonate de sodium dans un mélange de 60 cm³ d'eau distillée et de 30 cm³ de dioxanne-1,4. Après traitement, on obtient 5 g d'acide tert-butoxycarbonyl-1 (fluoro-2 phényl)-5 phénylsulfonyl-4 pyrrolidinecarboxylique-2-(2RS,4SR,5RS) fondant à 186°C.
F Le chlorhydrate de l'acide (fluoro-2 phényl)-5 phénylsulfonyl-4 pyrrolidinecarboxylique-2-(2RS,4SR,5RS) peut être préparé de la manière suivante : à une solution de 15,3 g de chlorhydrate de (fluoro-2 phényl)-5 phénylsulfonyl-4 pyrrolidinecarboxylate-2 de benzyle-(2RS,4SR,5RS) dans un mélange de 250 cm³ de méthanol et de 80 cm³ d'eau distillée, on ajoute 3,6 g d'hydroxyde de potassium. Le mélange réactionnel est agité pendant vingt heures à une température voisine de 20°C puis est concentré sous pression réduite. Le résidu est repris dans 100 cm³ d'eau distillée. La phase aqueuse est lavée par deux fois 100 cm³ d'acétate d'éthyle, acidifiée à un pH voisin de 1 par une solution aqueuse 4N d'acide chlorhydrique et refroidie pendant quinze minutes à une température voisine de 4°C. Le solide qui précipite est séparé par filtration, lavé par trois fois 50 cm³ d'eau distillée et séché sous pression réduite à une temérature voisine de 40°C. On obtient ainsi 10,3 g de chlorhydrate de l'acide (fluoro-2 phényl)-5 phénylsulfonyl-4 pyrrolidinecarboxylique-2-(2RS,4SR,5RS) fondant à 212°C.
G Le chlorhydrate de (fluoro-2 phényl)-5 phénylsulfonyl-4 pyrrolidinecarboxylate-2 de benzyle-(2RS,4SR,5RS) peut être préparé comme décrit à l'exemple 2 C, mais à partir de 27,4 g de (fluoro-2 benzylidèneamino)acétate de benzyle, de 16,85 g de phénylvinylsulfone, de 25,1 g d'acétate d'argent et de 12 cm³ de triéthylamine dans 400 cm³ d'acétonitrile. Après traitement, on obtient 15,3 g de chlorhydrate de (fluoro-2 phényl)-5 phénylsulfonyl-4 pyrrolidinecarboxylate-2 de benzyle-(2RS,4SR,5RS)

### Exemple 36

A On opère d'une manière analogue à celle décrite à l'exemple 3, mais à partir de 2,5 g de ({[(fluoro-2 phényl)-5 phénylsulfonyl-4 (pyrrolidinyl-1 carbonyl)-2 pyrrolidinyl-1]-2 oxo-2 éthyl}-3 uréido)-3 phénylacétate de méthyle-(2RS,4SR,5RS) et de 0,22 g d'hydroxyde de potassium dans un mélange de 20 cm³ d'eau distillée et de 60 cm³ de méthanol. Après traitement, on obtient 1,6 g d'acide ({[(fluoro-2 phényl)-5 phénylsulfonyl-4 (pyrrolidinyl-1 carbonyl)-2 pyrrolidinyl-1]-2 oxo-2 éthyle-3 uréido)-3 phénylacétique-(2RS,4SR,5RS) [Rf = 0,2; éluant : chlorure de méthylène-méthanol (90/10)].
B Le ({[(fluoro-2 phényl)-5 phénylsulfonyl-4 (pyrrolidinyl-1 carbonyl)-2 pyrrolidinyl-1]-2 oxo-2 éthyl}-3 uréido)-3 phénylacétate de méthyle-(2RS,4SR,5RS) peut être préparé comme décrit à l'exemple 2 B, mais à partir de 2,3 g de (fluoro-2 phényl)-5 phénylsulfonyl-4 N,N-tétraméthylènepyrrolidinecarboxamide-2-(2RS,4SR,5RS), de 1,52 g d'acide [(méthoxycarbonylméthyl-3 phényl)-3 uréido]-2 acétique et de 1,18 g de N,N'-dicyclohexylcarbodiimide dans 50 cm³ d'acétonitrile. Après traitement, on obtient 2,5 g de ({[(fluoro-2 phényl)-5 phénylsulfonyl-4 (pyrrolidinyl-1 carbonyl)-2 pyrrolidinyl-1]-2 oxo-2 éthyl}-3 uréido)-3 phénylacétate de méthyle-(2RS,4SR,5RS) fondant à 156°C.
C Le (fluoro-2 phényl)-5 phénylsulfonyl-4 N,N-tétraméthylène-pyrrolidinecarboxamide-2-(2RS,4SR,5RS) peut être préparé comme décrit à l'exemple 29 C, mais à partir de 3 g de tert-butoxycarbonyl-1 (fluoro-2 phényl)-5 phénylsulfonyl-4 N,N-tétraméthylène-pyrrolidinecarboxamide-2-(2RS,4SR,5RS) et de 0,85 cm³ d'iodotriméthylsilane dans 50 cm³ de chloroforme. Après traitement, on obtient 2,3 g de (fluoro-2 phényl)-5 phénylsulfonyl-4 N,N-tétraméthylène-pyrrolidinecarboxamide-2-(2RS,4SR,5RS) sous forme d'une meringue utilisée telle quelle dans les synthèses ultérieures.
D Le tert-butoxycarbonyl-1 (fluoro-2 phényl)-5 phénylsulfonyl-4 N,N-tétraméthylène-pyrrolidinecarboxamide-2-(2RS,4SR,5RS) peut être préparé comme décrit à l'exemple 30 D, mais à partir de 2,8 g d'acide tert-butoxycarbonyl-1 (fluoro-2 phényl)-5 phénylsulfonyl-4 pyrrolidinecarboxylique-2-(2RS,4SR,5RS), de 0,85 cm³ de pyrrolidine, de 0,05 g de diméthylamino-4 pyridine et de 1,1 g de N,N-diimidazole-carbonyle dans 50 cm³ d'acétonitrile. Après traitement, on obtient 3 g de tert-butoxycarbonyl-1 (fluoro-2 phényl)-5 phénylsulfonyl-4 N,N-tétraméthylène-pyrrolidinecarboxamide-2-(2RS,4SR,5RS) sous forme d'une meringue utilisée telle quelle dans les syntèses ultérieures.

### Exemple 37

A On opère d'une manière analogue à celle décrite à l'exemple 2 A, mais à partir de 0,4 g de [({[benzyloxycarbonyl-1 éthyl-(forme B)]-3 phényl}-3 uréido)-2 acétyl]-1 (fluoro-2 phényl)-5 phénylsulfonyl-4 pyrrolidinecarboxylate-2 de tert-butyle-(2R*,4S*,5R*) et de 0,05 g de palladium à 10% sur charbon dans 30 cm³ d'éthanol. Après traitement, on obtient 0,23 g d'acide [({[tert-butoxycarbonyl-2 (fluoro-2 phényl)-5 phénylsulfonyl-4 pyrrolidinyl-1-(2R*,4S*,5R*)]-2 oxo-2 éthyl}-3 uréido)-3 phényl-2 propionique-(forme B) [Rf = 0,69; éluant: chlorure de méthylène-méthanol (90/10)].
B Le [({[benzyloxycarbonyl-1 éthyl-(forme B)]-3 phényl}-3 uréido)-2 acétyl]-1 (fluoro-2 phényl)-5 phénylsulfonyl-4 pyrrolidinecarboxylate-2 de tert-butyle-(2R*,4S*,5R*) peut être préparé comme décrit à l'exemple 17 B, mais à partir de 0,75 g d'(amino-2 acétyl)-1 (fluoro-2 phényl)-5 phénylsulfonyl-4 pyrrolidinecarboxylate-2 de tert-butyle-(2R*,4S*,5R*) et de 0,9 g d'(isocyanato-3 phényl)-2 propionate de benzyle (forme B) dans 50 cm³ de tétrahydrofuranne. Après traitement, on obtient 0,4 g de [({[benzyloxycarbonyl-1 éthyl-(forme B)]-3 phényl}-3 uréido)-2 acétyl]-1 (fluoro-2 phényl)-5 phénylsulfonyl-4 pyrrolidinecarboxylate-2 de tert-butyle-(2R*,4S*,5R*) sous forme d'une meringue utilisée telle quelle dans les synthèses ultérieures.
C L'(amino-2 acétyl)-1 (fluoro-2 phényl)-5 phénylsulfonyl-4 pyrrolidinecarboxylate-2 de tert-butyle-(2R*,4S*,5R*) peut être préparé comme décrit à l'exemple 16 C, mais à partir de 4,5 g de (tert-butoxycarbonylamino-2 acétyl)-1 (fluoro-2 phényl)-5 phénylsulfonyl-4 pyrrolidinecarboxylate-2 de tert-butyle-(2R*,4S*,5R*) et de 1,15 cm³ d'iodotriméthylsilane dans 240 cm³ de chloroforme. Après traitement, on obtient 3,75 g d'(amino-2 acétyl)-1 (fluoro-2 phényl)-5 phénylsulfonyl-4 pyrrolidinecarboxylate-2 de tert-butyle-(2R*,4S*,5R*) sous forme d'une huile utilisée telle quelle dans les synthèses ultérieures.
D Le (tert-butoxycarbonylamino-2 acétyl)-1 (fluoro-2 phényl)-5 phénylsulfonyl-4 pyrrolidinecarboxylate-2 de tert-butyle-(2R*,4S*,5R*) peut être préparé de la manière suivante : 11,35 g de (tert-butoxycarbonylamino-2 acétyl)-1 (fluoro-2 phényl)-5 phénylsulfonyl-4 pyrrolidinecarboxylate-2 de tert-butyle-(2RS,4SR,5RS) sont séparés par chromatographie liquide à haute performance en 37 injections sur 400 g de support constitué de silice enrobée de tris-(diméthyl-3,5 phényl)carbamate de cellulose préparé selon J. Amer. Chem. Soc., 106, 5357 (1984) et contenu dans une colonne de 23 cm de longueur et de 6 cm de diamètre avec comme phase mobile un mélange hexane/éthanol 95/5 au débit de 40 cm³/minute en éluant successivement l'énantiomère lévogyre puis le dextrogyre. Les fractions contenant chacun des deux énantiomères sont réunies et concentrées sous pression réduite. On obtient ainsi :
   - 5,25 g de (-)-(tert-butoxycarbonylamino-2 acétyl)-1 (fluoro-2 phényl)-5 phénylsulfonyl-4 pyrrolidinecarboxylate-2 de tert-butyle-(2R*,4S*,5R*) dont le pouvoir rotatoire est [α]²⁰_{D} = -7,5° (c = 1,0 ; méthanol), puis
   - 4,76 g de (+)-(tert-butoxycarbonylamino-2 acétyl)-1 (fluoro-2 phényl)-5 phénylsulfonyl-4 pyrrolidinecarboxylate-2 de tert-butyle-(2R*,4S*,5R*) dont le pouoir rotatoire est [α]²⁰_{D} = +7,2° (c = 0,72 ; méthanol).
E Le (tert-butoxycarbonylamino-2 acétyl)-1 (fluoro-2 phényl)-5 phénylsulfonyl-4 pyrrolidinecarboxylate-2 de tert-butyle-(2RS,4SR,5RS) peut être préparé comme décrit à l'exemple 16 D, mais à partir de 16 g de (fluoro-2 phényl)-5 phénylsulfonyl-4 pyrrolidinecarboxylate-2 de tert-butyle-(2RS,4SR,5RS), de 6,9 g d'acide tert-butoxycarbonylamino-2 acétique et de 8,14 g de N,N'-dicyclohexylcarbodiimide dans 400 cm³ d'acétonitrile. Après traitement, on obtient 18 g de (tert-butoxycarbonylamino-2 acétyl)-1 (fluoro-2 phényl)-5 phénylsulfonyl-4 pyrrolidineearboxylate-2 de tert-butyle-(2RS,4SR,5RS) fondant à 134°C.
F L'(isocyanato-3 phényl)-2 propionate de benzyle (forme B) peut être préparé comme à l'exemple 17 C mais à partir de 2,85 g de (+)-(amino-3 phényl)-2 propionate de benzyle, de 1,48 cm³ de chloroformiate de trichlorométhyle et de 0,24 g de charbon. On obtient ainsi 3,1 g d'(isocyanato-3 phényl)-2 propionate de benzyle (forme B) sous forme d'une huile utilisée telle quelle dans les synthèses ultérieures.
G Le (+)-(amino-3 phényl)-2 propionate de benzyle peut être préparé de la manière suivante : à une solution de 8,0 g de (+)-(nitro-3 phényl)-2 propionate de benzyle dans un mélange de 35 cm³ de méthanol et de 300 cm³ d'eau, on ajoute 75 g de chlorure d'ammonium et 37,0 g de zinc en poudre. Le milieu réactionnel est chauffé à reflux pendant une heure puis refroidi à une température voisine de 0°C et filtré. Le filtrat est extrait par trois fois 200 cm³ d'oxyde de diéthyle. Les phases organiques sont réunies, lavées successivement par 100 cm³ d'eau puis 100 cm³ d'une solution aqueuse saturée de chlorure de sodium, séchées sur sulfate de magnésium et concentrées sous pression réduite. On obtient ainsi 6,7 g de (+)-(amino-3 phényl)-2 propionate de benzyle sous forme d'une huile jaune utilisée telle quelle dans les synthèses ultérieures.
H Le (+)-(nitro-3 phényl)-2 propionate de benzyle peut être préparé de la manière suivante : à un mélange contenant 9,75 g d'acide (+)-(nitro-3 phényl)-2 propionique et 0,5 cm³ de N,N-diméthyl-formamide dans 100 cm³ de dichloro-1,2 éthane, on ajoute 4,72 cm³ de chlorure d'oxalyle. Le milieu réactionnel est agité pendant trois heures à une température voisine de 25°C, puis on ajoute 5,4 g d'alcool benzylique. L'agitation est poursuivie 12 h à cette même température puis le mélange réactionnel est lavé successivement par 2 fois 200 cm³ d'une solution aqueuse saturée d'hydrogénocarbonate de sodium, 100 cm³ d'eau et 100 cm³ d'une solution aqueuse saturée de chlorure de sodium. Les phases organiques sont réunies, lavées successivement par 100 cm³ d'eau puis 100 cm³ d'une solution aqueuse saturée de chlorure de sodium, séchées sur sulfate de magnésium et concentrées sous pression réduite. Le résidu est purifié par chromatographie sur silice [éluant : acétate d'éthyle-cyclohexane (30/70 en volumes)]. Les fractions contenant le produit attendu sont réunies et concentrées sous pression réduite. On obtient ainsi 11,5 g de (+)-(nitro-3 phényl)-2 propioniate de benzyle sous forme d'une huile jaune utilisée telle quelle dans les synthèses ultérieures.
I L'acide (+)-(nitro-3 phényl)-2 propionique peut être préparé de la manière suivante : une solution de 21,5 g de (nitro-3 phényl)-2 N-[hydroxy-2 phényl-1 éthyl-(R)]-propionamide (forme B) dans un mélange de 450 cm³ de dioxanne-1,4 et de 450 cm³ d'une solution aqueuse 4N d'acide chlorhydrique est chauffée à une température voisine de 80°C pendant cinq heures, puis agitée douze heures à une température voisine de 20°C. Le milieu réactionnel est concentré de moitié par évaporation sous pression réduite, dilué par addition de 500 cm³ d'eau distillée et extrait par deux fois 500 cm³ d'oxyde de diéthyle. Les phases organiques sont réunies, lavées successivement par trois fois 250 cm³ d'eau puis 250 cm³ d'une solution aqueuse saturée de chlorure de sodium, séchées sur sulfate de magnésium et concentrées sous pression réduite. On obtient ainsi 14 g d'acide (+)-(nitro-3 phényl)-2 propionique (forme B) sous forme d'un solide crème utilisé tel quel dans les synthèses ultérieures.
J Le (nitro-3 phényl)-2 N-[hydroxy-2 phényl-1 éthyl-(R)]-propionamide (forme B) peut être préparé de la manière suivante : à un mélange contenant 39,0 g d'acide (nitro-3 phényl)-2 propionique-(RS) et 0,5 cm³ de N,N-diméthyl-formamide dans 400 cm³ de dichloro-1,2 éthane, on ajoute lentement 17,2 cm³ de chlorure d'oxalyle. Le milieu réactionnel est agité pendant trois heures à une température voisine de 20°C puis concentré sous pression réduite. Le résidu est dissous dans 150 cm³ de dichloro-1,2 éthane et ajouté à une solution de 27,4 g de phényl-2 glycinol-(R) en maintenant la température du milieu réactionnel en dessous de 10°C. Le mélange réactionnel est agité pendant douze heures à une température voisine de 20°C puis est lavé successivement par 1000 cm³ d'eau distillée, 500 cm³ d'une solution aqueuse normale d'acide chlorhydrique, deux fois 500 cm³ d'eau distillée et 500 cm³ d'une solution aqueuse saturée de chlorure de sodium. La phase organique est séchée sur sulfate de magnésium et concentrée sous pression réduite. Les deux diastéréoisomères obtenus sont séparés par chromatographie sur silice [éluant : chlorure de méthylène-acétate d'éthyle (70/30 en volumes)]. Les fractions contenant chacun des deux diastéréoisomères sont réunies et concentrées sous pression réduite. On obtient ainsi 21,0 g de (nitro-3 phényl)-2 N-[hydroxy-2 phényl-1 éthyl-(R)]-propionamide (forme A) (premier produit d'élution) fondant à 135°C et 19,0 g de (nitro-3 phényl)-2 N-[hydroxy-2 phényl-1 éthyl-(R)]-propionamide (forme B) (deuxième produit d'élution) fondant à 150°C.

L'acide (nitro-3 phényl)-2 propionique-(RS) peut être préparé selon la méthode décrite par E. FELDER et coll. J. Med. Chem., 13, 559 (1970).

### Exemple 38

A On opère d'une manière analogue à celle décrite à l'exemple 2 A, mais à partir de 1,95 g de [({[benzyloxycarbonyl-1 méthoxy-1 méthyl-(S)]-3 phényl}-3 uréido)-2 acétyl]-1 (fluoro-2 phényl)-5 phénylsulfonyl-4 pyrrolidinecarboxylate-2 de tert-butyle-(2R*,4S*,5R*) et de 0,2 g de palladium à 10% sur charbon dans 100 cm³ d'éthanol. Après traitement, on obtient 0,72 g d'acide [({[tert-butoxycarbonyl-2 (fluoro-2 phényl)-5 phénylsulfonyl-4 pyrrolidinyl-1-(2R*,4S*,5R*)]-2 oxo-2 éthyl}-3 uréido)-3 phényl]-2 méthoxy-2 acétique-(S) [Rf = 0,27; éluant : chlorure de méthylène-méthanol (90/10)].
B Le [({[benzyloxycarbonyl-1 méthoxy-1 méthyl-(S)]-3 phényl}-3 uréido)-2 acétyl]-1 (fluoro-2 phényl)-5 phénylsulfonyl-4 pyrrolidinecarboxylate-2 de tert-butyle-(2R*,4S*,5R*) peut être préparé comme décrit à l'exemple 17 B, mais à partir de 2,95 g d'(amino-2 acétyl)-1 (fluoro-2 phényl)-5 phénylsulfonyl-4 pyrrolidinecarboxylate-2 de tert-butyle-(2R*,4S*,5R*) et de 2,1 g d'(isocyanato-3 phényl)-2 méthoxy-2 acétate de benzyle-(S) dans 100 cm³ de tétrahydrofuranne. Après traitement, on obtient 2,05 g de [({[tert-butoxycarbonyl-2 (fluoro-2 phényl)-5 phénylsulfonyl-4 pyrrolidinyl-1-(2R*,4S*,5R*)]-2 oxo-2 éthyl}-3 uréido)-3 phényl]-2 méthoxy-2 acétate de benzyle-(S) sous forme de meringue utilisée telle quelle dans les synthèses ultérieures.
C L'(isocyanato-3 phényl)-2 méthoxy-2 acétate de benzyle-(S) peut être préparé comme à l'exemple 17 C mais à partir de 2,6 g d'(amino-3 phényl)-2 méthoxy-2 acétate de benzyle-(S), de 1,16 cm³ de chloroformiate de trichlorométhyle et de 0,4 g de charbon dans 75 cm³ de toluène. On obtient ainsi 3 g d'(isocyanato-3 phényl)-2 méthoxy-2 acétate de benzyle-(S) sous forme d'une huile utilisée telle quelle dans les synthèses ultérieures.
D L'(amino-3 phényl)-2 méthoxy-2 acétate de benzyle-(S) peut être préparé comme à l'exemple 37 G, mais à partir de 8 g de méthoxy-2 (nitro-3 phényl)-2 acétate de benzyle-(S), de 72,7 g de chlorure d'ammonium et de 34,6 g de zinc en poudre dans un mélange de 35 cm³ de méthanol et de 350 cm³ d'eau distillée. Après traitement, on obtient 2,6 g d'(amino-3 phényl)-2 méthoxy-2 acétate de benzyle-(S) sous forme d'une huile jaune utilisée telle quelle dans les synthèses ultérieures.
E Le méthoxy-2 (nitro-3 phényl)-2 acétate de benzyle-(S) peut être préparé comme décrit à l'exemple 37 H, mais à partir de 7,1 g d'acide méthoxy-2 (nitro-3 phényl)-2 acétique-(S), de 4,25 cm³ de chlorure d'oxalyle, de 0,5 cm³ de N,N-diméthyl-formamide et de 6,0 cm³ d'alcool benzylique dans 120 cm³ de dichloro-1,2 éthane. Après traitement, on obtient 11,5 g de méthoxy-2 (nitro-3 phényl)-2 acétate de benzyle-(S) sous forme d'une huile jaune utilisée telle quelle dans les synthèses ultérieures.
F L'acide méthoxy-2 (nitro-3 phényl)-2 acétique-(S) peut être préparé comme décrit à l'exemple 37 l, mais à partir de 16,5 g méthoxy-2 (nitro-3 phényl)-2 N-[hydroxy-2 phényl-1 éthyl-(R)]-acétamide-(S) et de 200 cm³ d'une solution aqueuse 4N d'acide chlorhydrique dans 260 cm³ de dioxanne-1,4. Après traitement, on obtient 9,5 g d'acide méthoxy-2 (nitro-3 phényl)-2 acétique-(S) fondant à 112°C.
G Le méthoxy-2 (nitro-3 phényl)-2 N-[hydroxy-2 phényl-1 éthyl-(R)]-acétamide-(S) peut être préparé comme décrit à l'exemple 37 J, mais à partir de 42,2 g d'acide méthoxy-2 (nitro-3 phényl)-2 acétique-(RS), de 0,5 cm³ de N,N-diméthyl-formamide et de 17,2 cm³ de chlorure d'oxalyle dans 200 cm³ de dichloro-1,2 éthane, puis de 27,4 g de phényl-2 glycinol-(R) dans 200 cm³ de dichloro-1,2 éthane. Après traitement, on obtient 20 g de méthoxy-2 (nitro-3 phényl)-2 N-[hydroxy-2 phényl-1 éthyl-(R)]-acétamide-(S) sous forme d'un solide utilisé tel quel dans les synthèses ultérieures.

L'acide méthoxy-2 (nitro-3 phényl)-2 acétique-(RS) peut être préparé selon la méthode décrite par A. TAKEDA, Contribs. Boyce Thompson Institute, 20, 197-203 (1959).

### Exemple 39

A On opère d'une manière analogue à celle décrite à l'exemple 2 B, mais à partir de 3,05 g de phényl-5 pyrrolidinedicarboxylate-2,4 de (2) tert-butyle et de (4) méthyle-(2RS,4RS,5SR), de 2,32 g d'acide (indolyl-2 carbonylamino)-2 propionique-(RS) et de 2,1 g de N,N'-dicyclohexylcarbodiimide dans 50 cm³ d'acétonitrile. Après traitement, on obtient 2,35 g d' [(indolyl-2 carbonylamino)-2 propionyl-(forme A)]-1 phényl-5 pyrrolidinedicarboxylate-2,4 de (2) tert-butyle et de (4) méthyle-(2RS,4RS,5SR) fondant à 182°C et 1,38 g d' [(indolyl-2 carbonylamino)-2 propionyl-(forme B)]-1 phényl-5 pyrrolidinedicarboxylate-2,4 de (2) tert-butyle et de (4) méthyle-(2RS,4RS,5SR) fondant à 207°C.
B L'acide (indolyl-2 carbonylamino)-2 propionique-(RS) peut être préparé de la manière suivante : à une solution de 4,4 g d'alanine-(RS) et 14,34 g de carbonate de potassium dans 90 cm³ d'eau distillée, on ajoute, à une température comprise entre 5 et 10°C, une solution de 9,75 g de chlorure de l'acide indole-2 carboxylique dans 70 cm³ de dioxanne-1,4. Le mélange est agité pendant vingt heures à une température voisine de 20°C, dilué par addition de 50 cm³ d'eau distillée puis acidifié à pH voisin de 1 par addition d'une solution aqueuse normale d'acide chlorhydrique. Le solide qui précipite est séparé par filtration, rincé par deux fois 25 cm³ d'eau distillée, essoré et séché sous pression réduite à une température voisine de 40°C. On obtient ainsi 8,3 g d'acide (indolyl-2 carbonylamino)-2 propionique-(RS) fondant à 240°C.

### Exemple 40

A On opère d'une manière analogue à celle décrite à l'exemple 3, mais à partir de 1,15 g d'[(indolyl-2 carbonylamino)-2 propionyl-(forme A)]-1 phényl-5 pyrrolidinedicarboxylate-2,4 de (2) tert-butyle et de (4) méthyle-(2RS,4RS,5SR) (forme A) et de 0,12 g d'hydroxyde de potassium dans 40 cm³ d'eau distillée et 60 cm³ de méthanol. Après traitement, on obtient 0,46 g d' [(indolyl-2 carbonylamino)-2 propionyl-(forme A)]-1 phényl-5 pyrrolidinedicarboxylate-2,4 acide de (2) tert-butyle-(2RS,4SR,5SR) (forme A) [Rf = 0,22; éluant : chlorure de méthylène-méthanol (90/10)].

### Exemple 41

A On opère d'une manière analogue à celle décrite à l'exemple 3, mais à partir de 1,5 g d' {[(éthoxycarbonyl-3 phényl)-3 uréido]-2 acétyl}-1 (fluoro-2 phényl)-5 morpholinocarbonyl-4 pyrrolidinecarboxylate-2 de tert-butyle-(2RS,4SR,5SR) et de 2,4 cm³ d'une solution aqueuse normale d'hydroxyde de potassium dans un mélange de 12,5 cm³ d'eau distillée et de 50 cm³ de méthanol. Après traitement, on obtient 0,28 g d'acide {[(tert-butoxycarbonyl-2 (fluoro-2 phényl)-5 morpholinocarbonyl-4 pyrrolidinyl-1)-2 oxo-2 éthyl]-3 uréido}-3 benzoïque-(2RS,4SR,5SR) [Rf = 0,19; éluant : chlorure de méthylène-méthanol (90/10)].
B L' {[(éthoxycarbonyl-3 phényl)-3 uréido]-2 acétyl}-1 (fluoro-2 phényl)-5 morpholinocarbonyl-4 pyrrolidinecarboxylate-2 de tert-butyle-(2RS,4SR,5SR) peut être préparé comme décrit à l'exemple 17 B, mais à partir de 1,7 g d'(amino-2 acétyl)-1 (fluoro-2 phényl)-5 morpholinocarbonyl-4 pyrrolidinecarboxylate-2 de tert-butyle-(2RS,4SR,5SR) et de 0,75 g d'isocyanato-3 benzoate d'éthyle dans 80 cm³ de tétrahydrofuranne. Après traitement, on obtient 1,5 g d' ([(éthoxycarbonyl-3 phényl)-3 uréido]-2 acétyl}-1 (fluoro-2 phényl)-5 morpholinocarbonyl-4 pyrrolidinecarboxylate-2 de tert-butyle-(2RS,4SR,5SR) sous forme d'une meringue utilisée telle quelle dans les synthèses ultérieures.
C L'(amino-2 acétyl)-1 (fluoro-2 phényl)-5 morpholinocarbonyl-4 pyrrolidinecarboxylate-2 de tert-butyle-(2RS,4SR,5SR) peut être préparé comme décrit à l'exemple 16 C, mais à partir de 2,6 g de (tert-butoxycarbonylamino-2 acétyl)-1 (fluoro-2 phényl)-5 morpholinocarbonyl-4 pyrrolidinecarboxylate-2 de tert-butyle-(2RS,4SR,5SR) et de 0,93 cm³ d'iodotriméthylsilane dans 120 cm³ de chloroforme. Après traitement, on obtient 1,7 g d'(amino-2 acétyl)-1 (fluoro-2 phényl)-5 morpholinocarbonyl-4 pyrrolidinecarboxylate-2 de tert-butyle-(2RS,4SR,5SR) sous forme d'une meringue utilisée telle quelle dans les synthèses ultérieures.
D Le (tert-butoxycarbonylamino-2 acétyl)-1 (fluoro-2 phényl)-5 morpholinocarbonyl-4 pyrrolidinecarboxylate-2 de tert-butyle-(2RS,4SR,5SR) peut être préparé comme décrit à l'exemple 20 D, mais à partir de 3,73 g de (tert-butoxycarbonylamino-2 acétyl)-1 (fluoro-2 phényl)-5 pyrrolidinedicarboxylate-2,4 acide de (2) tert-butyle-(2RS,4SR,5SR), de 0,70 g de morpholine et de 1,65 g de N,N'-dicyclohexylcarbodiimide dans 120 cm³ d'acétonitrile. Après traitement, on obtient 2,65 g de (tert-butoxycarbonylamino-2 acétyl)-1 (fluoro-2 phényl)-5 morpholinocarbonyl-4 pyrrolidinecarboxylate-2 de tert-butyle-(2RS,4SR,5SR) sous forme d'une meringue utilisée telle quelle dans les synthèses ultérieures.
E Le (tert-butoxycarbonylamino-2 acétyl)-1 (fluoro-2 phényl)-5 pyrrolidinedicarboxylate-2,4 acide de (2) tert-butyle-(2RS,4SR,5SR) peut être préparé d'une manière analogue à celle décrite à l'exemple 20 E, mais à partir de 24 g de (tert-butoxycarbonylamino-2 acétyl)-1 (fluoro-2 phényl)-5 pyrrolidinedicarboxylate-2,4 de (2) tert-butyle et de (4) méthyle-(2RS,4RS,5SR) et de 50 cm³ d'une solution aqueuse normale d'hydroxyde de sodium dans un mélange de 750 cm³ de méthanol et de 200 cm³ d'eau. Après traitement, on obtient 21,4 g de (tert-butoxycarbonylamino-2 acétyl)-1 (fluoro-2 phényl)-5 pyrrolidinedicarboxylate-2,4 acide de (2) tert-butyle-(2RS,4SR,5SR) sous forme d'un solide utilisé tel quel dans les synthèses ultérieures.
F Le (tert-butoxycarbonylamino-2 acétyl)-1 (fluoro-2 phényl)-5 pyrrolidinedicarboxylate-2,4 de (2) tert-butyle et de (4) méthyle-(2RS,4RS,5SR) peut être préparé d'une manière analogue à celle décrite à l'exemple 16 D, mais à partir de 29 g de (fluoro-2 phényl)-5 pyrrolidinedicarboxylate-2,4 de (2) tert-butyle et de (4) méthyle-(2RS,4RS,5SR), de 15,7 g d'acide tert-butoxycarbonylamino-2 acétique et de 18,5 g de N,N'-dicyclohexylcarbodiimide dans 200 cm³ d'acétonitrile. Après traitement, on obtient 34 g de (tert-butoxycarbonylamino-2 acétyl)-1 (fluoro-2 phényl)-5 pyrrolidinedicarboxylate-2,4 de (2) tert-butyle et de (4) méthyle-(2RS,4RS,5SR) fondant à 142°C.

### Exemple 42

A On opère d'une manière analogue à celle décrite à l'exemple 3, mais à partir de 1,75 g d' {[(éthoxycarbonyl-3 phényl)-3 uréido]-2 acétyl}-1 diéthylaminocarbonyl-4 (fluoro-2 phényl)-5 pyrrolidinecarboxylate-2 de tert-butyle-(2RS,4SR,5SR) et de 2,8 cm³ d'une solution aqueuse normale d'hydroxyde de potassium dans un mélange de 15 cm³ d'eau distillée et de 65 cm³ de méthanol. Après traitement, on obtient 0,50 g d'acide {[(tert-butoxycarbonyl-2 diéthylaminocarbonyl-4 (fluoro-2 phényl)-5 pyrrolidinyl-1)-2 oxo-2 éthyl]-3 uréido}-3 benzoïque-(2RS,4SR,5SR) fondant à 150°C.
B L' {[(éthoxycarbonyl-3 phényl)-3 uréido]-2 acétyl}-1 diéthylaminocarbonyl-4 (fluoro-2 phényl)-5 pyrrolidinecarboxylate-2 de tert-butyle-(2RS,4SR,5SR) peut être préparé comme décrit à l'exemple 17 B, mais à partir de 2,7 g d'(amino-2 acétyl)-1 diéthylaminocarbonyl-4 (fluoro-2 phényl)-5 pyrrolidinecarboxylate-2 de tert-butyle-(2RS,4SR,5SR) et de 1,21 g d'isocyanato-3 benzoate d'éthyle dans 80 cm³ de tétrahydrofuranne. Après traitement, on obtient 1,75 g d' {[(éthoxycarbonyl-3 phényl)-3 uréido]-2 acétyl}-1 diéthylaminocarbonyl-4 (fluoro-2 phényl)-5 pyrrolidinecarboxylate-2 de tert-butyle-(2RS,4SR,5SR) sous forme d'une meringue utilisée telle quelle dans les synthèses ultérieures.
C L'(amino-2 acétyl)-1 diéthylaminocarbonyl-4 (fluoro-2 phényl)-5 pyrrolidinecarboxylate-2 de tert-butyle-(2RS,4SR,5SR) peut être préparé comme décrit à l'exemple 16 C, mais à partir de 4,5 g de (tert-butoxycarbonylamino-2 acétyl)-1 diéthylaminocarbonyl-4 (fluoro-2 phényl)-5 pyrrolidinecarboxylate-2 de tert-butyle-(2RS,4SR,5SR) et de 1,2 cm³ d'iodotriméthylsilane dans 180 cm³ de chloroforme. Après traitement, on obtient 2,7 g d'(amino-2 acétyl)-1 diéthylaminocarbonyl-4 (fluoro-2 phényl)-5 pyrrolidinecarboxylate-2 de tert-butyle-(2RS,4SR,5SR) sous forme d'une meringue utilisée telle quelle dans les synthèses ultérieures.
D Le (tert-butoxycarbonylamino-2 acétyl)-1 diéthylaminocarbonyl-4 (fluoro-2 phényl)-5 pyrrolidinecarboxylate-2 de tert-butyle-(2RS,4SR,5SR) peut être préparé comme décrit à l'exemple 20 D, mais à partir de 4,66 g de (tert-butoxycarbonylamino-2 acétyl)-1 (fluoro-2 phényl)-5 pyrrolidinedicarboxylate.2,4 acide de (2) tert-butyle-(2RS,4SR,5SR), de 0,73 g de diéthylamine, de 10 mg de diméthylamino-4 pyridine et de 1,78 g de N,N'-dicyclohexylcarbodiimide dans 120 cm³ d'acétonitrile. Après traitement, on obtient 4,5 g de (tert-butoxycarbonylamino-2 acétyl)-1 diéthylaminocarbonyl-4 (fluoro-2 phényl)-5 pyrrolidinecarboxylate-2 de tert-butyle-(2RS,4SR,5SR) sous forme d'une meringue utilisée telle quelle dans les synthèses ultérieures.

### Exemple 43

A On opère d'une manière analogue à celle décrite à l'exemple 3, mais à partir de 3,1 g de {[(méthoxycarbonylméthyl-3 phényl)-3 uréido]-2 acétyl}-1 phényl-5 méthyl-2 pyrrolidinedicarboxylate-2,4 de (2) tert-butyle et de (4) méthyle-(2RS,4RS,5SR) et de 0,62 g d'hydroxyde de potassium dans un mélange de 100 cm³ d'eau distillée et de 150 cm³ de méthanol. Après traitement, on obtient 0,85 g d'acide {[(tert-butoxycarbonyl-2 méthoxycarbonyl-4 méthyl-2 phényl-5 pyrrolidinyl-1)-2 oxo-2 éthyl]-3 uréido}-3 phénylacétique(2RS,4RS,5SR) [Rf = 0,29; éluant : chlorure de méthylène-méthanol (90/10)].
B Le {[(méthoxycarbonylméthyl-3 phényl)-3 uréido]-2 acétyl}-1 phényl-5 méthyl-2 pyrrolidinedicarboxylate-2,4 de (2) tert-butyle et de (4) méthyle-(2RS,4RS,5SR) peut être préparé comme décrit à l'exemple 2 B, mais à partir de 5,2 g de méthyl-2 phényl-5 pyrrolidinedicarboxylate-2,4 de (2) tert-butyle et de (4) méthyle-(2RS,4RS,5SR), de 4,34 g d'acide [(méthoxycarbonylméthyl-3 phényl)-3 uréido]-2 acétique et de 3,36 g de N,N'-dicyclohexylcarbodiimide dans 90 cm³ d'acétonitrile. Après traitement, on obtient 3,1 g de {[(méthoxycarbonylméthyl-3 phényl)-3 uréido]-2 acétyl}-1 phényl-5 méthyl-2 pyrrolidinedicarboxylate-2,4 de (2) tert-butyle et de (4) méthyle-(2RS,4RS,5SR) sous forme d'un solide amorphe utilisé tel quel dans les synthèses ultérieures.
C Le méthyl-2 phényl-5 pyrrolidinedicarboxylate-2,4 de (2) tert-butyle et de (4) méthyle-(2RS,4RS,5SR) peut être préparé comme décrit à l'exemple 2 C, mais à partir de 4,5 g de benzylidèneamino-2 propionate de tert-butyle, de 1,74 cm³ d'acrylate de méthyle, de 4,84 g d'acétate d'argent et de 3,26 cm³ de triéthylamine dans 200 cm³ d'acétonitrile. Après traitement, on obtient 5,2 g de méthyl-2 phényl-5 pyrrolidinedicarboxylate-2,4 de (2) tert-butyle et de (4) méthyle-(2RS,4RS,5SR) sous forme d'une huile utilisée telle quelle dans les synthèses ultérieures.
D Le benzylidèneamino-2 propionate de tert-butyle peut être préparé comme décrit à l'exemple 2 D, mais à partir de 2,03 cm³ de benzaldéhyde, de 3,63 g de chlorhydrate d'alaninate de tert-butyle, de 3 g de tamis 4Å et de 2,8 cm³ de triéthylamine dans 50 cm³ de dichlorométhane. Après traitement, on obtient 4,5 g de benzylidèneamino-2 propionate de tert-butyle sous forme d'une huile utilisée telle quelle dans les synthèses ultérieures.

### Exemple 44

A On opère d'une manière analogue à celle décrite à l'exemple 2 A, mais à partir de 2,83 g de ({[(benzyloxycarbonylméthyl)-3 phényl]-3 uréido}-2 acétyl)-1 (fluoro-2 phényl)-5 (tétrahydro-1,2,3,4 quinolyl-1)carbonyl-2 phénylsulfonyl-4 pyrrolidine-(2RS,4SR,5RS) et de 0,36 g de palladium à 10% sur charbon dans 150 cm³ d'éthanol. Après traitement, on obtient 1,67 g d'acide ({[(fluoro-2 phényl)-5 (tétrahydro-1,2,3,4 quinolyl-1)carbonyl-2 phénylsulfonyl-4 pyrrolidinyl-1]-2 oxo-2 éthyl}-3 uréido)-3 phénylacétique-(2RS,4RS,5SR) [Rf = 0,49; éluant : chlorure de méthylène-méthanol (90/10)].
B Le ({[(benzyloxycarbonylméthyl)-3 phényl]-3 uréido}-2 acétyl)-1 (fluoro-2 phényl)-5 phénylsulfonyl-4 (tétrahydro-1,2,3,4 quinolyl-1)carbonyl-2 pyrrolidine-(2RS,4SR,5RS) peut être préparé comme décrit à l'exemple 2 B, mais à partir de 2,3 g de (fluoro-2 phényl)-5 phénylsulfonyl-4 (tétrahydro-1,2,3,4 quinolyl-1)carbonyl-2 pyrrolidine-(2RS,4SR,5RS), de 1,69 g d'acide {[(benzyloxycarbonylméthyl)-3 phényl]-3 uréido}-2 acétique et de 1,02 g de N,N'-dicyclohexylcarbodiimide dans 50 cm³ d'acétonitrile. Après traitement, on obtient 2,83 g de ({[(benzyloxycarbonylméthyl)-3 phényl]-3 uréido}-2 acétyl)-1 (fluoro-2 phényl)-5 phénylsulfonyl-4 (tétrahydro-1,2,3,4 quinolyl-1) carbonyl-2 pyrrolidine-(2RS,4SR,5RS) sous forme d'un solide amorphe utilisé tel quel dans les synthèses ultérieures.
C La (fluoro-2 phényl)-5 phénylsulfonyl-4 (tétrahydro-1,2,3,4 quinolyl)carbonyl-2 pyrrolidine-(2RS,4SR,5RS) peut être préparée comme décrit à l'exemple 2 C, mais à partir de 3,68 g de [(fluoro-2 benzylidène)amino-2 acétyl]-1 tétrahydro-1,2,3,4 quinoléine, de 2,1 g de phénylvinylsulfone, de 3,12 g d'acétate d'argent et de 1,75 cm³ de triéthylamine dans 100 cm³ d'acétonitrile. Après traitement, on obtient 2,3 g de (fluoro-2 phényl)-5 phénylsulfonyl-4 (tétrahydro-1,2,3,4 quinolyl-1)carbonyl-2 pyrrolidine-(2RS,4SR,5RS) sous forme d'une meringue utilisée telle quelle dans les synthèses ultérieures.
D La [(fluoro-2 benzylidène)amino-2 acétyl]-1 tétrahydro-1,2,3,4 quinoléine peut être préparée comme décrit à l'exemple 2 D, mais à partir de 1,53 cm³ de fluoro-2 benzaldéhyde, de 3,95 g de bromhydrate d'(amino-2 acétyl)-1 tétrahydro-1,2,3,4 quinoléine, de 2,2 g de tamis 4Å et de 2,05 cm³ de triéthylamine dans 50 cm³ de dichlorométhane. Après traitement, on obtient 3,68 g de [(fluoro-2 benzylidène)amino-2 acétyl]-1 tétrahydro-1,2,3,4 quinoléine sous forme d'une huile utilisée telle quelle dans les synthèses ultérieures.
E Le bromhydrate d'(amino-2 acétyl)-1 tétrahydro-1,2,3,4 quinoléine peut être préparé de la manière suivante : une solution de 5,08 g de (bromo-2 acétyl)-1 tétrahydro-1,2,3,4 quinoléine dans 150 cm³ d'une solution méthanolique 7N d'ammoniac est agitée pendant huit heures à une température voisine de 20°C. Le milieu réactionnel est ensuite concentré sous pression réduite et le résidu cristallisé dans 150 cm³ d'acétonitrile. Le solide est séparé par filtration et séché sous pression réduite. On obtient ainsi 3,95 g de bromhydrate d'(amino-2 acétyl)-1 tétrahydro-1,2,3,4 quinoléine fondant à 241°C.
F La (bromo-2 acétyl)-1 tétrahydro-1,2,3,4 quinoléine peut être préparée de la manière suivante : à une solution de 39,96 g de tétrahydro-1,2,3,4-quinoléine dans un mélange de 46,8 cm³ de triéthylamine et de 195 cm³ de dichlorométhane, on ajoute goutte à goutte, à une température voisine de - 5°C, une solution de 27,5 cm³ de bromure de bromacétyle dans 30 cm³ de dichlorométhane. Le mélange réactionnel est agité pendant deux heures à une température voisine de 20°C puis versé dans 300 cm³ d'eau distillée. La phase organique est séparée par décantation, lavée par trois fois 100 cm³ d'une solution aqueuse saturée en chlorure de sodium, séchée sur sulfate de magnésium et concentrée sous pression réduite. Le résidu est repris par 300 cm³ d'oxyde de diisopropyle et filtré. Le filtrat est concentré sous pression réduite. On obtient ainsi 67,48 g de (bromo-2 acétyl)-1 tétrahydro-1,2,3,4 quinoléine sous forme d'une huile utilisée telle quelle dans les synthèses ultérieures.

### Exemple 45

A On opère d'une manière analogue à celle décrite à l'exemple 3, mais à partir de 3,4 g de (fluoro-2 phényl)-5 {[(méthoxycarbonylméthyl-3 phényl)-3 uréido]-2 acétyl}-1 (tétrahydro-1,2,3,4-quinolyl-1)carbonyl-2 pyrrolidinecarboxylate-4 de méthyle-(2RS,4RS,5SR) et de 0,61 g d'hydroxyde de potassium dans un mélange de 30 cm³ d'eau distillée et de 50 cm³ de méthanol. Après traitement, on obtient 2,5 g d'acide {[(carboxyméthyl-3 phényl)-3 uréido]-2 acétyl}-1 (fluoro-2 phényl)-5 (tétrahydro-1,2,3,4-quinolyl-1) carbonyl-2 pyrrolidinecarboxylique-4-(2RS,4SR,5SR) [Rf = 0,36; éluant : chlorure de méthylène-méthanol (90/10)].
B Le (fluoro-2 phényl)-5 {[(méthoxycarbonylméthyl-3 phényl)-3 uréido]-2 acétyl}-1 (tétrahydro-1,2,3,4-quinolyl-1)carbonyl-2 pyrrolidine- carboxylate-4 de méthyle-(2RS,4RS,5SR) peut être préparé comme décrit à l'exemple 2 B, mais à partir de 3,23 g de (fluoro-2 phényl)-5 (tétrahydro-1,2,3,4-quinolyl-1)carbonyl-2 pyrrolidinecarboxylate-4 de méthyle-(2RS,4RS,5SR), de 2,25 g d'acide [(méthoxycarbonylméthyl-3 phényl)-3 uréido]-2 acétique et de 1,75 g de N,N'-dicyclohexylcarbodiimide dans 90 cm³ d'acétonitrile. Après traitement, on obtient 3,6 g de (fluoro-2 phényl)-5 (tétrahydro-1,2,3,4-quinolyl-1)carbonyl-2 [((méthoxycarbonylméthyl-3 phényl)-3 uréido]-2 acétyl}-1 pyrrolidinecarboxylate-4 de méthyle-(2RS,4RS,5SR) sous forme d'un solide amorphe utilisé tel quel dans les synthèses ultérieures.
C Le (fluoro-2 phényl)-5 (tétrahydro-1,2,3,4-quinolyl-1)carbonyl-2 pyrrolidinecarboxylate-4 de méthyle-(2RS,4RS,5SR) peut être préparé comme décrit à l'exemple 2 C, mais à partir de 4,86 g de [(fluoro-2 benzylidène)amino-2 acétyl]-1 tétrahydro-1,2,3,4 quinoléine, de 1,48 cm³ d'acrylate de méthyle, de 4,11 g d'acétate d'argent et de 2,77 cm³ de triéthylamine dans 175 cm³ d'acétonitrile. Après traitement, on obtient 5,55 g de (fluoro-2 phényl)-5 (tétrahydro-1,2,3,4-quinolyl-1)carbonyl-2 pyrrolidinecarboxylate-4 de méthyle-(2RS,4RS,5SR) sous forme d'une huile utilisée telle quelle dans les synthèses ultérieures.

### Exemple 46

A On opère d'une manière analogue à celle décrite à l'exemple 16 B, mais à partir de 2,3 g d'(amino-2 acétyl)-1 (fluoro-2 phényl)-5 phénylsulfonyl-4 pyrrolidinecarboxylate-2 de tert-butyle-(2RS,4SR,5RS), de 0,9 g de N,N'-diimidazole-carbonyle et de 0,81 g d' (amino-3 phényl)-5 tétrazole dans 70 cm³ de dichloro-1,2 éthane. Après traitement, on obtient 0,6 g de [({[tert-butoxycarbonyl-2 (fluoro-2 phényl)-5 phénylsulfonyl-4 pyrrolidinyl-1]-2 oxo-2 éthyl}-3 uréido)-3 phényl]-5 tétrazole-(2RS,4SR,5RS) sous forme de sel de sodium [Rf = 0,17; éluant : chlorure de méthylène-méthanol (90/10)].
B L'(amino-2 acétyl)-1 (fluoro-2 phényl)-5 phénylsulfonyl-4 pyrrolidinecarboxylate-2 de tert-butyle-(2RS,4SR,5RS) peut être préparé comme décrit à l'exemple 16 C, mais à partir de 2,82 g de (tert-butoxycarbonylamino-2 acétyl)-1 (fluoro-2 phényl)-5 phénylsulfonyl-4 pyrrolidinecarboxylate-2 de tert-butyle-(2RS,4SR,5RS) et de 0,72 cm³ d'iodotriméthylsilane dans 50 cm³ de chloroforme. Après traitement, on obtient 2,3 g d'(amino-2 acétyl)-1 (fluoro-2 phényl)-5 phénylsulfonyl-4 pyrrolidinecarboxylate-2 de tert-butyle-(2RS,4SR,5RS) sous forme d'une meringue utilisée telle quelle dans les synthèses ultérieures.

L'(amino-3 phényl)-5 tétrazole peut être préparé selon la méthode décrite dans la demande de brevet EP 0508796 (MERCK & Co Inc.).

### Exemple 47

A On opère d'une manière analogue à celle décrite à l'exemple 3, mais à partir de 3,5 g de (fluoro-2 phényl)-5 {[(méthoxycarbonylméthyl-3 phényl)-3 uréido]-2 acétyl}-1 morpholinocarbonyl-2 phénylsulfonyl-4 pyrrolidine-(2RS,4SR,5RS) et de 0,3 g d'hydroxyde de potassium dans un mélange de 30 cm³ d'eau distillée et de 50 cm³ de méthanol. Après traitement, on obtient 2,45 g d'acide ({[(fluoro-2 phényl)-5 morpholinocarbonyl-2 phénylsulfonyl-4 pyrrolidinyl-1]-2 oxo-2 éthyl}-3 uréido)-3 phénylacétique-(2RS,4SR,5RS) [Rf = 0,64; éluant : chlorure de méthylène-méthanol (80/20)].
B Le (fluoro-2 phényl)-5 {[(méthoxycarbonylméthyl-3 phényl)-3 uréido]-2 acétyl}-1 morpholinocarbonyl-2 phénylsulfonyl-4 pyrrolidine-(2RS,4SR,5RS) peut être préparé comme décrit à l'exemple 2 B, mais à partir de 2,1 g de (fluoro-2 phényl)-5 morpholinocarbonyl-2 phénylsulfonyl-4 pyrrolidine-(2RS,4SR,5RS), de 1,33 g d'acide [(méthoxycarbonylméthyl-3 phényl)-3 uréido]-2 acétique et de 1,03 g de N,N'-dicyclohexylcarbodiimide dans 75 cm³ d'acétonitrile. Après traitement, on obtient 3,56 g de (fluoro-2 phényl)-5 {[(méthoxycarbonylméthyl-3 phényl)-3 uréido]-2 acétyl}-1 morpholinocarbonyl-2 phénylsulfonyl-4 pyrrolidine-(2RS,4SR,5RS) sous forme d'une meringue utilisée telle quelle dans les synthèses ultérieures.
C La (fluoro-2 phényl)-5 morpholinocarbonyl-2 phénylsulfonyl-4 pyrrolidine-(2RS,4SR,5RS) peut être préparée comme décrit à l'exemple 2 C, mais à partir de 6 g de [(fluoro-2 benzylidène)amino-2 acétyl]-4 morpholine, de 4,05 g de phénylvinylsulfone, de 6 g d'acétate d'argent et de 4,05 cm³ de triéthylamine dans 200 cm³ d'acétonitrile. Après traitement, on obtient 2,9 g de (fluoro-2 phényl)-5 morpholinocarbonyl-2 phénylsulfonyl-4 pyrrolidine-(2RS,4SR,5RS) sous forme d'une huile utilisée telle quelle dans les synthèses ultérieures.
D La [(fluoro-2 benzylidène)amino-2 acétyl]-4 morpholine peut être préparée comme décrit à l'exemple 2 D, mais à partir de 5,3 cm³ de fluoro-2 benzaldéhyde, de 9,1 g de chlorhydrate d' (amino-2 acétyl)-4 morpholine, de 6 g de tamis 4Å et de 7 cm³ de triéthylamine dans 150 cm³ de dichlorométhane. Après traitement, on obtient 12 g de [(fluoro-2 benzylidène)amino-2 acétyl]-4 morpholine sous forme d'une huile utilisée telle quelle dans les synthèses ultérieures.
E Le chlorhydrate d' (amino-2 acétyl)-4 morpholine peut être préparé comme décrit à l'exemple 44 E, mais à partir de 16 g de chloroacétyl-4 morpholine dans 140 cm³ d'une solution méthanolique 7N d'ammoniac. Après traitement, on obtient 9,1 g de chlorhydrate d'(amino-2 acétyl)-4 morpholine fondant à 158°C.
F La chloracétyl-4 morpholine peut être préparée de la manière suivante : à une solution de 8,71 cm³ de morpholine dans 50 cm³ de dichloro-1,2 éthane, on ajoute 25 cm³ d'une solution aqueuse à 20% d'hydroxyde de sodium. Le mélange est refroidi à une température voisine de -20°C puis on ajoute goutte à goutte 9,9 cm³ de chlorure de chloracétyle. Le milieu réactionnel est agité pendant deux heures à une température voisine de -20°C puis pendant vingt heures à une température voisine de 20°C. On ajoute ensuite 100 cm³ d'eau distillée et on extrait par trois fois 100 cm³ de dichlorométhane. Les phases organiques sont réunies, lavées par trois fois 100 cm³ d'une solution aqueuse à 3% d'acide chlorhydrique, deux fois 100 cm³ d'une solution aqueuse à 10% d'hydrogénocarbonate de sodium et deux fois 100 cm³ d'une solution aqueuse saturée en chlorure de sodium puis séchées sur sulfate de magnésium et concentrées sous pression réduite. On obtient ainsi 16 g de chloracétyl-4 morpholine sous forme d'une huile utilisée telle quelle dans les synthèses ultérieures.

### Exemple 48

A On opère d'une manière analogue à celle décrite à l'exemple 3, mais à partir de 3,75 g de (fluoro-2 phényl)-5 isobutylcarbamoyl-2 {[(méthoxycarbonylméthyl-3 phényl)-3 uréido]-2 acétyl}-1 phénylsulfonyl-4 pyrrolidine-(2RS,4SR,5RS) et de 0,32 g d'hydroxyde de potassium dans un mélange de 55 cm³ d'eau distillée et de 140 cm³ de méthanol. Après traitement, on obtient 1,84 g d'acide ({[(fluoro-2 phényl)-5 isobutylcarbamoyl-2 phénylsulfonyl-4 pyrrolidinyl-1]-2 oxo-2 éthyl}-3 uréido)-3 phénylacétique-(2RS,4SR,5RS) [Rf = 0,73; éluant : chlorure de méthylène-méthanol (80/20)].
B La (fluoro-2 phényl)-5 isobutylcarbamoyl-2 {[(méthoxycarbonylméthyl-3 phényl)-3 uréido]-2 acétyl}-1 phénylsulfonyl-4 pyrrolidine-(2RS,4SR,5RS) peut être préparée comme décrit à l'exemple 2 B, mais à partir de 2,3 g de (fluoro-2 phényl)-5 isobutylcarbamoyl-2 phénylsulfonyl-4 pyrrolidine-(2RS,4SR,5RS), de 1,5 g d'acide [(méthoxycarbonylméthyl-3 phényl)-3 uréido]-2 acétique et de 1,17 g de N,N'-dicyclohexylcarbodiimide dans 100 cm³ d'acétonitrile. Après traitement, on obtient 3,77 g de (fluoro-2 phényl)-5 isobutylcarbamoyl-2 {[(méthoxycarbonylméthyl-3 phényl)-3 uréido]-2 acétyl}-1 phénylsulfonyl-4 pyrrolidine-(2RS,4SR,5RS) sous forme d'une meringue utilisée telle quelle dans les synthèses ultérieures.
C La (fluoro-2 phényl)-5 isobutylcarbamoyl-2 phénylsulfonyl-4 pyrrolidine-(2RS,4SR,5RS) peut être préparée comme décrit à l'exemple 2 C, mais à partir de 5,5 g de (fluoro-2 benzylidène)amino-2 N-isobutyl-acétamide, de 3,7 g de phénylvinylsulfone, de 5,45 g d'acétate d'argent et de 3,7 cm³ de triéthylamine dans 200 cm³ d'acétonitrile. Après traitement, on obtient 2,7 g de (fluoro-2 phényl)-5 isobutylcarbamoyl-2 phénylsulfonyl-4 pyrrolidine-(2RS,4SR,5RS) sous forme d'une huile utilisée telle quelle dans les synthèses ultérieures.
D La (fluoro-2 benzylidène)amino-2 N-isobutyl-acétamide peut être préparée comme décrit à l'exemple 2 D, mais à partir de 4,75 cm³ de fluoro-2 benzaldéhyde, de 7,5 g de chlorhydrate d'amino-2 N-isobutyl-acétamide, de 6 g de tamis 4Å et de 6,3 cm³ de triéthylamine dans 150 cm³ de dichlorométhane. Après traitement, on obtient 11 g de (fluoro-2 benzylidène)amino-2 N-isobutyl-acétamide sous forme d'une huile utilisée telle quelle dans les synthèses ultérieures.
E Le chlorhydrate d'amino-2 N-isobutyl-acétamide peut être préparé comme décrit à l'exemple 44 E, mais à partir de 15 g de chloro-2 N-isobutyl-acétamide dans 140 cm³ d'une solution méthanolique 7N d'amoniac. Après traitement, on obtient 7,5 g de chlorhydrate d'amino-2 N-isobutyl-acétamide fondant à 154°C.
F La chloro-2 N-isobutyl-acétamide peut être préparée comme décrit à l'exemple 47 F, mais à partir de 10,2 cm³ d'isobutylamine, de 9,9 cm³ de chlorure de chloracétyle et de 25 cm³ d'une solution aqueuse à 20% d'hydroxyde de sodium dans 50 cm³ de dichloro-1,2 éthane. Après traitement, on obtient 15 g de chloro-2 N-isobutyl-acétamide sous forme d'une huile utilisée telle quelle dans les synthèses ultérieures.

### Exemple 49

A On opère d'une manière analogue à celle décrite à l'exemple 3, mais à partir de 3,7 g de (fluoro-2 phényl)-5 isobutylcarbamoyl-2 {[(méthoxycarbonylméthyl-3 phényl)-3 uréido]-2 acétyl}-1 pyrrolidinecarboxylate-4 de méthyle-(2RS,4RS,5SR) et de 0,56 g d'hydroxyde de potassium dans un mélange de 20 cm³ d'eau distillée et de 60 cm³ de méthanol. Après traitement, on obtient 1,9 g d'acide {[(carboxyméthyl-3 phényl)-3 uréido]-2 acétyl}-1 (fluoro-2 phényl)-5 isobutylcarbamoyl-2 pyrrolidinecarboxylique-4-(2RS,4SR,5SR) [Rf = 0,15; éluant : chlorure de méthylène-méthanol (90/10)].
B Le (fluoro-2 phényl)-5 isobutylcarbamoyl-2 {[(méthoxycarbonylméthyl-3 phényl)-3 uréido]-2 acétyl}-1 pyrrolidinecarboxylate-4 de méthyle-(2RS,4RS,5SR) peut être préparé comme décrit à l'exemple 2 B, mais à partir de 2,65 g de (fluoro-2 phényl)-5 isobutylcarbamoyl-2 pyrrolidinecarboxylate-4 de méthyle-(2RS,4RS,5SR), de 1,34 g d'acide [(méthoxycarbonylméthyl-3 phényl)-3 uréido]-2 acétique et de 1,05 g de N,N'-dicyclohexylcarbodiimide dans 50 cm³ d'acétonitrile. Après traitement, on obtient 3,7 g d'un mélange de (fluoro-2 phényl)-5 isobutylcarbamoyl-2 {[(méthoxycarbonylméthyl-3 phényl)-3 uréido]-2 acétyl}-1 pyrrolidinecarboxylate-4 de méthyle-(2RS,4RS,5SR) et de dicyclohexyl-1,3 urée sous forme d'une meringue utilisée telle quelle dans les synthèses ultérieures.
C Le (fluoro-2 phényl)-5 isobutylcarbamoyl-2 pyrrolidinecarboxylate-4 de méthyle-(2RS,4RS,5SR) peut être préparé comme décrit à l'exemple 2 C, mais à partir de 5,5 g de (fluoro-2 benzylidène)amino-2 N-isobutyl-acétamide, de 2 cm³ d'acrylate de méthyle, de 5,46 g d'acétate d'argent et de 3,7 cm³ de triéthylamine dans 200 cm³ d'acétonitrile. Après traitement, on obtient 1,6 g de (fluoro-2 phényl)-5 isobutylcarbamoyl-2 pyrrolidinecarboxylate-4 de méthyle-(2RS,4RS,5SR) sous forme d'une huile utilisée telle quelle dans les synthèses ultérieures.

### Exemple 50

A On opère d'une manière analogue à celle décrite à l'exemple 3, mais à partir de 2,5 g de (fluoro-2 phényl)-5 {[(méthoxycarbonylméthyl-3 phényl)-3 uréido]-2 acétyl}-1 morpholinocarbonyl-2 pyrrolidinecarboxylate-4 de méthyle-(2RS,4RS,5SR) et de 0,48 g d'hydroxyde de potassium dans un mélange de 30 cm³ d'eau distillée et de 60 cm³ de méthanol. Après traitement, on obtient 1,1 g d'acide {[(carboxyméthyl-3 phényl)-3 uréido]-2 acétyl}-1 (fluoro-2 phényl)-5 morpholinocarbonyl-2 pyrrolidinecarboxylique-4-(2RS,4SR,5SR) [Rf = 0,15; éluant : chlorure de méthylène-méthanol (90/10)].
B Le (fluoro-2 phényl)-5 {[(méthoxycarbonylméthyl-3 phényl)-3 uréido]-2 acétyl}-1 morpholinocarbonyl-2 pyrrolidinecarboxylate-4 de méthyle-(2RS,4RS,5SR) peut être préparé comme décrit à l'exemple 2 B, mais à partir de 1,68 g de (fluoro-2 phényl)-5 morpholinocarbonyl-2 pyrrolidinecarboxylate-4 de méthyle-(2RS,4RS,5SR), de 1,34 g d'acide [(méthoxycarbonylméthyl-3 phényl)-3 uréido]-2 acétique et de 1,05 g de N,N'-dicyclohexylcarbodiimide dans 50 cm³ d'acétonitrile. Après traitement, on obtient 2,5 g de (fluoro-2 phényl)-5 {[(méthoxycarbonylméthyl-3 phényl)-3 uréido]-2 acétyl}-1 morpholinocarbonyl-2 pyrrolidinecarboxylate-4 de méthyle-(2RS,4RS,5SR) sous forme d'une meringue utilisée telle quelle dans les synthèses ultérieures.
C Le (fluoro-2 phényl)-5 morpholinocarbonyl-2 pyrrolidinecarboxylate-4 de méthyle-(2RS,4RS,5SR) peut être préparé comme décrit à l'exemple 2 C, mais à partir de 6 g de [(fluoro-2 benzylidène)amino-2 acétyl]-4 morpholine, de 2,16 cm³ d'acrylate de méthyle, de 6 g d'acétate d'argent et de 4,05 cm³ de triéthylamine dans 200 cm³ d'acétonitrile. Après traitement, on obtient 1,6 g de (fluoro-2 phényl)-5 morpholinocarbonyl-2 pyrrolidinecarboxylate-4 de méthyle-(2RS,4RS,5SR) sous forme d'une huile utilisée telle quelle dans les synthèses ultérieures.

### Exemple 51

En opérant comme décrit à l'exemple 37 D, mais à partir de 1 g d'acide ({[tert-butoxycarbonyl-2 (fluoro-2 phényl)-5 phénylsulfonyl-4 pyrrolidinyl-1]-2 oxo-2 éthyl}-3 uréido)-3 benzoïque-(2RS,4SR,5RS) en deux injections sur 400 g de support constitué de silice enrobée de tris-(diméthyl-3,5 phényl)carbamate de cellulose avec comme phase mobile un mélange hexane/éthanol (85/15) au débit de 40 cm³/minute on élue successivement l'énantiomère lévogyre puis le dextrogyre. Les fractions contenant chacun des deux énantiomères sont réunies et concentrées sous pression réduite. On obtient ainsi
- 0,466 g d'acide-(-) ({[tert-butoxycarbonyl-2 (fluoro-2 phényl)-5 phénylsulfonyl-4 pyrrolidinyl-1]-2 oxo-2 éthyl}-3 uréido)-3 benzoïque-(2R*,4S*,5R*) dont le pouvoir rotatoire est [α]²⁰_{D} = -8,7° (c = 0,5 ; méthanol), puis
- 0,490 g d'acide-(+) ({[tert-butoxycarbonyl-2 (fluoro-2 phényl)-5 phénylsulfonyl-4 pyrrolidinyl-1]-2 oxo-2 éthyl}-3 uréido)-3 benzoïque-(2R*,4S*,5R*) dont le pouvoir rotatoire est [α]²⁰_{D} = +10° (c = 0,5 ; méthanol).

### Exemple 52

En opérant comme décrit à l'exemple 37 D, mais à partir de 0,3 g de {[(carboxyméthyl-3 phényl)-3 uréido]-2 acétyl}-1 (fluoro-2 phényl)-5 pyrrolidinedicarboxylate-2,4 acide de (2) tert-butyle-(2RS,4SR,5SR) en une injection sur 400 g de support constitué de silice enrobée de tris-(diméthyl-3,5 phényl)carbamate de cellulose avec comme phase mobile un mélange hexane/éthanol (70/30) au débit de 40 cm³/minute on élue successivement l'énantiomère dextrogyre puis le lévogyre. Les fractions contenant chacun des deux énantiomères sont réunies et concentrées sous pression réduite. On obtient ainsi:
- 0,135 g de (+)-{[(carboxyméthyl-3 phényl)-3 uréido]-2 acétyl}-1 (fluoro-2 phényl)-5 pyrrolidinedicarboxylate-2,4 acide de (2) tert-butyle-(2R*,4S*,5S*) dont le pouvoir rotatoire est [α]²⁰_{D} = +15,7° (c = 0,5 ; méthanol), puis
- 0,121 g de (-)-{[(carboxyméthyl-3 phényl)-3 uréido]-2 acétyl}-1 (fluoro-2 phényl)-5 pyrrolidinedicarboxylate-2,4 acide de (2) tert-butyle-(2R*,4S*,5S*) dont le pouvoir rotatoire est [α]²⁰_{D} = -20,3° (c = 0,5 ; méthanol).

### Exemple 53

A On opère d'une manière analogue à celle décrite à l'exemple 3, mais à partir de 1,5 g de cyclohexyl-5 {[(méthyl-3 phényl)-3 uréido]-2 acétyl}-1 pyrrolidinedicarboxylate-2,4 de (2) tert-butyle et de (4) méthyle-(2RS,4RS,5SR) et de 0,17 g d'hydroxyde de potassium dans un mélange de 20 cm³ d'eau distillée et de 60 cm³ de méthanol. Après traitement, on obtient 0,5 g de cyclohexyl-5 {[(méthyl-3 phényl)-3 uréido]-2 acétyl}-1 pyrrolidinedicarboxylate-2,4 acide de (2) tert-butyle-(2RS,4RS,5SR) [Rf = 0,31; éluant : chlorure de méthylène-méthanol (90/10)].
B Le cyclohexyl-5 {[(méthyl-3 phényl)-3 uréido]-2 acétyl}-1 pyrrolidinedicarboxylate-2,4 de (2) tert-butyle et de (4) méthyle-(2RS,4RS,5SR) peut être préparé comme décrit à l'exemple 2 B, mais à partir de 1,25 g de cyclohexyl-5 pyrrolidinedicarboxylate-2,4 de (2) tert-butyle et de (4) méthyle-(2RS,4RS,5SR), de 0,83 g d'acide [(méthyl-3 phényl)-3 uréido]-2 acétique et de 0,83 g de N,N'-dicyclohexylcarbodiimide dans 35 cm³ d'acétonitrile. Après traitement, on obtient 1,5 g de cyclohexyl-5 {[(méthyl-3 phényl)-3 uréido]-2 acétyl}-1 pyrrolidinedicarboxylate-2,4 de (2) tert-butyle et de (4) méthyle-(2RS,4RS,5SR) sous forme d'une meringue utilisée telle quelle dans les synthèses ultérieures.
C Le cyclohexyl-5 pyrrolidinedicarboxylate-2,4 de (2) tert-butyle et de (4) méthyle-(2RS,4RS,5SR) peut être préparé comme décrit à l'exemple 2 C, mais à partir de 4,5 g de cyclohexylméthylèneaminoacétate de tert-butyle, de 1,8 cm³ d'acrylate de méthyle, de 5 g d'acétate d'argent et de 2,8 cm³ de triéthylamine dans 200 cm³ d'acétonitrile. Après traitement, on obtient 2,8 g de cyclohexyl-5 pyrrolidinedicarboxylate-2,4 de (2) tert-butyle et de (4) méthyle-(2RS,4RS,5SR) sous forme d'une huile utilisée telle quelle dans les synthèses ultérieures.
D Le cyclohexylméthylèneaminoacétate de tert-butyle peut être préparé comme décrit à l'exemple 2 D, mais à partir de 2,43 cm³ de cyclohexanecarbaldéhyde, de 3,4 g de chlorhydrate de glycinate de tert-butyle, de 3g de tamis 4Å et de 2,8 cm³ de triéthylamine dans 50 cm³ de dichlorométhane. Après traitement, on obtient 4,5 g de cyclohexylméthylèneaminoacétate de tert-butyle sous forme d'une huile utilisée telle quelle dans les synthèses ultérieures.

### Exemple 54

A On opère d'une manière analogue à celle décrite à l'exemple 3, mais à partir de 0,95 g de cyclohexyl-5 {[(méthoxycarbonyl-3 phényl)-3 uréido]-2 acétyl}-1 pyrrolidinedicarboxylate-2,4 de (2) tert-butyle et de (4) méthyle-(2RS,4RS,5SR) et de 0,20 g d'hydroxyde de potassium dans un mélange de 15 cm³ d'eau distillée et de 50 cm³ de méthanol. Après traitement, on obtient 0,25 g de cyclohexyl-5 {[(carboxy-3 phényl)-3 uréido]-2 acétyl}-1 pyrrolidinedicarboxylate-2,4 acide de (2) tert-butyle-(2RS,4RS,5SR) fondant à 150°C.
B Le cyclohexyl-5 {[(méthoxycarbonyl-3 phényl)-3 uréido]-2 acétyl}-1 pyrrolidinedicarboxylate-2,4 de (2) tert-butyle et de (4) méthyle-(2RS,4RS,5SR) peut être préparé comme décrit à l'exemple 2 B, mais à partir de 1,4 g de cyclohexyl-5 pyrrolidinedicarboxylate-2,4 de (2) tert-butyle et de (4) méthyle-(2RS,4RS,5SR), de 1,15 g d'acide [(méthoxycarbonyl-3 phényl)-3 uréido]-2 acétique et de 0,93 g de N,N'-dicyclohexylcarbodiimide dans 50 cm³ d'acétonitrile. Après traitement, on obtient 1,0 g de cyclohexyl-5 {[(méthoxycarbonyl-3 phényl)-3 uréido]-2 acétyl}-1 pyrrolidinedicarboxylate-2,4 de (2) tert-butyle et de (4) méthyle-(2RS,4RS,5SR) sous forme d'une meringue utilisée telle quelle dans les synthèses ultérieures.

### Exemple 55

A On opère d'une manière analogue à celle décrite à l'exemple 3, mais à partir de 2,8 g d' {[(éthoxycarbonyl-3 phényl)-3 uréido]-2 acétyl}-1 (fluoro-2 phényl)-5 phénylcarbamoyl-4 pyrrolidinecarboxylate-2 de tert-butyle-(2RS,4SR,5SR) dans un mélange de 4,4 cm³ d'une solution aqueuse normale d'hydroxyde de potassium, de 20 cm³ d'eau distillée et de 100 cm³ de méthanol. Après traitement, on obtient 0,11 g d'acide ({[tert-butoxycarbonyl-2 (fluoro-2 phényl)-5 phénylcarbamoyl-4 pyrrolidinyl-1]-2 oxo-2 éthyl}-3 uréido)-3 benzoïque-(2RS,4SR,5SR) fondant à 170°C.
B L' {[(éthoxycarbonyl-3 phényl)-3 uréido]-2 acétyl}-1 (fluoro-2 phényl)-5 phénylcarbamoyl-4 pyrrolidinecarboxylate-2 de tert-butyle-(2RS,4SR,5SR) peut être préparé comme décrit à l'exemple 17 B, mais à partir de 2,5 g d'(amino-2 acétyl)-1 (fluoro-2 phényl)-5 phénylcarbamoyl-4 pyrrolidinecarboxylate-2 de tert-butyle-(2RS,4SR,5SR) et de 1,27 g d'isocyanato-3 benzoate d'éthyle dans 140 cm³ de tétrahydrofuranne. Après traitement, on obtient 2,9 g d' {[(éthoxycarbonyl-3 phényl)-3 uréido]-2 acétyl}-1 (fluoro-2 phényl)-5 phénylcarbamoyl-4 pyrrolidinecarboxylate-2 de tert-butyle-(2RS,4SR,5SR) sous forme d'une meringue utilisée telle quelle dans les synthèses ultérieures.
C L'(amino-2 acétyl)-1 (fluoro-2 phényl)-5 phénylcarbamoyl-4 pyrrolidinecarboxylate-2 de tert-butyle-(2RS,4SR,5SR) peut être préparé comme décrit à l'exemple 16 C, mais à partir de 3,1 g de (tert-butoxycarbonylamino-2 acétyl)-1 (fluoro-2 phényl)-5 phénylcarbamoyl-4 pyrrolidinecarboxylate-2 de tert-butyle-(2RS,4SR,5SR) et de 1,15 cm³ d'iodotriméthylsilane dans 100 cm³ de chloroforme. Après traitement, on obtient 2,5 g d'(amino-2 acétyl)-1 (fluoro-2 phényl)-5 phénylcarbamoyl-4 pyrrolidinecarboxylate-2 de tert-butyle-(2RS,4SR,5SR) sous forme d'une meringue utilisée telle quelle dans les synthèses ultérieures.
D Le (tert-butoxycarbonylamino-2 acétyl)-1 (fluoro-2 phényl)-5 phénylcarbamoyl-4 pyrrolidinecarboxylate-2 de tert-butyle-(2RS,4SR,5SR) peut être préparé comme décrit à l'exemple 20 D, mais à partir de 4,4 g de (tert-butoxycarbonylamino-2 acétyl)-1 (fluoro-2 phényl)-5 pyrrolidinedicarboxylate-2,4 acide de (2) tert-butyle-(2RS,4SR,5SR), de 0,86 cm³ d'aniline et de 1,95 g de N,N'-dicyclohexylcarbodiimide dans 150 cm³ d'acétonitrile. Après traitement, on obtient 4,3 g de (tert-butoxycarbonylamino-2 acétyl)-1 (fluoro-2 phényl)-5 phénylcarbamoyl-4 pyrrolidinecarboxylate-2 de tert-butyle-(2RS,4SR,5SR) sous forme d'une meringue utilisée telle quelle dans les synthèses ultérieures.

### Exemple 56

A On opère d'une manière analogue à celle décrite à l'exemple 3, mais à partir de 4,2 g d' {[(éthoxycarbonyl-3 phényl)-3 uréido]-2 acétyl}-1 (fluoro-2 phényl)-5 isobutylcarbamoyl-4 pyrrolidinecarboxylate-2 de tert-butyle-(2RS,4SR,5SR) dans un mélange de 6,8 cm³ d'une solution aqueuse normale d'hydroxyde de potassium, de 30 cm³ d'eau distillée et de 140 cm³ de méthanol. Après traitement, on obtient 0,65 g d'acide ({[tert-butoxycarbonyl-2 (fluoro-2 phényl)-5 isobutylcarbamoyl-4 pyrrolidinyl-1]-2 oxo-2 éthyl}-3 uréido)-3 benzoïque-(2RS,4SR,5SR) fondant à 160°C.
B L'{[(éthoxycarbonyl-3 phényl)-3 uréido]-2 acétyl}-1 (fluoro-2 phényl)-5 isobutylcarbamoyl-4 pyrrolidinecarboxylate-2 de tert-butyle-(2RS,4SR,5SR) peut être préparé comme décrit à l'exemple 17 B, mais à partir de 5,2 g d'(amino-2 acétyl)-1 (fluoro-2 phényl)-5 isobutylcarbamoyl-4 pyrrolidinecarboxylate-2 de tert-butyle-(2RS,4SR,SSR) et de 2,2 g d'isocyanato-3 benzoate d'éthyle dans 140 cm³ de tétrahydrofuranne. Après traitement, on obtient 2,9 g d' {[(éthoxycarbonyl-3 phényl)-3 uréido]-2 acétyl}-1 (fluoro-2 phényl)-5 isobutylcarbamoyl-4 pyrrolidinecarboxylate-2 de tert-butyle-(2RS,4SR,5SR) sous forme d'une meringue utilisée telle quelle dans les synthèses ultérieures.
C L'(amino-2 acétyl)-1 (fluoro-2 phényl)-5 isobutylcarbamoyl-4 pyrrolidinecarboxylate-2 de tert-butyle-(2RS,4SR,5SR) peut être préparé comme décrit à l'exemple 16 C, mais à partir de 7,4 g de (tert-butoxycarbonylamino-2 acétyl)-1 (fluoro-2 phényl)-5 isobutylcarbamoyl-4 pyrrolidinecarboxylate-2 de tert-butyle-(2RS,4SR,5SR) et de 2,8 cm³ d'iodotriméthylsilane dans 350 cm³ de chloroforme. Après traitement, on obtient 5,2 g d'(amino-2 acétyl)-1 (fluoro-2 phényl)-5 isobutylcarbamoyl-4 pyrrolidinecarboxylate-2 de tert-butyle-(2RS,4SR,5SR) sous forme d'une meringue utilisée telle quelle dans les synthèses ultérieures.
D Le (tert-butoxycarbonylamino-2 acétyl)-1 (fluoro-2 phényl)-5 isobutylcarbamoyl-4 pyrrolidinecarboxylate-2 de tert-butyle-(2RS,4SR,5SR) peut être préparé comme décrit à l'exemple 26 D, mais à partir de 5,6 g de (tert-butoxycarbonylamino-2 acétyl)-1 (fluoro-2 phényl)-5 pyrrolidinedicarboxylate.2,4 acide de (2) tert-butyle-(2RS,4SR,5SR), de 2,15 g de N,N-diimidazole carbonyle, de 50 mg de diméthylamino-4 pyridine et de 0,88 cm³ d'isobutylamine dans 180 cm³ de dichloro-1,2 éthane. Après traitement, on obtient 6 g de (tert-butoxycarbonylamino-2 acétyl)-1 (fluoro-2 phényl)-5 isobutylcarbamoyl-4 pyrrolidinecarboxylate-2 de tert-butyle-(2RS,4SR,5SR) sous forme d'une meringue utilisée telle quelle dans les synthèses ultérieures.

### Exemple 57

A Une solution de 0,7 g d'acétoxyméthyl-4 [(méthyl-3 phényl)-3 uréido-2 acétyl]-1 phényl-5 pyrrolidinecarboxylate-2 de tert-butyle-(2RS,4SR,5SR) et de 77 mg d'hydroxyde de potassium dans un mélange de 3 cm³ d'eau distillée et de 9 cm³ de méthanol est agitée pendant vingt heures à une température voisine de 20°C. Le milieu réactionnel est ensuite concentré sous pression réduite et le résidu repris par 50 cm³ d'acétate d'éthyle. La phase organique est lavée par trois fois 20 cm³ d'eau distillée, séchée sur sulfate de magnésium et concentrée sous pression réduite. Le résidu est cristallisé dans 50 cm³ de pentane et recristallisé dans un mélange de 40 cm³ de cyclohexane et de 20 cm³ d'acétate d'éthyle. On obtient ainsi 0,39 g d'hydroxyméthyl-4 [(méthyl-3 phényl)-3 uréido-2 acétyl]-1 phényl-5 pyrrolidinecarboxylate-2 de tert-butyle-(2RS,4SR,5SR) fondant à 168°C.
B L'acétoxyméthyl-4 [(méthyl-3 phényl)-3 uréido-2 acétyl]-1 phényl-5 pyrrolidinecarboxylate-2 de tert-butyle-(2RS,4SR,5SR) peut être préparé comme décrit à l'exemple 17 B, mais à partir de 0,81 g d'acétoxyméthyl-4 (amino-2 acétyl)-1 phényl-5 pyrrolidinecarboxylate-2 de tert-butyle-(2RS,4SR,5SR) et de 0,25 cm³ d'isocyanate de méta-tolyle dans 10 cm³ de tétrahydrofuranne. Après traitement, on obtient 0,8 g d'acétoxyméthyl-4 [(méthyl-3 phényl)-3 uréido-2 acétyl]-1 phényl-5 pyrrolidinecarboxylate-2 de tert-butyle-(2RS,4SR,5SR) sous forme d'une huile utilisée telle quelle dans les synthèses ultérieures.
C L'acétoxyméthyl-4 (amino-2 acétyl)-1 phényl-5 pyrrolidinecarboxylate-2 de tert-butyle-(2RS,4SR,5SR) peut être préparé comme décrit à l'exemple 16 C, mais à partir d'acétoxyméthyl-4 (tert-butoxycarbonylamino-2 acétyl)-1 phényl-5 pyrrolidinecarboxylate-2 de tert-butyle-(2RS,4SR,5SR) et de 0,29 cm³ d'iodotriméthylsilane dans 10 cm³ de chloroforme. Après traitement, on obtient 0,82 g d'acétoxyméthyl-4 (amino-2 acétyl)-1 phényl-5 pyrrolidinecarboxylate-2 de tert-butyle-(2RS,4SR,5SR) sous forme d'une huile utilisée telle quelle dans les synthèses ultérieures.
D L'acétoxyméthyl-4 (tert-butoxycarbonylamino-2 acétyl)-1 phényl-5 pyrrolidinecarboxylate-2 de tert-butyle-(2RS,4SR,5SR) peut être préparé de la manière suivante : à une solution de 1,5 g de (tert-butoxycarbonylamino-2 acétyl)-1 hydroxyméthyl-4 phényl-5 pyrrolidinecarboxylate-2 de tert-butyle-(2RS,4SR,5SR) dans un mélange de 0,64 cm³ de triéthylamine et de 50 cm³ de dichlorométhane, on ajoute, à une température voisine de 5°C, 0,27 cm³ de chlorure d'acétyle. Le milieu réactionnel est agité pendant vingt heures à une température voisine de 20°C puis est lavé par trois fois 30 cm³ d'eau distillée. Les phases aqueuses sont extraites par 20 cm³ de dichlorométhane puis les phases organiques sont réunies, séchées sur sulfate de magnésium et concentrées sous pression réduite. Le résidu est purifié par chromatographie sur silice [éluant : cyclohexane-acétate d'éthyle (70-30 en volumes)]. Les fractions contenant le produit attendu sont réunies et concentrées sous pression réduite. On obtient ainsi 1 g d'acétoxyméthyl-4 (tert-butoxycarbonylamino-2 acétyl)-1 phényl-5 pyrrolidinecarboxylate-2 de tert-butyle-(2RS,4SR,5SR) sous forme d'une huile utilisée telle quelle dans les synthèses ultérieures.
E Le (tert-butoxycarbonylamino-2 acétyl)-1 hydroxyméthyl-4 phényl-5 pyrrolidinecarboxylate-2 de tert-butyle-(2RS,4SR,5SR) peut être préparé de la manière suivante : à une solution de 4 g de (tert-butoxycarbonylamino-2 acétyl)-1 phényl-5 pyrrolidinedicarboxylate-2,4 de (2) tert-butyle et de (4) méthyle-(2RS,4RS,SSR) dans 50 cm³ de tertiobutanol on ajoute, à une température voisine de 20°C, 1,2 g de borohydrure de sodium. Le mélange réactionnel est chauffé au reflux et on ajoute 9,7 cm³ de méthanol. Le milieu est encore agité au reflux pendant quarante minutes puis refroidi à une température voisine de 20°C, hydrolysé par 100 cm³ d'eau distillée et concentré sous pression réduite. Le résidu aqueux est saturé par un excès de chlorure de sodium et extrait par trois fois 30 cm³ d'oxyde de diéthyle. Les phases organiques sont réunies, séchées sur sulfate de magnésium et concentrées sous pression réduite. Le résidu est purifié par chromatographie sur silice [éluant : cyclohexane-acétate d'éthyle (30-70 en volumes)]. Les fractions contenant le produit attendu sont réunies et concentrées sous pression réduite. On obtient ainsi 1,6 g de (tert-butoxycarbonylamino-2 acétyl)-1 hydroxyméthyl-4 phényl-5 pyrrolidinecarboxylate-2 de tert-butyle-(2RS,4SR,5SR) sous forme d'une huile utilisée telle quelle dans les synthèses ultérieures.

### Exemple 58

A On opère d'une manière analogue à celle décrite à l'exemple 3, mais à partir de 0,6 g de (-)-(fluoro-2 phényl)-5 {[(méthoxycarbonylméthyl-3 phényl)-3 uréido]-2 acétyl}-1 pyrrolidinedicarboxylate-2,4 de (2) tert-butyle et de (4) méthyle-(2R*,4R*,5S*) et de 0,12 g d'hydroxyde de potassium dans un mélange de 10 cm³ d'eau distillée et de 25 cm³ de méthanol. Après traitement, on obtient 0,37 g de (+)-{[(carboxyméthyl-3 phényl)-3 uréido]-2 acétyl}-1 (fluoro-2 phényl)-5 pyrrolidinedicarboxylate-2,4 acide de (2) tert-butyle-(2R*,4S*,5S*) dont les analyses spectroscopiques sont conformes à celles du premier produit de l'exemple 52.
B Le (-)-(fluoro-2 phényl)-5 {[(méthoxycarbonylméthyl-3 phényl)-3 uréido]-2 acétyl}-1 pyrrolidinedicarboxylate-2,4 de (2) tert-butyle et de (4) méthyle-(2R*,4R*,5S*) peut être préparé comme décrit à l'exemple 2 B, mais à partir de 1 g de (fluoro-2 phényl)-5 pyrrolidinedicarboxylate-2,4 de (2) tert-butyle et de (4) méthyle-(2R*,4R*,5S*)-(énantiomère A), de 0,82 g d'acide [(méthoxycarbonylméthyl-3 phényl)-3 uréido]-2 acétique et de 0,64 g de N,N'-dicyclohexylcarbodiimide dans 50 cm³ d'acétonitrile. Après traitement, on obtient 0,6 g de (-)-(fluoro-2 phényl)-5 {[(méthoxycarbonylméthyl-3 phényl)-3 uréido]-2 acétyl}-1 pyrrolidinedicarboxylate-2,4 de (2) tert-butyle et de (4) méthyle-(2R*,4R*,5S*) sous forme d'une meringue utilisée telle quelle dans les synthèses ultérieures.
C Le (fluoro-2 phényl)-5 pyrrolidinedicarboxylate-2,4 de (2) tert-butyle et de (4) méthyle-(2R*,4R*,5S*)-(énantiomère A) peut être préparé de la manière suivante : une solution de 2,18 g de (fluoro-2 phényl)-5 [phényl-3 (phényl-3 thio-uréido)-2 propionyl-(S)]-1 pyrrolidinedicarboxylate-2,4 de (2) tert-butyle et de (4) méthyle-(2R*,4R*,5S*)-(forme A) dans un mélange de 25 cm³ de dichlorométhane et de 1,76 cm³ d'acide trifluoroacétique est agitée à une température voisine de 20°C pendant sept heures. Le mélange réactionnel est ensuite versé dans 150 cm³ d'une solution aqueuse saturée en hydrogénocarbonate de sodium. La phase organique est séparée par décantation, lavée par deux fois 50 cm³ d'eau distillée, séchée sur sulfate de magnésium et concentrée sous pression réduite. Le résidu est purifié par chromatographie sur silice [éluant cyclohexane-acétate d'éthyle (85-15 puis 70-30 en volumes)]. Les fractions contenant le produit attendu sont réunies et concentrées sous pression réduite. On obtient ainsi 1 g de (fluoro-2 phényl)-5 pyrrolidinedicarboxylate-2,4 de (2) tert-butyle et de (4) méthyle-(2R*,4R*,5S*)-(énantiomère A) sous forme d'une huile utilisée telle quelle dans les synthèses ultérieures.
D Le (fluoro-2 phényl)-5 [phényl-3 (phényl-3 thio-uréido)-2 propionyl-(S)]-1 pyrrolidinedicarboxylate-2,4 de (2) tert-butyle et de (4) méthyle-(2R*,4R*,5S*)-(forme A) peut être préparé comme décrit à l'exemple 17 B, mais à partir de 1,8 g d'[amino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme A) et de 0,5 cm³ d'isothiocyanate de phényle dans 100 cm³ de dichlorométhane. Après traitement, on obtient 2,18 g de (fluoro-2 phényl)-5 [phényl-3 (phényl-3 thio-uréido)-2 propionyl-(S)]-1 pyrrolidinedicarboxylate-2,4 de (2) tert-butyle et de (4) méthyle-(2R*,4R*,5S*)-(forme A) sous forme d'une meringue utilisée telle quelle dans les synthèses ultérieures.
E L'[amino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme A) peut être préparé de la manière suivante : à une solution de 2,3 g de [benzyloxycarbonylamino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme A) dans 150 cm³ d'éthanol on ajoute, à une température voisine de 20°C, 0,38 g de palladium à 10% sur charbon. Le mélange réactionnel est agité pendant six heures sous atmosphère d'hydrogène (130 kPa). Le catalyseur est séparé par filtration sur célite, rincé par deux fois 10 cm³ de méthanol et le filtrat est concentré à sec sous pression réduite. On obtient ainsi 1,8 g d'[amino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme A) sous forme d'une huile utilisée telle quelle dans les synthèses ultérieures.
F Le [benzyloxycarbonylamino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme A) peut être préparé comme décrit à l'exemple 16 D, mais à partir de 3,23 g de (fluoro-2 phényl)-5 pyrrolidinedicarboxylate-2,4 de (2) tert-butyle et de (4) méthyle-(2RS,4RS,5SR), de 2,99 g de N-benzyloxycarbonyl phénylalanine-(S) et de 2,06 g de N,N'-dicyclohexylcarbodiimide dans 60 cm³ d'acétonitrile. Après traitement, on obtient, par ordre d'élution :
   - 2,32 g de [benzyloxycarbonylamino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme A) puis
   - 1,24 g de [benzyloxycarbonylamino-2 phényl-3 propionyl-(S)]-1 (fluoro-2 phényl)-5 pyrrolidinedicarboxylate-2,4 de (2) tert-butyle et de (4) méthyle-(2R*,4R*,5S*)-(forme B) sous forme de meringues blanches utilisées telles quelles dans les synthèses ultérieures.

### Exemple 59

A On opère d'une manière analogue à celle décrite à l'exemple 3, mais à partir de 4,4 g de benzylcarbamoyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5SR) et de 0,38 g d'hydroxyde de potassium dans 40 cm³ d'eau distillée et 150 cm³ de méthanol. Après traitement, on obtient 0,14 g de ({[benzylcarbamoyl-4 (tert-butoxycarbonyl-2 (fluoro-2 phényl)-5 pyrrolidinyl-1]-2 oxo-2 éthyl}-3 uréido)-3 benzoïque-(2RS,4SR,5SR) fondant à 217°C.
B Le benzylcarbamoyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5SR) peut être préparé comme décrit à l'exemple 17 B, mais à partir de 3,5 g d'(amino-2 acétyl)-1 benzylcarbamoyl-4 (fluoro-2 phényl)-5 pyrrolidinecarboxylate-2 de tert-butyle-(2RS,4SR,5SR) et de 1,43 g d'isocyanato-3 benzoate d'éthyle dans 140 cm³ de tétrahydrofuranne. Après traitement, on obtient 4,5 g de benzylcarbamoyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5SR) sous forme d'une meringue utilisée telle quelle dans les synthèses ultérieures.
C L'(amino-2 acétyl)-1 benzylcarbamoyl-4 (fluoro-2 phényl)-5 pyrrolidinecarboxylate-2 de tert-butyle-(2RS,4SR,5SR) peut être préparé comme décrit à l'exemple 16 C, mais à partir de 4,15 g de benzylcarbamoyl-4 (tert-butoxycarbonylamino-2 acétyl)-1 (fluoro-2 phényl)-5 pyrrolidinecarboxylate-2 de tert-butyle-(2RS,4SR,5SR) et de 1,07 cm³ d'iodotriméthylsilane dans 150 cm³ de chloroforme. Après traitement, on obtient 3,5 g d'(amino-2 acétyl)-1 benzylcarbamoyl-4 (fluoro-2 phényl)-5 pyrrolidinecarboxylate-2 de tert-butyle-(2RS,4SR,5SR) sous forme d'une huile utilisée telle quelle dans les synthèses ultérieures.
D Le benzylcarbamoyl-4 (tert-butoxycarbonylamino-2 acétyl)-1 (fluoro-2 phényl)-5 pyrrolidinecarboxylate-2 de tert-butyle-(2RS,4SR,5SR) peut être préparé comme décrit à l'exemple 26 D, mais à partir de 6,1 g de (tert-butoxycarbonylamino-2 acétyl)-1 (fluoro-2 phényl)-5 pyrrolidinedicarboxylate-2,4 acide de (2) tert-butyle-(2RS,4SR,5SR), de 2,1 g de N,N-diimidazole carbonyle, de 50 mg de diméthylamino-4 pyridine et de 1,43 cm³ de benzylamine dans 180 cm³ de dichloro-1,2 éthane. Après traitement, on obtient 4,3 g de benzylcarbamoyl-4 (tert-butoxycarbonylamino-2 acétyl)-1 (fluoro-2 phényl)-5 pyrrolidinecarboxylate-2 de tert-butyle-(2RS,4SR,5SR) sous forme d'une laque utilisée telle quelle dans les synthèses ultérieures.

Les médicaments selon l'invention sont constitués par un composé de formule (I) sous forme libre ou sous forme d'un sel pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la dépression, de la neurodégénération, des attaques de panique, de la maladie de Parkinson, de la diskynésie tardive, du syndrome du colon irritable, de la pancréatite aiguë, des ulcères, des désordres de la motilité intestinale, de certaines tumeurs sensibles à la CCK, des troubles de la mémoire, dans le sevrage aux traitements chroniques et abus d'alcool ou de médicaments, comme constricteurs de la pupille de l'oeil, comme analgésiques, comme potentialisateurs de l'activité analgésique des médicaments analgésiques narcotiques et non narcotiques et comme régulateurs de l'appétit.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'age, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p 4 cm³

## Revendications

1. Composés de formule : 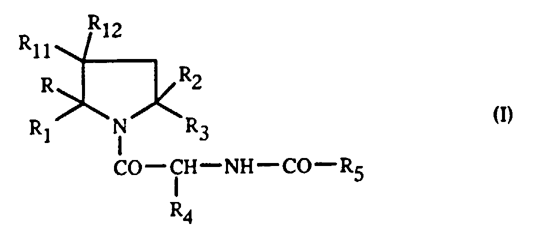 dans laquelle,
R représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy,
R₁ représente un atome d'hydrogène ou un radical alkyle,
R₂ représente une chaîne -(CH₂)ₙ-CO-R₆, -(CH₂)ₘ-O-CO-R"₆, -(CH₂)ₘ-NR₉R₁₀ ou un radical oxazolinyle éventuellement substitué par un ou plusieurs radicaux alkyle ou alkyl-3 oxadiazolyle,
R₃ représente un atome d'hydrogène ou un radical alkyle,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 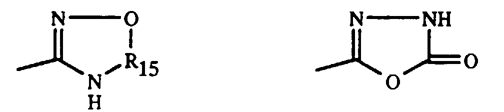 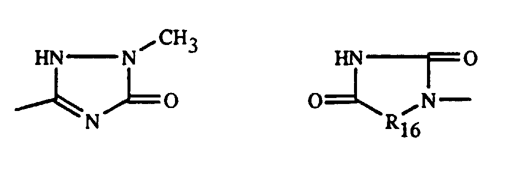 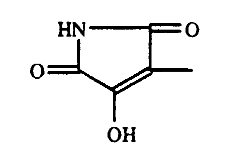 ou diméthyl-2,2 dioxo-4,6 dioxanne-1,3-yl-5,
R₆ représente un radical hydroxy, alcoxy, cycloalkyloxy, cycloalkylalkyloxy, phényle ou -NR₉R₁₀,
R"₆ représente un radical alcoxy, cycloalkyloxy, cycloalkylalkyloxy, ou -NR₉R₁₀,
R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₁ représente un atome d'hydrogène ou un radical alkyle ou phénylalkyle,
R₁₂ représente un radical alkyle, phénylalkyle, phénylsulfonyle, -(CH₂)ₚ-CO-R₁₇, cyano, -CXO, -CX=NOH, -CX=N-O-alk-COOX, -CHX-OH, -CHX-O-CO-alk, -NH₂ ou -NH-CO-alk,
R₁₃ représente un radical alkyle, cycloalkyle trifluorométhyle, phényle éventuellement substitué par un ou plusieurs substituants choisis parmi les radicaux cyano, alcoxy, nitro, amino et les atomes d'halogène,
R₁₄ représente un radical tétrazolyl-5,
R₁₅ représente C=O ou S=O,
R₁₆ représente O ou C=O,
R₁₇ représente un radical hydroxy, alcoxy, cycloalkyloxy, cycloalkylalkyloxy, phényle, phénylalcoxy, alkyle ou -NR₉R₁₀,
n est égal à 0, 1 ou 2,
m est égal à 1 ou 2,
p est égal à 0 ou 1
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène,
étant entendu que, sauf mention contraire, les radicaux alkyle, alkylène et alcoxy et les portions alkyle, alkylène et alcoxy contiennent 1 à 4 atomes de carbone en chaîne droite ou ramifiée, les radicaux et portions acyle contiennent 2 à 4 atomes de carbone et les radicaux et portions cycloalkyle contiennent 3 à 6 atomes de carbone,
ainsi que leurs sels et leurs isomères lorsqu'ils comportent au moins un centre asymétrique.

2. Composés de formule (I) selon la revendication 1 pour lesquels R représente un radical isopropylidène, cyclohexyle, tétrahydrophényle, cyclopentadiènyle, dihydrophényle, norbornyle, adamantyle ou norbornènyle ainsi que leurs sels et leurs isomères lorsqu'ils comportent au moins un centre asymétrique.

3. Composés de formule (I) selon la revendication 1 pour lesquels R₇ et R₈ forment avec l'atome d'azote auquel ils sont rattachés un hétérocycle choisi parmi les cycles pipéridino éventuellement substitué par un ou plusieurs radicaux alkyle ou tétrahydro-1,2,3,4 quinoléine ainsi que leurs sels et leurs isomères lorsqu'ils comportent au moins un centre asymétrique.

4. Composés de formule (I) selon la revendication 1 pour lesquels R₉ et R₁₀ forment avec l'atome d'azote auquel ils sont rattachés un hétérocycle choisi parmi les cycles pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle ainsi que leurs sels et leurs isomères lorsqu'ils comportent au moins un centre asymétrique.

5. Composés de formule (I) selon la revendication 1 pour lesquels R représente un radical phényle éventuellement substitué par un ou plusieurs atomes d'halogène, R₁ représente un atome d'hydrogène, R₂ représente une chaîne -(CH₂)ₙ-CO-R₆, R₃ représente un atome d'hydrogène, R₄ représente un atome d'hydrogène, R₅ représente un radical phénylamino dont le noyau phényle est sustitué par un ou plusieurs substituants choisis parmi les radicaux carboxy, -S-alk-COOX, -alk-COOX et tétrazolyl-5, R₆ représente un radical hydroxy ou alcoxy, R₁₁ représente un atome d'hydrogène, R₁₂ représente un radical phénylsulfonyle, -(CH₂)ₚ-CO-R₁₇, R₁₇ représente un radical hydroxy, phényle ou -NR₉R₁₀, p est égal à 0, n est égal à 0, X représente un atome d'hydrogène, R₉ et R₁₀ ont les mêmes significations que dans la revendication 1, leurs sels et leurs isomères lorsqu'ils comportent au moins un centre asymétrique.

6. Les composés de formule (I) selon la revendication 1 suivants :
- acide {[(tert-butoxycarbonyl-2 phényl-5 phénylsulfonyl-4 pyrrolidinyl-1)-2 oxo-2 éthyl]-3 uréido}-3 benzoïque-(2RS,4SR,5RS),
- {[(carboxyméthylthio-3 phényl)-3 uréido]-2 acétyl}-1 phényl-5 pyrrolidinedicarboxylate-2,4 acide de (2) tert-butyle-(2RS,4SR,5SR),
- acide ({[tert-butoxycarbonyl-2 (fluoro-2 phényl)-5 phénylsulfonyl-4 pyrrolidinyl-1]-2 oxo-2 éthyl}-3 uréido)-3 benzoïque-(2RS,4SR,5RS),
- acide ({[(diméthyl-3,3 pipéridino)carbonyl-2 (fluoro-2 phényl)-5 phénylsulfonyl-4 pyrrolidinyl-1]-2 oxo-2 éthyl}-3 uréido)-3 benzoïque-(2RS,4SR,5RS),
- {[(carboxyméthyl-3 phényl)-3 uréido]-2 acétyl}-1 fluoro-2 phényl)-5 pyrrolidinedicarboxylate-2,4 acide de (2) tert-butyle-(2RS,4SR,5SR),
- acide ({[tert-butoxycarbonyl-2 (fluoro-2 phényl)-5 phénylsulfonyl-4 pyrrolidinyl-1]-2 oxo-2 éthyl}-3 uréido)-3 phénylacétique-(2RS,4SR,5RS),
- acide {[(tert-butoxycarbonyl-2 phényl-5 (pyrrolidinyl-1) carbonyl-4 pyrrolidinyl-1)-2 oxo-2 éthyl]-3 uréido}-3 benzoïque-(2RS,4SR,5SR),
- acide {[(tert-butoxycarbonyl-2 diméthylcarbamoyl-4 phényl-5 pyrrolidinyl-1)-2 oxo-2 éthyl]-3 uréido}-3 benzoïque-(2RS,4SR,5SR),
- acide ({[benzoyl-4 tert-butoxycarbonyl-2 (fluoro-2 phényl)-5 pyrrolidinyl-1]-2 oxo-2 éthyl}-3 uréido)-3 benzoïque-(2RS-4SR,5SR),
- acide [({[tert-butoxycarbonyl-2 (fluoro-2 phényl)-5 phénylsulfonyl-4 pyrrolidinyl-1-(2R*,4S*,5R*)]-2 oxo-2 éthyl}-3 uréido)-3 phényl]-2 propionique,
- acide [({[tert-butoxycarbonyl-2 (fluoro-2 phényl)-5 phénylsulfonyl-4 pyrrolidinyl-1-(2R*,45*,5R*)]-2 oxo-2 éthyl}-3 uréido)-3 phényl]-2 méthoxy-2 acétique-(S),
- acide {[(tert-butoxycarbonyl-2 (fluoro-2 phényl)-5 morpholinocarbonyl-4 pyrrolidinyl-1)-2 oxo-2 éthyl]-3 uréido}-3 benzoïque-(2RS,4SR,5SR),
- acide {[(tert-butoxycarbonyl-2 diéthylaminocarbonyl-4 (fluoro-2 phényl)-5 pyrrolidinyl-1)-2 oxo-2 éthyl]-3 uréido}-3 benzoïque-(2RS,4SR,5SR),
- [({[tert-butoxycarbonyl-2 (fluoro-2 phényl)-5 phénylsulfonyl-4 pyrrolidinyl-1]-2 oxo-2 éthyl}-3 uréido)-3 phényl]-5 tétrazole-(2RS,4SR,5RS),
- acide ({[(fluoro-2 phényl)-5 isobutylcarbamoyl-2 phénylsulfonyl-4 pyrrolidinyl-1]-2 oxo-2 éthyl}-3 uréido)-3 phénylacétique-(2RS,4SR,5RS),
- acide-(-) ({[tert-butoxycarbonyl-2 (fluoro-2 phényl)-5 phénylsulfonyl-4 pyrrolidinyl-1]-2 oxo-2 éthyl}-3 uréido)-3 benzoïque-(2R*,4S*,5R*),
- (+)-{[(carboxyméthyl-3 phényl)-3 uréido]-2 acétyl}-1 (fluoro-2 phényl)-5 pyrrolidinedicarboxylate-2,4 acide de (2) tert-butyle-(2R*,4S*,5S*),
leurs sels et leurs isomères lorsqu'ils comportent au moins un centre asymétrique.

7. Procédé de préparation des composés de formule (I) selon la revendication 1 pour lesquels R₅ représente un radical phénylamino dont le noyau phényle est éventuellement substitué caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 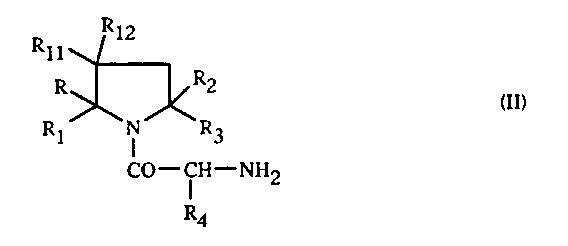 dans laquelle R, R₁, R₂, R₃,R₄, R₁₁ et R₁₂ ont les mêmes significations que dans la revendication 1, sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 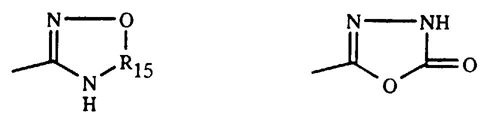 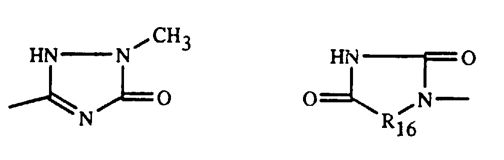 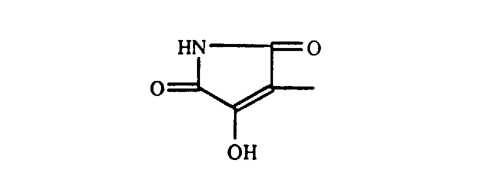 ou diméthyl-2,2 dioxo-4,6 dioxanne-1,3-yle-5, isole le produit et le transforme éventuellement en sel.

8. Procédé de préparation des composés de formule (I) selon la revendication 1 pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène caractérisé en ce que l'on fait réagir un dérivé de formule : 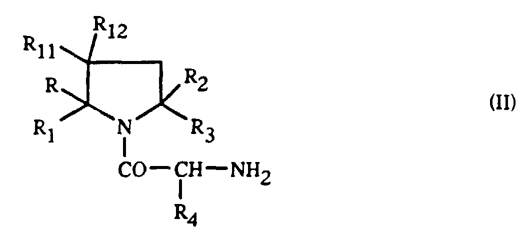 dans laquelle R, R₁, R₂, R₃, R₄, R₁₁ et R₁₂ ont les mêmes significations que dans la revendication 1, sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène, isole le produit et le transforme éventuellement en sel.

9. Procédé de préparation des composés de formule (I) selon la revendication 1 pour lesquels R₅ représente un radical phényle éventuellement substitué, naphtyle, indolyle, quinolyle caractérisé en ce que l'on fait réagir un dérivé de formule : 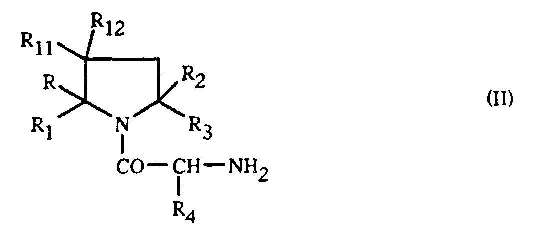 dans laquelle R, R₁, R₂, R₃, R₄, R₁₁ et R₁₂ ont les mêmes significations que dans la revendication 1, sur un acide de formule HOOC-R₅ dans laquelle R₅ a les mêmes significations que précédemment ou un dérivé réactif de cet acide, isole le produit et le transforme éventuellement en sel.

10. Procédé de préparation des composés de formule (I) selon la revendication 1 pour lesquels R₅ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH, -CX=N-O-alk-COOH et/ou R₁₂ représente un radical -(CH₂)ₚ-COOH, R, R₁, R₂, R₃, R₄, R₆, R₁₁ et R₁₂ sont définis comme dans la revendication 1 caractérisé en ce que l'on hydrolyse ou, selon le cas, hydrogénolyse un ester correspondant de formule (I), isole le produit et le transforme éventuellement en sel.

11. Procédé de préparation des composés de formule (I) lesquels R₅ représente un radical phénylamino dont le noyau phényle est substitué par un radical hydroxyiminoalkyle ou alcoxyiminoalkyle caractérisé en ce que l'on fait réagir un dérivé acylé de formule (I) correspondant avec un dérivé de formule :
H₂N-OR₂₁ (XVIII)
dans laquelle R₂₁ représente un atome d'hydrogène ou un radical alkyle, isole le produit et le transforme éventuellement en sel.

12. Procédé de préparation des composés de formule (I) selon la revendication 1 pour lesquels R₂ représente une chaîne -(CH₂)ₙ-CO-R₆, R₆ n'étant pas un radical hydroxy, R₁₂ représente un radical alkyle, phénylalkyle, phénylsulfonyle, cyano, -CXO ou -(CH₂)ₚ-CO-R₁₇, R₁₇ n'étant pas un radical hydroxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène caractérisé en ce que l'on fait réagir un dérivé de formule : 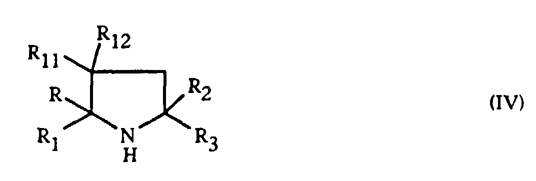 dans laquelle R, R₁, R₃ et R₁₁ ont les mêmes significations que dans la revendication 1, R₂ et R₁₂ ont les mêmes significations que précédemment, sur un acide de formule : 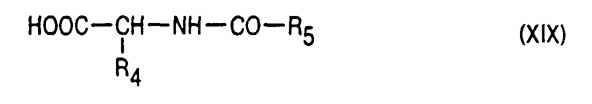 dans laquelle R₅ a les mêmes significations que ci-dessus ou un dérivé réactif de cet acide et R₄ a les mêmes significations que dans la revendication 1, isole le produit et le transforme éventuellement en sel.

13. Procédé de préparation des composés de formule (I) selon la revendication 1 pour lesquels R₁₂ représente un radical -CX=NOH, -CX=N-O-alk-COOX caractérisé en ce que l'on condense un dérivé de formule :
H₂N - OR₂₁ (XVIII)
dans laquelle R₂₁ représente un atome d'hydrogène ou un radical -alk-COOX sur un composé de formule (I) correspondant pour lequel R₁₂ représente un radical -CXO, isole le produit et le transforme éventuellement en sel.

14. Médicaments caractérisés en ce qu'ils contiennent comme principe actif au moins un des composés selon l'une des revendications 1 à 6.

15. Médicaments selon la revendication 14 utilisables pour le traitement des désordres liés à la CCK et à la gastrine.

## Patentansprüche

1. Verbindungen der Formel (I) 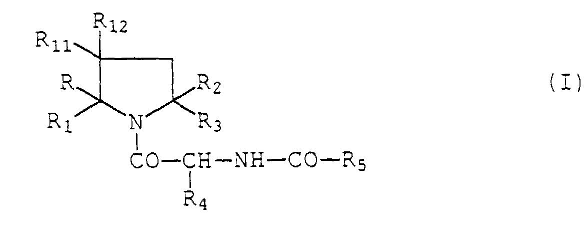 in der
R einen Alkylrest mit 1 bis 12 Kohlenstoffatomen in gerader oder verzweigter Kette und gegebenenfalls mono- oder polyungesättigt, Cycloalkyl mit 3 bis 12 Kohlenstoffatomen und gegebenenfalls mono- oder polyungesättigt, Polycycloalkyl mit 6 bis 12 Kohlenstoffatomen und gegebenenfalls mono- oder polyungesättigt, Phenylalkyl, dessen Phenylkern gegebenenfalls substituiert ist (durch einen oder mehrere Substituenten, ausgewählt unter den Resten Alkyl, Alkoxy oder den Halogenatomen), Diphenylalkyl, Cinnamyl, Pyridyl, gegebenenfalls substituiert durch einen oder mehrere Alkylreste, Furyl, gegebenenfalls substituiert durch einen oder mehrere Alkylreste, Thienyl, gegebenenfalls substituiert durch einen oder mehrere Alkylreste, Chinolyl, gegebenenfalls substituiert durch einen oder mehrere Alkylreste, Naphthyl, gegebenenfalls substituiert durch einen oder mehrere Alkylreste, Indolyl, gegebenenfalls substituiert durch einen oder mehrere Alkylreste oder Phenyl, gegebenenfalls substituiert durch einen oder mehrere Substituenten, ausgewählt unter den Halogenatomen und den Resten Alkyl, Alkoxy, Hydroxy, Nitro, Amino, Monoalkylamino, Dialkylamino, Alkcxycarbonyl, -CO-NR₇R₈, -NH-CO-CH₃, Trifluormethyl oder Trifluormethoxy darstellt,
R₁ ein Wasserstoffatom oder einen Alkylrest bedeutet,
R₂ eine Kette -(CH₂)ₙ-CO-R₆, -(CH₂)ₘ-O-CO-R"₆, -(CH₂)ₘ-NR₉R₁₀ oder einen Oxazolinylrest, gegebenenfalls substituiert durch einen oder mehrere Alkylreste oder 3-Alkyl-oxadiazolyl darstellt,
R₃ ein Wasserstoffatom oder einen Alkylrest bedeutet,
R₄ ein Wasserstoffatom oder einen Alkylrest bedeutet,
R₅ einen Phenylrest (gegebenenfalls substituiert durch einen oder mehrere Substituenten, ausgewählt unter den Halogenatomen und den Resten Alkyl, Alkoxy und Alkylthio), Naphthyl, Indolyl, Chinolyl oder Phenylamino, dessen Phenylkern gegebenenfalls substituiert ist durch einen oder mehrere Substituenten, ausgewählt unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H (in Form von Salz), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 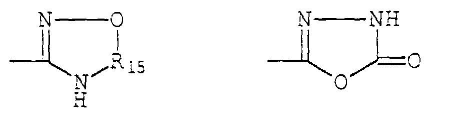 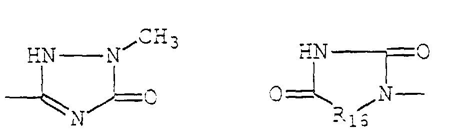 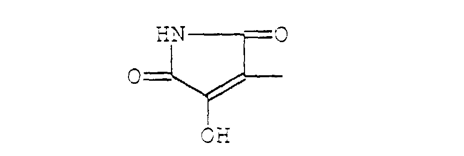 oder 2,2-Dimethyl-4,6-dioxo-1,3-dioxan-5-yl darstellt,
R₆ einen Rest hydroxy, Alkoxy, Cycloalkyloxy, Cycloalkylalkyloxy, Phenyl oder -NR₉R₁₀ bedeutet,
R"₆ einen Rest Alkoxy, Cycloalkyloxy, Oycloalkylalkyloxy, Phenyl oder -NR₉R₁₀ darstellt,
R₇ ein Wasserstoffatom oder einen Rest Alkyl, Phenylalkyl oder Phenyl, gegebenenfalls substituiert durch einen oder mehrere Substituenten, ausgewählt unter den Halogenatomen und den Resten Alkyl, Alkoxy und Alkylthio, bedeutet,
R₈ einen Rest Alkyl, Phenylalkyl oder Phenyl, gegebenenfalls substituiert durch einen oder mehrere Substituenten, ausgewählt unter den Halogenatomen und den Resten Alkyl, Alkoxy und Alkylthio, darstellt,
oder R₇ und R₈ zusammen mit dem Stickstoffatom, an das sie gebunden sind, einen mono- oder polycyclischen, gesättigten oder ungesättigten Heterocyclus mit 4 bis 9 Kohlenstoffatomen und einem oder mehreren Heteroatomen (O, N) und gegebenenfalls substituiert durch einen oder mehrere Alkylreste, bilden,
R₉ ein Wasserstoffatom oder einen Rest Alkyl, Cycloalkylalkyl, Cycloalkyl, Phenylalkyl, oder Phenyl, gegebenenfalls substituiert durch einen oder mehrere Substituenten, ausgewählt unter den Halogenatomen und den Resten Alkyl, Alkoxy und Alkylthio, bedeutet,
R₁₀ einen Rest Alkyl, Cycloalkylalkyl, Cycloalkyl, Phenylalkyl, oder Phenyl, gegebenenfalls substituiert durch einen oder mehrere Substituenten, ausgewählt unter den Halogenatomen und den Resten Alkyl, Alkoxy und Alkylthio, darstellt,
oder R₉ und R₁₀ zusammen mit dem Stickstoffatom, an das sie gebunden sind, einen mono- oder polycyclischen, gesättigten oder ungesättigten Heterocyclus mit 4 bis 9 Kohlenstoffatomen und einem oder mehreren Heteroatomen (O, N, S) und gegebenenfalls substituiert durch einen oder mehrere Alkylreste, bilden,
R₁₁ ein Wasserstoffatom oder einen Rest Alkyl oder Phenylalkyl bedeutet,
R₁₂ einen Rest Alkyl, Phenylalkyl, Phenylsulfonyl, -(CH₂)ₚ-CO-R₁₇, Cyano, -CXO, -CX=NOH, -CX=N-O-alk-COOX, -CHX-OH, -CHX-O-CO-alk, -NH₂ oder -NH-CO-alk darstellt,
R₁₃ einen Rest Alkyl, Cycloalkyl, Trifluormethyl, Phenyl, gegebenenfalls substituiert durch einen oder mehrere Substituenten, ausgewählt unter den Resten Cyano, Alkoxy, Nitro, Amino und den Halogenatomen, bedeutet,
R₁₄ einen 5-Tetrazolylrest darstellt,
R₁₅ C=O oder S=O bedeutet,
R₁₆ O oder C=O bedeutet,
R₁₇ einen Rest Hydroxy, Alkoxy, Cycloalkyloxy, Cycloalkylalkyloxy, Phenyl, Phenylalkoxy, Alkyl oder -NR₉R₁₀ darstellt,
n gleich 0, 1 oder 2 ist,
m gleich 1 oder 2 ist,
p gleich 0 oder 1 ist,
X ein Wasserstoffatom, einen Rest Alkyl oder Phenylalkyl darstellt,
alk einen Rest Alkyl oder Alkylen bedeutet,
alk' einen Rest Hydroxyalkyl, Hydroxyalkylen, Alkoxyalkyl oder Alkoxyalkylen bedeutet,
mit der Maßgabe, daß außer gegenteiliger Erwähnung die Reste Alkyl, Alkylen und Alkoxy und die Teile Alkyl, Alkylen und Alkoxy 1 bis 4 Kohlenstoffatome in gerader oder verzweigter Kette, die Reste und Teile Acyl 2 bis 4 Kohlenstoffatome und die Reste und Teile Cycloalkyl 3 bis 6 Kohlenstoffatome enthalten,
sowie ihre Salze und ihre Isomere, wenn sie mindestens ein asymmetrisches Zentrum umfassen.

2. Verbindungen der Formel (I) nach Anspruch 1, worin R einen Rest Isopropyliden, Cyclohexyl, Tetrahydrophenyl, Cyclopentadienyl, Dihydrophenyl, Norbornyl, Adamantyl oder Norbornenyl darstellt, sowie ihre Salze und ihre Isomere, wenn sie mindestens ein asymmetrisches Zentrum umfassen.

3. Verbindungen der Formel (I) nach Anspruch 1, worin R₇ und R₈ zusammen mit dem Stickstoffatom, an das sie gebunden sind, einen Heterocyclus bilden, ausgewählt unter den Ringen Piperidino, gegebenenfalls substituiert durch einen oder mehrere Alkylreste, oder 1,2,3,4-Tetrahydrochinolin, sowie ihre Salze und ihre Isomere, wenn sie mindestens ein asymmetrisches Zentrum umfassen.

4. Verbindungen der Formel (I) nach Anspruch 1, worin R₉ und R₁₀ zusammen mit dem Stickstoffatom, an das sie gebunden sind, einen Heterocyclus bilden, ausgewählt unter den Ringen Piperidino, 1-Perhydroazepinyl, 1,2,3,6-Tetrahydro-1-pyridyl, 1,2,3,4-Tetrahydro-1-chinolyl, 1-Pyrrolidinyl, 1,2,3,4-Tetrahydro-2-isochinolyl, Thiomorpholino oder 1-Indolinyl, wobei diese Ringe gegebenenfalls durch einen oder mehrere Alkylreste substituiert sein können, sowie ihre Salze und ihre Isomere, wenn sie mindestens ein asymmetrisches Zentrum umfassen.

5. Verbindungen der Formel (I) nach Anspruch 1, worin R einen Phenylrest darstellt, gegebenenfalls substituiert durch ein oder mehrere Halogenatome, R₁ ein Wasserstoffatom bedeutet, R₂ eine Kette -(CH₂)ₙ-CO-R₆ ist, R₃ ein Wasserstoffatom darstellt, R₄ ein Wasserstoffatom ist, R₅ einen Phenylaminorest bedeutet, dessen Phenylkern durch einen oder mehrere Substituenten substituiert ist, ausgewählt unter den Resten Carboxy, -S-alk-COOX, -alk-COOX und 5-Tetrazolyl, R₆ einen Rest Hydroxy oder Alkoxy darstellt, R₁₁ ein Wasserstoffatom ist, R₁₂ einen Rest Phenylsulfonyl, -(CH₂)ₚ-CO-R₁₇ bedeutet, R₁₇ einen Rest Hydroxy, Phenyl oder -NR₉R₁₀ darstellt, p gleich 0 ist, n gleich 0 ist, X ein Wasserstoffatom darstellt, R₉ und R₁₀ die gleichen Bedeutungen wie in Anspruch 1 besitzen, sowie ihre Salze und ihre Isomere, wenn sie mindestens ein asymmetrisches Zentrum umfassen.

6. Die folgenden Verbindungen der Formel (I) nach Anspruch 1:
- 3-{3-[2-(2-Tert.-butoxycarbonyl-5-phenyl-4-phenylsulfonyl-1-pyrrolidinyl)-2-oxo-ethyl]-ureido}-(2RS,4SR,5RS)benzoesäure,
- 1-{2-[3-(3-Carboxymethylthio-phenyl)-ureldo]-acetyl}-5-phenyl-2,4-pyrrolidindicarbonsäure-2-tert.-butylester(2RS,4SR,5SR),
- 3-{3-{2-[5-(2-Fluorphenyl)-2-tert.-butoxycarbonyl-4-phenylsulfonyl-1-pyrrolidinyl]-2-oxo-ethyl}-ureido}-(2RS,4SR,5RS)benzoesäure,
- 3-{3-{2-[5-(2-Fluorphenyl)-2-(3,3-dimethyl-piperidino)-carbonyl-4-phenylsulfonyl-1-pyrrolidinyl]-2-oxo-ethyl}-ureido}-(2RS,4SR,5RS)benzoesäure,
- 1-{2-[3-(3-Carboxymethyl-phenyl)-ureido]-acetyl}-5-(2-fluorphenyl)-2,4-pyrrolidindicarbonsäure-2-tert.-butylester(2RS,4SR,5SR),
- 3-{3-{2-[5-(2-Fluorphenyl)-2-tert.-butoxycarbonyl-4-phenylsulfonyl-1-pyrrolidinyl]-2-oxo-ethyl}-ureido)-(2RS,4SR,5RS)-phenylessigsäure,
- 3-{3-{2-[2-Tert.-butoxycarbonyl-5-phenyl-(1-pyrrolidinyl)-4-carbonyl-1-pyrrolidinyl]-2-oxo-ethyl}-ureido}-(2RS,4SR,5SR)-benzoesäure,
- 3-{3-[2-(2-Tert.-butoxycarbonyl-5-phenyl-4-dimethylcarbamoyl-1-pyrrolidinyl)-2-oxo-ethyl]-ureido}-(2RS,4SR,5SR)benzoesäure,
- 3-{3-{2-[5-(2-Fluorphenyl)-2-tert.-butoxycarbonyl-4-benzoyl-1-pyrrolidinyl]-2-oxo-ethyl}-ureido}-(2RS, 4SR,5SR)benzoesäure,
- 2-{3-{3-{2-[5-(2-Fluorphenyl)-2-tert.-butoxycarbonyl-4-phenylsulfonyl-1-pyrrolidinyl(2R*,4S*,5R*)]-2-oxo-ethyl}-ureido}-phenyl}-propionsäure,
- 2-{3-(3-{2-[5-(2-Fluorphenyl)-2-tert.-butoxycarbonyl-4-phenylsulfonyl-1-pyrrolidinyl(2R*,4S*,5R*)]-2-oxo-ethyl}-ureido}-phenyl}-2-methoxy-essigsäure(S),
- 3-{3-{2-[2-Tert.-butoxycarbonyl-5-(2-fluorphenyl)-4-morpholinocarbonyl-1-pyrrolidinyl]-2-oxo-ethyl}-ureido}-(2RS,4SR,5SR)-benzoesäure,
- 3-{3-{2-[2-Tert.-butoxycarbonyl-5-(2-fluorphenyl)-4-diethylaminocarbonyl-1-pyrrolidinyl]-2-oxo-ethyl}-ureido}-(2RS,4SR,5SR)-benzoesäure,
- 5-{3-{3-{2-[2-Tert.-butoxycarbonyl-5-(2-fluorphenyl)-4-phenylsulfonyl-1-pyrrolidinyl]-2-oxo-ethyl}-ureido}-phenyl}-tetrazol-(2RS,4SR,5RS)
- 3-{3-{2-[5-(2-Fluorphenyl)-2-isobutylcarbamoyl-4-phenylsulfonyl-1-pyrrolidinyl]-2-oxo-ethyl}-ureido}-(2RS,4SR,5RS)phenylessigsäure,
- (-)3-{3-{2-[5-(2-Fluorphenyl)-2-tert.-butoxycarbonyl-4-phenylsulfonyl-1-pyrrolidinyl]-2-oxo-ethyl}-ureido}-(2R*,4S*,5R*)-benzoesäure,
- (+)1-{2-[3-(3-Carboxymethyl-phenyl)-ureido]-acetyl}-5-(2-fluorphenyl)-2,4-pyrrolidindicarbonsäure-2-tert.-butylester-(2R*,4S*,5S*)
sowie ihre Salze und ihre Isomere, wenn sie mindestens ein asymmetrisches Zentrum umfassen.

7. Verfahren zur Herstellung der Verbindungen der Formel (I) nach Anspruch 1, worin R₅ einen Phenylaminorest darstellt, dessen Phenylkern gegebenenfalls substituiert ist, dadurch gekennzeichnet, daß man ein reaktives Derivat der Carbaminsäure, gegebenenfalls in situ erhalten durch Umsetzung eines reaktiven Derivates der Carbonsäure, ausgewählt unter N,N'-Carbonyldiimidazol, Phosgen, Diphosgen, Triphosgen und Chlorameisensäure-p-nitrophenylester, mit einem Derivat der Formel (II) 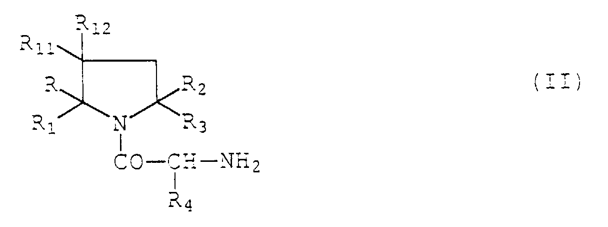 in der R, R₁, R₂, R₃, R₄, R₁₁ und R₁₂ die gleichen Bedeutungen wie in Anspruch 1 besitzen, mit einem Anilin zur Reaktion bringt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H (in Form von Salz), -CH=CH-alk', -C (=NOH) -COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 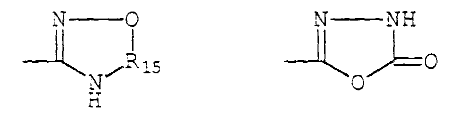 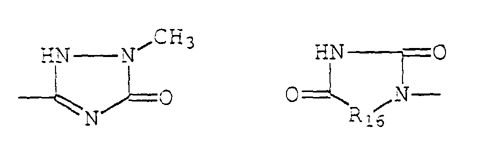 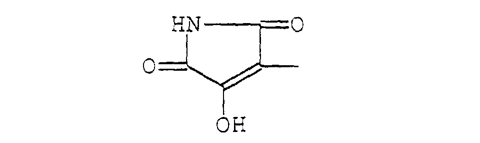 oder 2,2-Dimethyl-4,6-dioxo-1,3-dioxan-5-yl darstellt, das Produkt isoliert und gegebenenfalls in Salz überführt.

8. Verfahren zur Herstellung der Verbindungen der Formel (I) nach Anspruch 1, worin R₅ einen Phenylaminorest darstellt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X von einem Wasserstoffatom verschieden ist, dadurch gekennzeichnet, daß man ein Derivat der Formel (II) 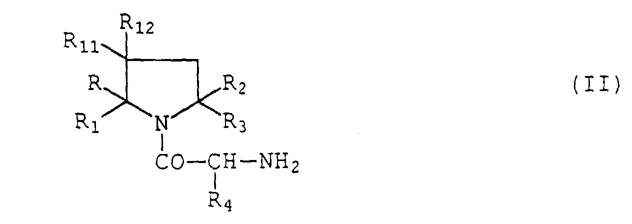 in der R, R₁, R₂, R₃, R₄, R₁₁ und R₁₂ die gleichen Bedeutungen wie in Anspruch 1 besitzen, mit einem Phenylisocyanat zur Reaktion bringt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X von einem Wasserstoffatom verschieden ist, das Produkt isoliert und gegebenenfalls in Salz überführt.

9. Verfahren zur Herstellung der Verbindungen der Formel (I) nach Anspruch 1, worin R₅ einen Rest Phenyl, gegebenenfalls substituiert, Naphthyl, Indolyl oder Chinolyl darstellt, dadurch gekennzeichnet, daß man ein Derivat der Formel (II) 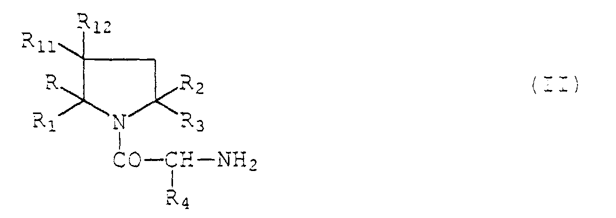 in der R, R₁, R₂, R₃, R₄, R₁₁ und R₁₂ die gleichen Bedeutungen wie in Anspruch 1 besitzen, mit einer Säure der Formel HOOC-R₅ oder mit einem reaktiven Derivat dieser Säure zur Reaktion bringt, worin R₅ die gleichen Bedeutungen wie oben besitzt, das Produkt isoliert und gegebenenfalls in Salz überführt.

10. Verfahren zur Herstellung der Verbindungen der Formel (I) nach Anspruch 1, worin R₅ einen Phenylaminorest darstellt, dessen Phenylkern durch einen Rest Carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH, -CX=N-O-alk-COOH, substituiert ist, und/oder R₁₂ einen Rest -(CH₂)ₚ-COOH bedeutet, R, R₁, R₂, R₃, R₄, R₆, R₁₁ und R₁₂ wie in Anspruch 1 definiert sind, dadurch gekennzeichnet, daß man einen entsprechenden Ester der Formel (I) hydrolysiert oder gegebenenfalls hydrogenolysiert, das Produkt isoliert und gegebenenfalls in Salz überführt.

11. Verfahren zur Herstellung der Verbindungen der Formel (I), worin R₅ einen Phenylaminorest darstellt, dessen Phenylkern durch einen Rest Hydroxyiminoalkyl oder Alkoxyiminoalkyl substituiert ist, dadurch gekennzeichnet, daß man ein entsprechendes Acylderivat der Formel (I) mit einem Derivat der Formel (XVIII)
H₂N-OR₂₁ (XVIII)
in der R₂₁ ein Wasserstoffatom oder einen Alkylrest darstellt, zur Reaktion bringt, das Produkt isoliert und gegebenenfalls in Salz überführt.

12. Verfahren zur Herstellung der Verbindungen der Formel (I) nach Anspruch 1, worin R₂ eine Kette -(CH₂)ₙ-CO-R₆ darstellt, R₆ kein Hydroxyrest ist, R₁₂ einen Rest Alkyl, Phenylalkyl, Phenylsulfonyl, Cyano, -CXO oder -(CH₂)ₚ-CO-R₁₇ bedeutet, R₁₇ kein Hydroxyrest ist und R₅ einen Rest Phenyl, gegebenenfalls substituiert, Naphthyl, Indolyl, Chinolyl oder Phenylamino darstellt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X von einem Wasserstoffatom verschieden ist, dadurch gekennzeichnet, daß man ein Derivat der Formel (IV) 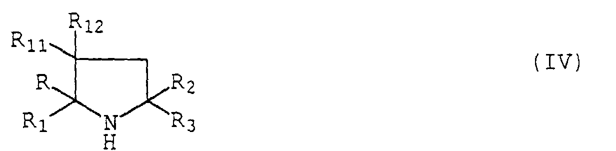 in der R, R₁, R₃ und R₁₁ die gleichen Bedeutungen wie in Anspruch 1 besitzen, R₂ und R₁₂ die gleichen Bedeutungen wie vorstehend aufweisen, mit einer Säure der Formel (XIX) 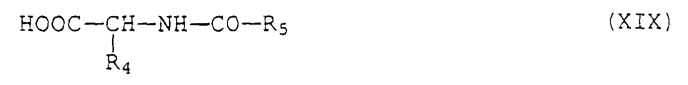 oder mit einem reaktiven Derivat dieser Säure zur Reaktion bringt, worin R₅ die gleiche Bedeutung wie oben und R₄ die gleiche Bedeutung wie in Anspruch 1 besitzt, das Produkt isoliert und gegebenenfalls in Salz überführt.

13. Verfahren zur Herstellung der Verbindungen der Formel (I) nach Anspruch 1, worin R₁₂ einen Rest -CX=NOH, -CX=N-O-alk-COOX, darstellt, dadurch gekennzeichnet, daß man ein Derivat der Formel (XVIII)
H₂N-OR₂₁ (XVIII)
in der R₂₁ ein Wasserstoffatom oder einen Rest -alk-COOX darstellt, mit einer entsprechenden Verbindung der Formel (I) kondensiert, in der R₁₂ einen Rest -CXO bedeutet, das Produkt isoliert und gegebenenfalls in Salz überführt.

14. Arzneimittel, dadurch gekennzeichnet, daß sie als Wirkstoff mindestens eine der Verbindungen nach einem der Ansprüche 1 bis 6 enthalten.

15. Arzneimittel nach Anspruch 14, anwendbar zur Behandlung von Störungen, die mit CCK und Gastrin in Zusammenhang stehen.

## Claims

1. Compounds of formula: 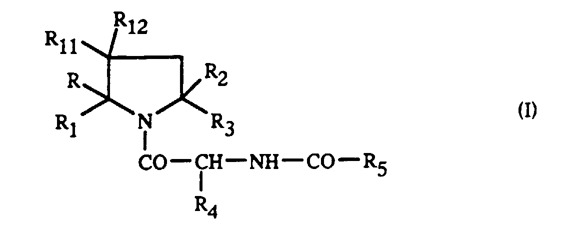 in which,
R represents an alkyl radical containing 1 to 12 carbon atoms in an optionally mono- or polyunsaturated, straight or branched chain, a cycloalkyl radical containing 3 to 12 carbon atoms and optionally mono- or polyunsaturated, a polycycloalkyl radical containing 6 to 12 carbon atoms and optionally mono- or polyunsaturated, a phenylalkyl radical in which the phenyl ring is optionally substituted (by one or a number of substituents chosen from alkyl or alkoxy radicals or halogen atoms), a diphenylalkyl radical, a cinnamyl radical, a pyridyl radical optionally substituted by one or a number of alkyl radicals, a furyl radical optionally substituted by one or a number of alkyl radicals, a thienyl radical optionally substituted by one or a number of alkyl radicals, a quinolyl radical optionally substituted by one or a number of alkyl radicals, a naphthyl radical optionally substituted by one or a number of alkyl radicals, an indolyl radical optionally substituted by one or a number of alkyl radicals or a phenyl radical optionally substituted by one or a number of substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or trifluoromethoxy radicals,
R₁ represents a hydrogen atom or an alkyl radical,
R₂ represents a -(CH₂)ₙ-CO-R₆, -(CH₂)ₘ-O-CO-R"₆ or -(CH₂)ₘ-NR₉R₁₀ chain, an oxazolinyl radical optionally substituted by one or a number of alkyl radicals, or a 3-alkyloxadiazolyl radical,
R₃ represents a hydrogen atom or an alkyl radical,
R₄ represents a hydrogen atom or an alkyl radical,
R₅ represents a phenyl radical (optionally substituted by one or a number of substituents chosen from halogen atoms and alkyl, alkoxy and alkylthio radicals), a naphthyl radical, an indolyl radical, a quinolyl radical or a phenylamino radical in which the phenyl ring is optionally substituted by one or a number of substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃-H (in the salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 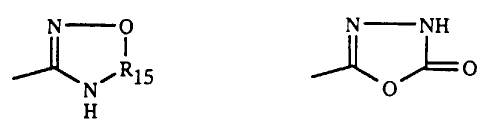 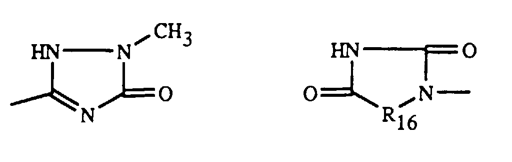 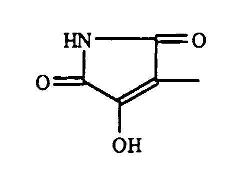 or 2,2-dimethyl-4,6-dioxo-1,3-dioxan-5-yl radicals,
R₆ represents a hydroxyl, alkoxy, cycloalkyloxy, cycloalkylalkyloxy, phenyl or -NR₉R₁₀ radical,
R"₆ represents an alkoxy, cycloalkyloxy, cycloalkylalkyloxy, phenyl or -NR₉R₁₀ radical,
R₇ represents a hydrogen atom or an alkyl radical, phenylalkyl radical or phenyl radical optionally substituted by one or a number of substituents chosen from halogen atoms and alkyl, alkoxy and alkylthio radicals,
R₈ represents an alkyl radical, phenylalkyl radical or phenyl radical optionally substituted by one or a number of substituents chosen from halogen atoms and alkyl, alkoxy and alkylthio radicals,
or else R₇ and R₈ form, with the nitrogen atom to which they are attached, a saturated or unsaturated, mono- or polycyclic heterocycle containing 4 to 9 carbon atoms and one or a number of heteroatoms (O, N) and optionally substituted by one or a number of alkyl radicals,
R₉ represents a hydrogen atom or an alkyl radical, cycloalkylalkyl radical, cycloalkyl radical, phenylalkyl radical or phenyl radical optionally substituted by one or a number of substituents chosen from halogen atoms and alkyl, alkoxy and alkylthio radicals,
R₁₀ represents an alkyl radical, cycloalkylalkyl radical, cycloalkyl radical, phenylalkyl radical or phenyl radical optionally substituted by one or a number of substituents chosen from halogen atoms and alkyl, alkoxy and alkylthio radicals,
or else R₉ and R₁₀ form, together with the nitrogen atom to which they are attached, a saturated or unsaturated, mono- or polycyclic heterocycle containing 4 to 9 carbon atoms and one or a number of heteroatoms (O, N, S) and optionally substituted by one or a number of alkyl radicals,
R₁₁ represents a hydrogen atom or an alkyl or phenylalkyl radical,
R₁₂ represents an alkyl, phenylalkyl, phenylsulphonyl, -(CH₂)ₚ-CO-R₁₇, cyano, -CXO, -CX=NOH, -CX=N-O-alk-COOX, -CHX-OH, -CHX-O-CO-alk, -N₂ or -NH-CO-alk radical,
R₁₃ represents an alkyl radical, a cycloalkyl radical, a trifluoromethyl radical or a phenyl radical optionally substituted by one or a number of substituents chosen from cyano, alkoxy, nitro or amino radicals and halogen atoms,
R₁₄ represents a 5-tetrazolyl radical,
R₁₅ represents C=O or S=O,
R₁₆ represents O or C=O,
R₁₇ represents a hydroxyl, alkoxy, cycloalkyloxy, cycloalkylalkyloxy, phenyl, phenylalkoxy, alkyl or -NR₉R₁₀ radical,
n is equal to 0, 1 or 2,
m is equal to 1 or 2,
p is equal to 0 or 1,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical,
it being understood that, except when otherwise mentioned, the alkyl, alkylene and alkoxy radicals and the alkyl, alkylene and alkoxy portions contain 1 to 4 carbon atoms in a straight or branched chain, the acyl radicals and portions contain 2 to 4 carbon atoms and the cycloalkyl radicals and portions contain 3 to 6 carbon atoms,
and their salts and their isomers when they contain at least one asymmetric centre.

2. Compounds of formula (I) according to claim 1, for which R represents an isopropyliden, cyclohexyl, tetrahydrophenyl, cyclopentadienyl, dihydrophenyl, norbornyl, adamantyl or norbornenyl radical and their salts and their isomers when they contain at least one asymmetric centre.

3. Compounds of formula (I) according to claim 1 for which R₇ and R₈ form, with the nitrogen atom to which they are attached, a heterocycle chosen from piperidino rings optionally substituted by one or a number of alkyl or 1,2,3,4-tetrahydroquinoline radicals and their salts and their isomers when they contain at least one asymmetric centre.

4. Compounds of formula (I) according to claim 1 for which R₉ and R₁₀ form, with the nitrogen atom to which they are attached, a heterocycle chosen from piperidino, 1-perhydroazepinyl, 1,2,3,6-tetrahydro-1-pyridyl, 1,2,3,4-tetrahydro-1-quinolyl, 1-pyrrolidinyl, 1,2,3,4-tetrahydro-2-isoquinolyl, thiomorpholino or 1-indolinyl rings, it being possible for these rings to be optionally substituted by at least one alkyl radical, and their salts and their isomers when they contain at least one asymmetric centre.

5. Compounds of formula (I) according to claim 1 for which R represents a phenyl radical optionally substituted by one or a number of halogen atoms, R₁ represents a hydrogen atom, R₂ represents a -(CH₂)ₙ-CO-R₆ chain, R₃ represents a hydrogen atom, R₄ represents a hydrogen atom, R₅ represents a phenylamino radical in which the phenyl ring is substituted by one or a number of substituents chosen from carboxyl, -S-alk-COOX, -alk-COOX and 5-tetrazolyl radicals, R₆ represents a hydroxyl or alkoxy radical, R₁₁ represents a hydrogen atom, R₁₂ represents a phenylsulphonyl or -(CH₂)ₚ-CO-R₁₇ radical, R₁₇ represents a hydroxyl, phenyl or -NR₉R₁₀ radical, p is equal to 0, n is equal to 0, X represents a hydrogen atom and R₉ and R₁₀ have the same meanings as in claim 1, their salts and their isomers when they contain at least one asymmetric centre.

6. The following compounds of formula (I) according to claim 1:
- (2RS,4SR,5RS)-3-{3-[2-(2-tert-butoxycarbonyl-5-phenyl-4-phenylsulphonyl-1-pyrrolidinyl)-2-oxoethyl]-ureido}benzoic acid,
- 2-tert-butyl hydrogen (2RS,4SR,5SR)-1-{2-[3-(3-(carboxymethylthio)phenyl)ureido]acetyl}-5-phenylpyrrolidine-2,4-dicarboxylate
- (2RS,4SR,5RS)-3-{-3-[2-(2-tert-butoxycarbonyl-5-(2-fluorophenyl)-4-phenylsulphonyl-1-pyrrolidinyl)-2-oxoethyl]ureido}benzoic acid,
- (2RS,4SR,5RS)-3-{3-[2-(2-(3,3-dimethylpiperidinocarbonyl)-5-(2-fluorophenyl)-4-phenylsulphonyl-1-pyrrol idinyl)-2-oxoethyl]ureido}benzoic acid,
- 2-tert-butyl hydrogen (2RS,4SR,5SR)-1-{2-[3-(3-(carboxymethyl)phenyl)ureido]acetyl}-5(2-fluorophenyl)-pyrrolidine-2,4-dicarboxylate,
- (2RS,4SR,5RS)-3-{3-{2-[2-tert-butoxycarbonyl-5-(2-fluorophenyl)-4-phenylsulphonyl-1-pyrrolidinyl]-2-oxoethyl}ureido)phenylacetic acid,
- (2RS,4SR,5SR)-3-{3-[2-(2-tert-butoxycarbonyl-5-phenyl-4-(1-pyrrolidinylcarbonyl)-1-pyrrolidinyl)-2-oxoethyl]ureido}benzoic acid,
- (2RS,4SR,5SR)-3-{3-[2-(2-tert-butoxycarbonyl-4-dimethylcarbamoyl-5-phenyl-1-pyrrolidinyl)-2-oxoethyl]ureido}benzoic acid,
- (2RS,4SR,5SR)-3-{3-[2-(4-benzoyl-2-tertbutoxycarbonyl-5-(2-fluorophenyl)-1-pyrrolidinyl)-2-oxoethyl]ureido}benzoic acid,
- 2-{3-[3-(2-((2R*,4S*,5R*)-2-tert-butoxycarbonyl-5-(2-fluorophenyl)-4-phenylsulphonyl-1-pyrrolidinyl)-2-oxoethyl)ureidolphenyl}propionic acid,
- (S)-2-{3-[3-(2-((2R*,4S*,5R*)-2-tertbutoxycarbonyl-5-(2-fluorophenyl)-4-phenylsulphonyl-1-pyrrolidinyl)-2-oxoethyl)ureido]phenyl}2-methoxyacetic acid,
- (2RS,4SR,5SR)-3-{3-[2-(2-tert-butoxycarbonyl-5-(2-fluorophenyl)-4-morpholinocarbonyl-1-pyrrolidinyl)-2-oxoethyl]ureido}benzoic acid,
- (2RS,4SR,5SR)-3-{3-[2-(2-tert-butoxycarbonyl-4-diethylaminocarbonyl-5-(2-fluorophenyl)-1-pyrrolidinyl)-2-oxoethyl]ureido}benzoic acid,
- (2RS,4SR,5RS)-5-{3-[3-(2-(2-tert-butoxycarbonyl-5-(2-fluorophenyl)-4-phenylsulphonyl-1-pyrrolidinyl)-2-oxoethyl)ureido]phenyl}tetrazole,
- (2RS,4SR,5RS)-3-{3-[2-(5-(2-fluorophenyl)2-isobutylcarbamoyl-4-phenylsulphonyl-1-pyrrolidinyl)2-oxoethyl]ureido}phenylacetic acid,
- (2R*,4S*,5R*)-(-)-3-{3-[2-(2-tert-butoxycarbonyl-5-(2-fluorophenyl)-4-phenylsulphonyl-1-pyrrolidinyl)-2-oxoethyl]ureido}benzoic acid,
- 2-tert-butyl hydrogen (2R*,4S*,5S*)-(+)-1-{2-[3-(3-(carboxymethyl)phenyl)ureido]acetyl}-5(2-fluorophenyl)pyrrolidine-2,4-dicarboxylate,
their salts and their isomers when they contain at least one asymmetric centre.

7. Process for the preparation of the compounds of formula (I) according to claim 1 for which R₅ represents a phenylamino radical in which the phenyl ring is optionally substituted, characterized in that a reactive derivative of carbamic acid, optionally obtained in situ by reacting a reactive derivative of carbonic acid chosen from N,N'-carbonyldiimidazole, phosgene, diphosgene, triphosgene and p-nitrophenyl chloroformate with a derivative of formula: 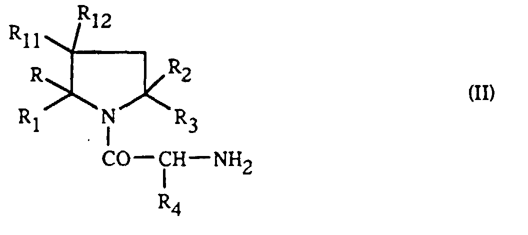 in which R, R₁, R₂, R₃, R₄, R₁₁ and R₁₂ have the same meanings as in claim 1, is reacted with an aniline in which the phenyl ring is optionally substituted by one or a number of substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃-H (in the salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₃, -SO₂-NH-SO₂-R₁₃, -CO-NH-CO-R₁₃, -CO-NH-SO₂-R₁₃, -B(OH)₂, -C(NH₂)=NOH, -SO₂-NH-R₁₄, -CO-NH-R₁₄, 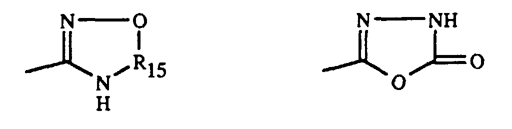 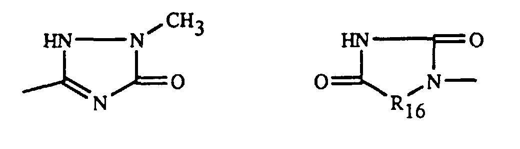 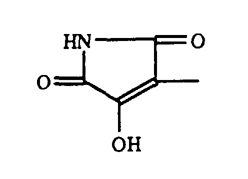 or 2,2-dimethyl-4,6-dioxo-1,3-dioxan-5-yl radicals, the product isolated and optionally converted to a salt.

8. Process for the preparation of the compounds of formula (I) according to claim 1 for which R₅ represents a phenylamino radical in which the phenyl ring is optionally substituted by one or a number of substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in the salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, characterized in that a derivative of formula: 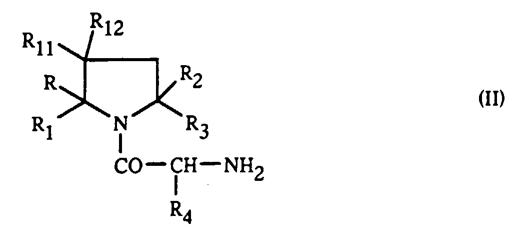 in which R, R₁, R₂, R₃, R₄, R₁₁ and R₁₂ have the same meanings as in claim 1, is reacted with a phenyl isocyanate in which the phenyl ring is optionally substituted by one or a number of substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in the salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, the product isolated and optionally converted to a salt.

9. Process for the preparation of the compounds of formula (I) according to claim 1 for which R₅ represents a naphthyl, indolyl, quinolyl or optionally substituted phenyl radical, characterized in that a derivative of formula: 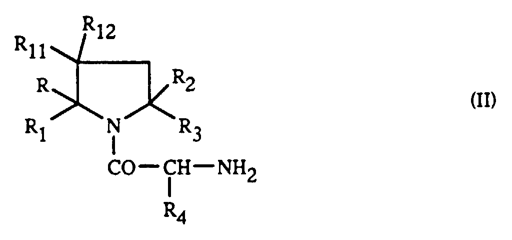 in which R, R₁, R₂, R₃, R₄, R₁₁ and R₁₂ have the same meanings as in claim 1, is reacted with an acid of formula HOOC-R₅ in which R₅ has the same meanings as above or a reactive derivative of this acid, the product isolated and optionally converted to a salt.

10. Process for the preparation of the compounds of formula (I) according to claim 1, for which R₅ represents a phenylamino radical in which the phenyl ring is substituted by a carboxyl, alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH or -CX=N-O-alk-COOH radical and/or R₁₂ represents a -(CH₂)ₚ-COOH radical, R, R₁, R₂, R₃, R₄, R₆, R₁₁ and R₁₂ are defined as in claim 1, characterized in that a corresponding ester of formula (I) is hydrolysed or, depending on the situation, hydrogenolysed, the product isolated and optionally converted to a salt.

11. Process for the preparation of the compounds of formula (I) in which R₅ represents a phenylamino radical in which the phenyl ring is substituted by a hydroxyiminoalkyl or alkoxyiminoalkyl radical, characterized in that a corresponding acylated derivative of formula (I) is reacted with a derivative of formula:
H₂N-OR₂₁ (XVIII)
in which R₂₁ represents a hydrogen atom or an alkyl radical, the product isolated and optionally converted to a salt.

12. Process for the preparation of the compounds of formula (I) according to claim 1 for which R₂ represents a -(CH₂)ₙ-CO-R₆ chain, R₆ not being a hydroxyl radical, R₁₂ represents an alkyl, phenylalkyl, phenylsulphonyl, cyano, -CXO or -(CH₂)ₚ-CO-R₁₇ radical, R₁₇ not being a hydroxyl radical, and R₅ represents a naphthyl, indolyl, quinolyl or optionally substituted phenyl radical or a phenylamino radical in which the phenyl ring is optionally substituted by one or a number of substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in the salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, characterized in that a derivative of formula: 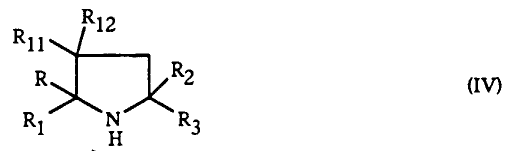 in which R, R₁, R₃ and R₁₁ have the same meanings as in claim 1 and R₂ and R₁₂ have the same meanings as above, is reacted with an acid of formula: 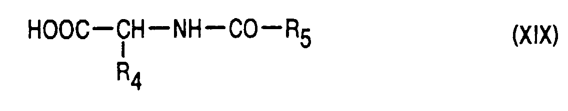 in which R₅ has the same meanings as above, or a reactive derivative of this acid and R₄ has the same meanings as in claim 1, the product isolated and optionally converted to a salt.

13. Process for the preparation of the compounds of formula (I) according to claim 1 for which R₁₂ represents a -CX=NOH or -CX=N-O-alk-COOX radical, characterized in that a derivative of formula:
H₂N-OR₂₁ (XVIII)
in which R₂₁ represents a hydrogen atom or an -alk-COOX radical, is condensed with a corresponding compound of formula (I) for which R₁₂ represents a -CXO radical, the product isolated and optionally converted to a salt.

14. Medicaments, characterized in that they contain, as active principle, at least one of the compounds according to one of claims 1 to 6.

15. Medicaments according to claim 14 which can be used for the treatment of disorders linked to CCK and to gastrin.
